# EUROPEAN PATENT SPECIFICATION

(11) **EP 2 121 694 B1**
(45) Date of publication and mention of the grant of the patent: **16.07.2014**
(21) Application number: 07758437.3
(22) Date of filing: 13.03.2007
(51) Int. Cl.: C07D 487/04, A61P 31/12, A61K 31/55

(54) **CYCLOPROPYL FUSED INDOLOBENZAZEPINE HCV NS5B INHIBITORS**
CYCLOPROPYL KONDENSIERTE INDOLOBENZAZEPINE ALS HCV-NS5B-INHIBITOREN
INHIBITEURS DE VHC NS5B DE TYPE INDOLOBENZAZÉPINE FUSIONNÉE À UN CYCLOPROPYLE

(43) Date of publication of application: 25.11.2009
(73) Proprietor: Bristol-Myers Squibb Company, Princeton, NJ 08543 (US)
(72) Inventor: MEANWELL, Nicholas, A., Wallingford, Connecticut 06492 (US); GENTLES, Robert G., Wallingford, Connecticut 06492 (US); DING, Min, Wallingford, Connecticut 06492 (US); BENDER, John A., Wallingford, Connecticut 06492 (US); KADOW, John F., Wallingford, Connecticut 06492 (US); HEWAWASAM, Piyasena, Wallingford, Connecticut 06492 (US); HUDYMA, Thomas W., Durham, Connecticut 06422 (US); ZHENG, Xiaofan, Wallingford, Connecticut 06492 (US)
(74) Representative: Reitstötter Kinzebach
(86) International application number: PCT/US2007/063885
(87) International publication number: WO 2008/111978

(56) References cited:
- WO-A-2006/020082
- WO-A-2006/046030
- WO-A-2007/033032

## Description

### BACKGROUND OF THE INVENTION

Hepatitis C virus (HCV) is a major human pathogen, infecting an estimated 170 million persons worldwide - roughly five times the number infected by human immunodeficiency virus type 1. A substantial fraction of these HCV infected individuals develop serious progressive liver disease, including cirrhosis and hepatocellular carcinoma (Lauer, G. M.; Walker, B. D. N. Engl. J. Med. 2001, 345, 41-52).

HCV is a positive-stranded RNA virus. Based on a comparison of the deduced amino acid sequence and the extensive similarity in the 5'-untranslated region, HCV has been classified as a separate genus in the Flaviviridae family. All members of the Flaviviridae family have enveloped virions that contain a positive stranded RNA genome encoding all known virus-specific proteins via translation of a single, uninterrupted, open reading frame.

Considerable heterogeneity is found within the nucleotide and encoded amino acid sequence throughout the HCV genome. At least six major genotypes have been characterized, and more than 50 subtypes have been described. The major genotypes of HCV differ in their distribution worldwide, and the clinical significance of the genetic heterogeneity of HCV remains elusive despite numerous studies of the possible effect of genotypes on pathogenesis and therapy.

The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non-structural (NS) proteins. In the case of HCV, the generation of mature non-structural proteins (NS2, NS3, NS4A, NS4B, NS5A, and NS5B) is effected by two viral proteases. The first one is believed to be a metalloprotease and cleaves at the NS2-NS3 junction; the second one is a serine protease contained within the N-terminal region of NS3 (also referred to as NS3 protease) and mediates all the subsequent cleavages downstream of NS3, both in cis, at the NS3-NS4A cleavage site, and in trans, for the remaining NS4A-NS4B, NS4B-NS5A, NS5A-NS5B sites. The NS4A protein appears to serve multiple functions, acting as a cofactor for the NS3 protease and possibly assisting in the membrane localization of NS3 and other viral replicase components. The complex formation of the NS3 protein with NS4A seems necessary to the processing events, enhancing the proteolytic efficiency at all of the sites. The NS3 protein also exhibits nucleoside triphosphatase and RNA helicase activities. NS5B (also referred to as HCV polymerase) is a RNA-dependent RNA polymerase that is involved in the replication of HCV. The HCV NS5B protein is described in "Structural Analysis of the Hepatitis C Virus RNA Polymerase in Complex with Ribonucleotides (Bressanelli; S. et al., Journal of Virology 2002, 3482-3492; and Defrancesco and Rice, Clinics in Liver Disease 2003, 7, 211-242.

Currently, the most effective HCV therapy employs a combination of alpha-interferon and ribavirin, leading to sustained efficacy in 40% of patients (Poynard, T. et al. Lancet 1998, 352, 1426-1432). Recent clinical results demonstrate that pegylated alpha-interferon is superior to unmodified alpha-interferon as monotherapy (Zeuzem, S. et al. N. Engl. J Med 2000, 343, 1666-1672). However, even with experimental therapeutic regimens involving combinations of pegylated alpha-interferon and ribavirin, a substantial fraction of patients do not have a sustained reduction in viral load. Thus, there is a clear and important need to develop effective therapeutics for treatment of HCV infection.

HCV NS5B inhibitors have been disclosed. See WO 2007/136982, WO 2008/097796, WO 2006/046039; WO 2006/046030; WO 2006/029912; WO 2005/080399; WO 2005080399; WO 2005014543; WO 200307945; WO 2003010140; WO 2003010141; WO 200204425; WO 200147883; Harper, S. A. et al. J. Med. Chem. 2005, 48, 4547; and Harper, S. A. et al. J. Med. Chem. 2005, 48, 1314, and references cited therein.

### DESCRIPTION OF THE INVENTION

The invention encompasses compounds of formula I, pharmaceutically acceptable salts thereof, compositions, and methods of treatment using these compounds.

One aspect of the invention is a compound selected from 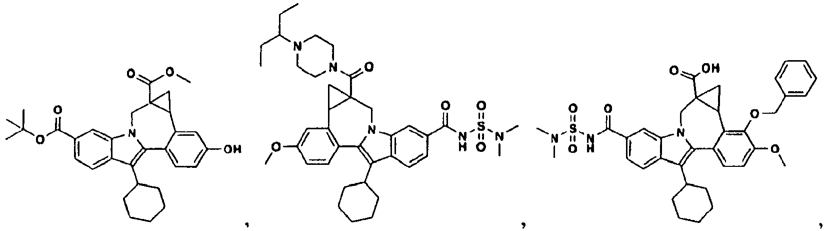 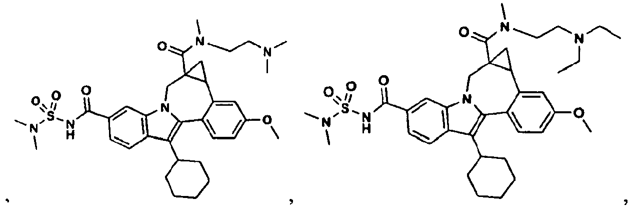 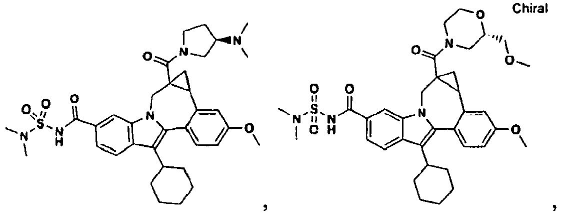 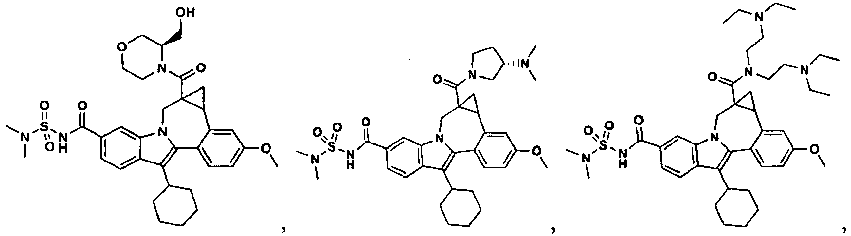 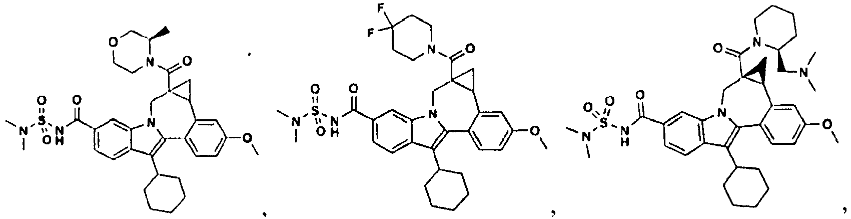 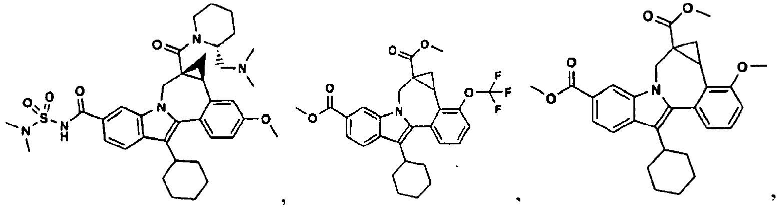 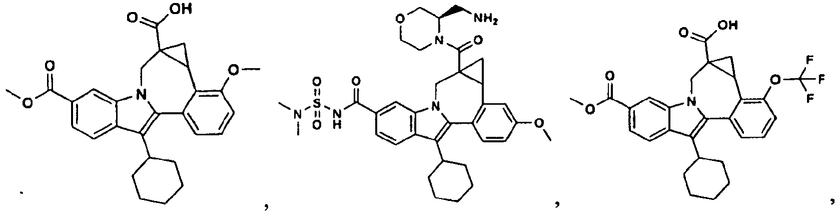 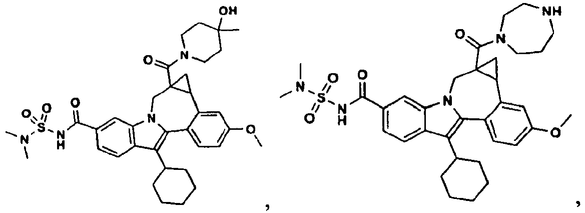 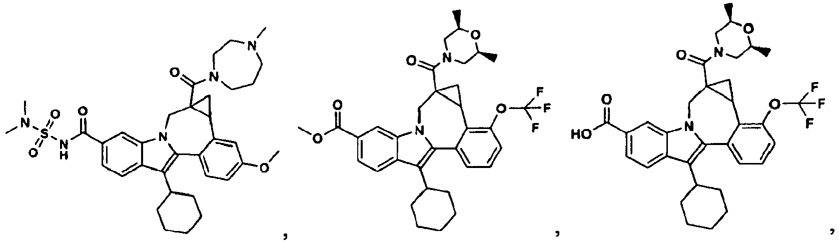 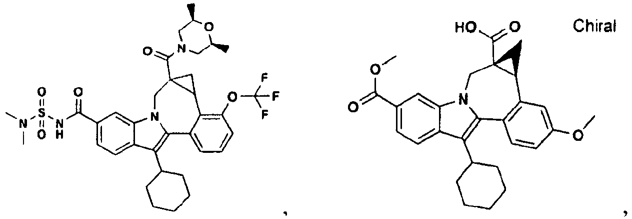 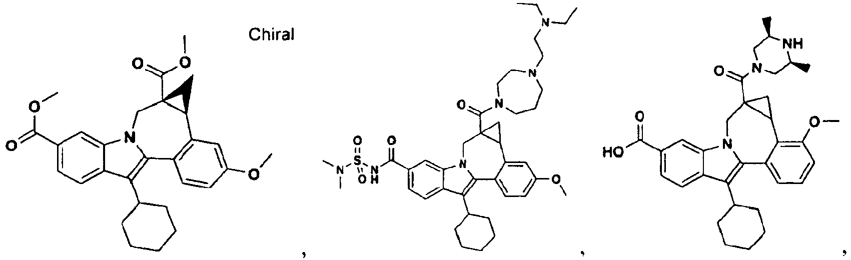 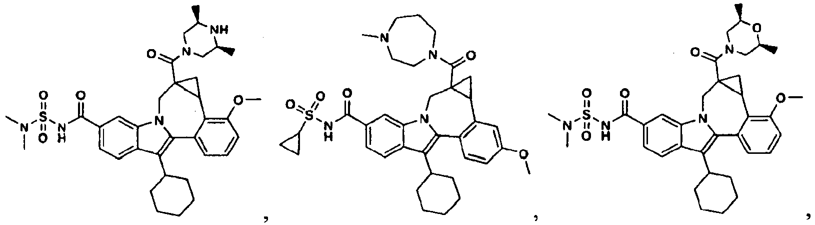 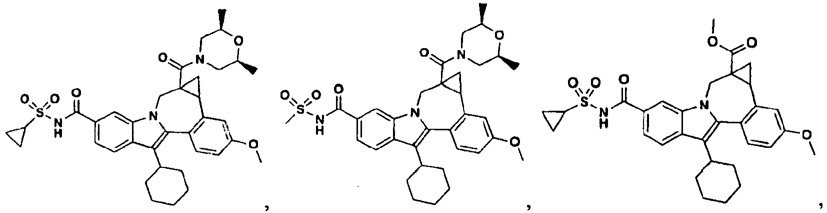 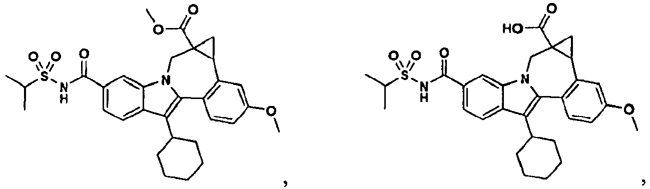 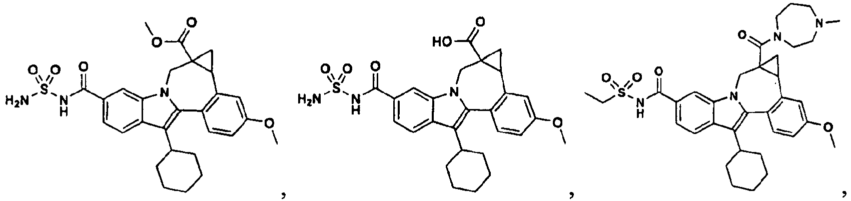 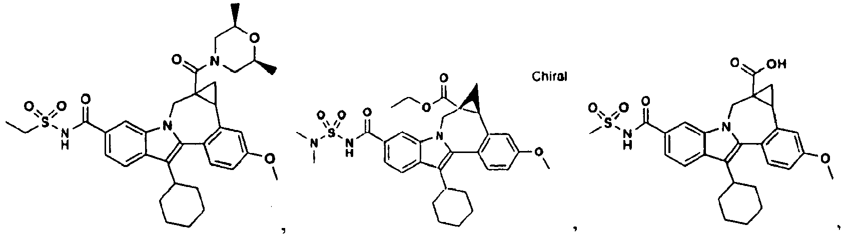 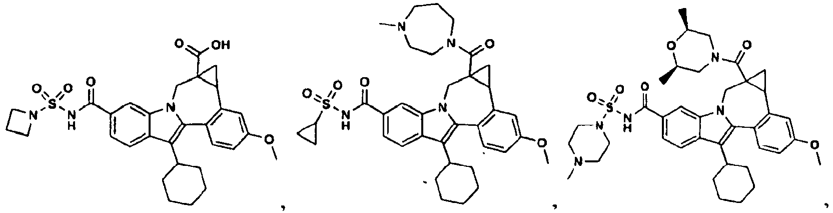 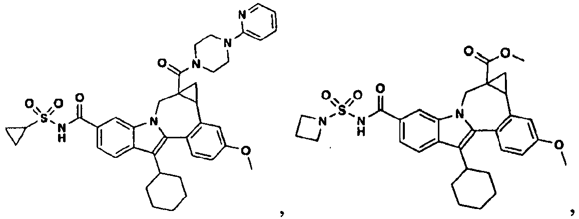 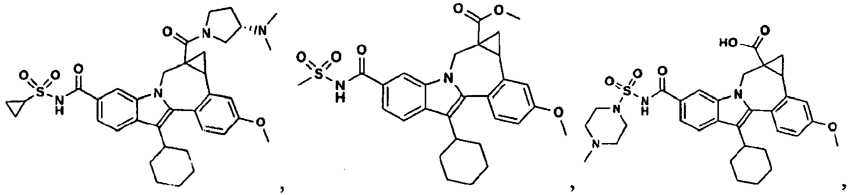 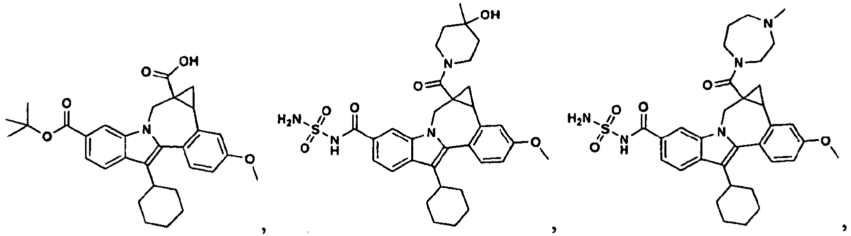 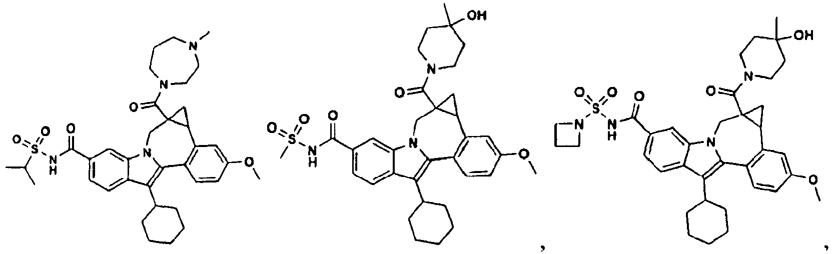 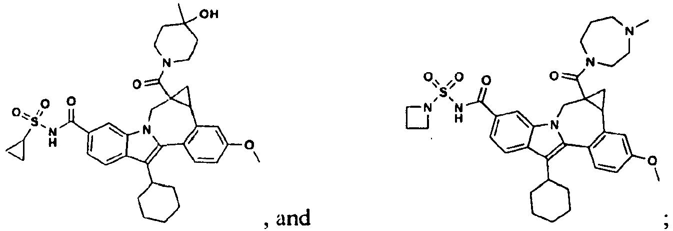 or a pharmaceutically acceptable salt thereof.

As an artifact of the software used to generate structures for some of the compounds, some compounds are missing hydrogens, especially hydrogens attached to a heteroatom. The specification should be read so as to include omitted hydrogens where one skilled in the art would reasonably understand a hydrogen would be part of the compound.

The invention includes all pharmaceutically acceptable salt forms of the compounds. Pharmaceutically acceptable salts are those in which the counter ions do not contribute significantly to the physiological activity or toxicity of the compounds and as such function as pharmacological equivalents. These salts can be made according to common organic techniques employing commercially available reagents. Some anionic salt forms include acetate, acistrate, besylate, bromide, chloride, citrate, fumarate, glucouronate, hydrobromide, hydrochloride, hydroiodide, iodide, lactate, maleate, mesylate, nitrate, pamoate, phosphate, succinate, sulfate, tartrate, tosylate, and xinofoate. Some cationic salt forms include ammonium, aluminum, benzathine, bismuth, calcium, choline, diethylamine, diethanolamine, lithium, magnesium, meglumine, 4-phenylcyclohexylamine, piperazine, potassium, sodium, tromethamine, and zinc.

Some of the compounds of the invention possess asymmetric carbon atoms (see, for example, the compound below). The invention includes all stereoisomeric forms, including enantiomers and diastereomers as well as mixtures of stereoisomers such as racemates. Some stereoisomers can be made using methods known in the art. Stereoisomeric mixtures of the compounds and related intermediates can be separated into individual isomers according to methods commonly known in the art. The use of wedges or hashes in the depictions of molecular structures in the following schemes and tables is intended only to indicate relative stereochemistry, and should not be interpreted as implying absolute stereochemical assignments.

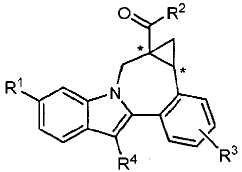

### Synthetic Methods

Formula I compounds may be made by methods known in the art including those described below. Some reagents and intermediates are known in the art. Other reagents and intermediates can be made by methods known in the art using readily available materials. The variables used to describe the synthesis of formula I compounds are intended only to illustrate how to make and are not to be confused with variables used in the claims or in other sections of the specification.

Abbreviations used within the schemes generally follow conventions used in the art. Some examples are as follows: THF means tetrahydrofuran; DMF means N,N-dimethylformamide; RCM means ring-closing methasis; Boc means tert-butoxycarbonyl; TFA means trifluoracetic acid; DMA means N,N-dimethylacetamide; PPh₃ means triphenylphosphine; OAc means acetate; Me means methyl; COD (or cod) means 1,5-cyclooctadiene; dtbpy means 4,4'-di-tert-butyl-2,2'-bipyridine; dba means dibenzylideneacetone; Xantphos means 4,5-bis(diphenylphosphino)-9,9-dimethylxanthine; aq means aqueous; EtOH means ethanol; MeOH means methanol; TBTU means 2-(1H-benzotriazole-1-yl)-1,1,3,3-tetramethyluronium tetrafluroborate; DMSO means dimethylsulfoxide; HATU means O-(7-azabenzotriazol-1-yl)-N,N,N',N'-tetramethyluronium hexafluorophosphate; EEDQ means 2-ethoxy-1-ethoxycarbonyl-1,2-dihydroquinoline; WSC means 1-[3-(dimethylamino)propyl]-3-ethylcarbodiimide hydrochloride; DMAP means 4-dimethylaminopyridine; n-Bu means n-butyl; BEMP means 2-tert-butylimino-2-diethylamino-1,3-dimethylperhydro-1,3,2-diazaphosphorine, polymer-bound; DIPEA means diisopropylethylamine; and TEA means triethylamine.

Some diester intermediates useful for the synthesis of formula I compounds may be prepared by using the general methodology depicted in Scheme 1.

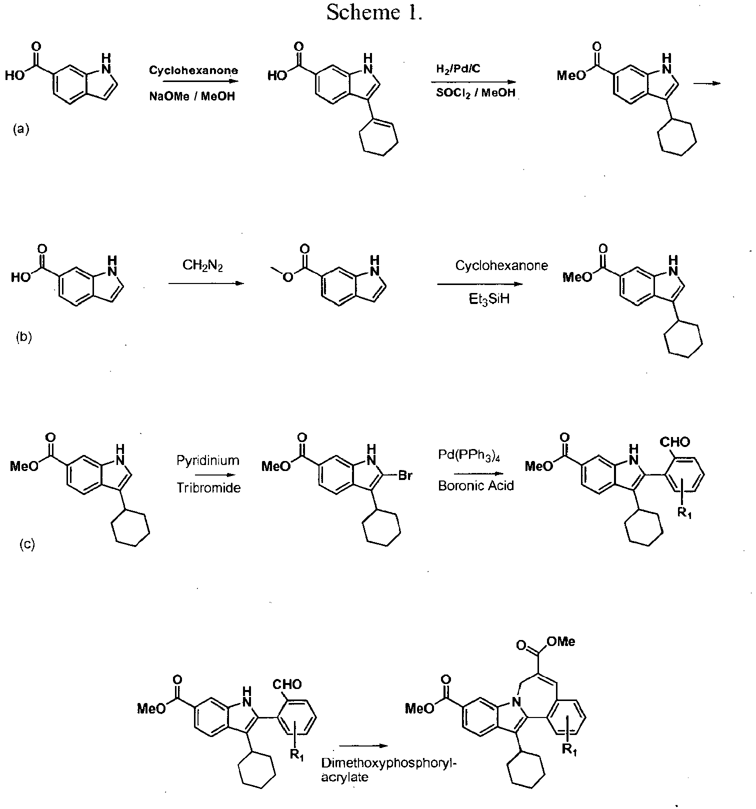

Condensation of 1H-indole-6-carboxylic acid with cyclohexanone can generate 3-cyclohexenyl-1H-indole-6-carboxylic acid. This indole ester can be subjected to sequential reduction and esterification to provide methyl 3-cyclohexanyl-1H-indole-6-carboxylate.

Alternatively, methyl 3-cyclohexanyl-1H-indole-6-carboxylate can be prepared in a two step procedure that involves an initial esterification of 1H-indole-6-carboxylic acid, for example using diazomethane in ether, followed by sequential condensation with cyclohexanone, followed by reduction.

Treatment of the resultant indole ester with pryridinium tribromide in a mixture of THF and chloroform can generate methyl 2-bromo-3-cyclohexanyl-1H-indole-6-carboxylate. This intermediate can be used in a variety of couplings, for example with 2-formyl-phenyl boronic acids using appropriate palladium catalysts, to generate the aromatic aldehyde intermediates shown. NMR analysis of this class of compound indicated that the aryl aldehydes are sometimes observed to exist in equilibrium with the related ring-closed hemiaminals, as shown below.

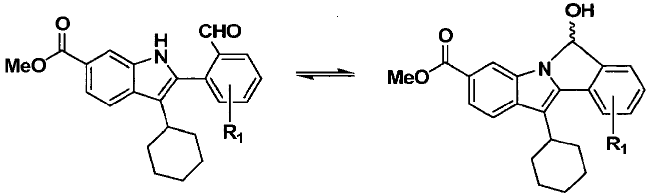

These intermediates can then be transformed into indolobenzazepine diester intermediates, for example by treating with methyl 2-(dimethoxyphosphoryl)acrylate under the influence of cesium carbonate in DMF via consecutive Michael and Horner Emmons reactions.

The resultant diester intermediates may be converted to cyclopropyl derivatives, for example as shown in Scheme 2.

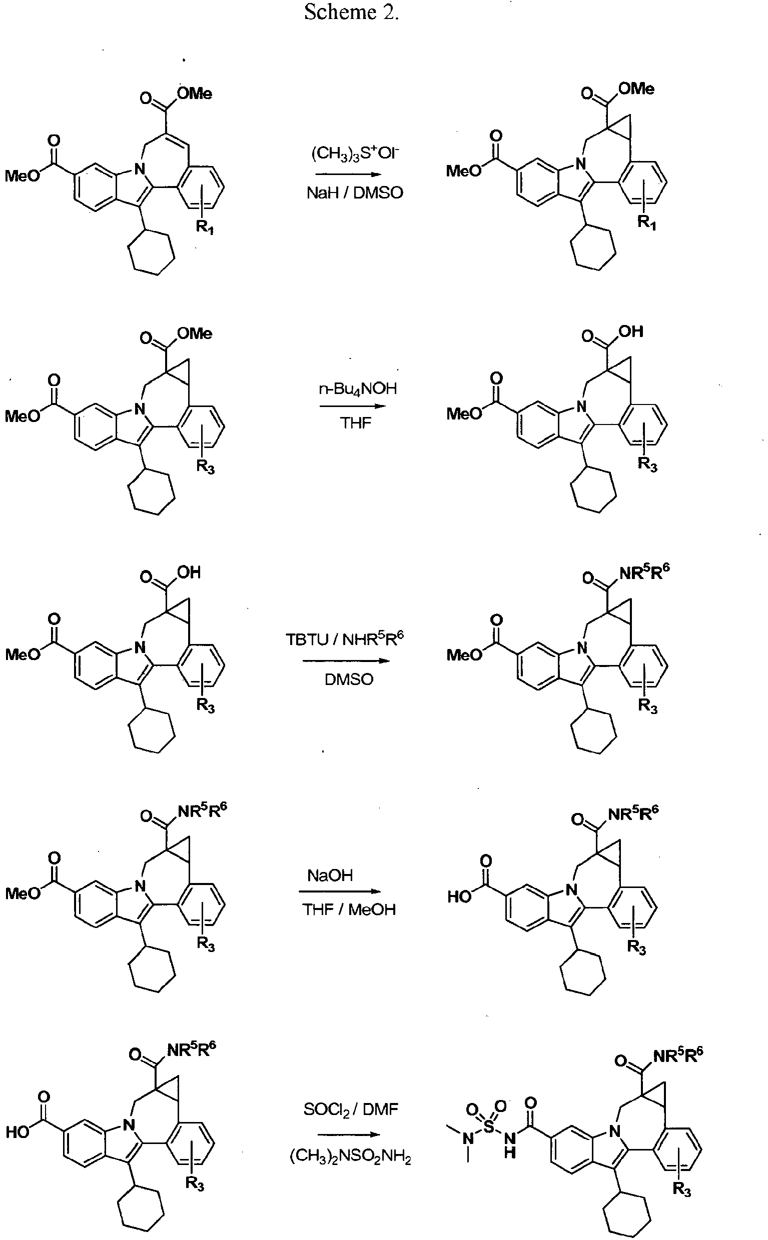

Fused cyclopropyl diester derivatives can be generated by methods known in the art including treatment of the indolobenzazepine diester intermeidates with trimethyl sulfoxonium iodide under strongly basic conditions in DMSO. The aliphatic ester moiety in these compounds can be selectively hydrolyzed using tetra-n-butylammonium hydroxide in methanol, and the resultant mono-acids can subsequently be condensed with a wide selection of primary and secondary amines to provide carboxamides depicted in the above scheme. These intermediates may be subjected to an additional hydrolysis reaction that provides the 8-cyclohexyl-cycloprop[d]indolo[2,1-a][2]benzazepine-5-carboxylic acid. Additionally, these compounds can serve as intermediates in additional coupling reactions with appropriate sulfonyl ureas that can generate acyl sulfonyl urea compounds.

In an alternative procedure, indolo[2,1-a][2]benzazepine-10-carboxylate intermediates may first be subjected to a base catalysed selective hydrolysis reaction that results in the generation of the mixed acid-ester class of compound (see Scheme 3). Subsequent coupling with amines can generate carboxamides. These intermediates can be cyclopropanated, for example by treatment with trimethylsulfoxonium iodide under basic conditions, to generate the cyclopropyl ring-fused derivatives. Subsequent hydrolysis of the remaining ester moiety can generate carboxylic acid compounds of formula I. These compounds may be converted to their corresponding acyl sulfonyl ureas derivatives.

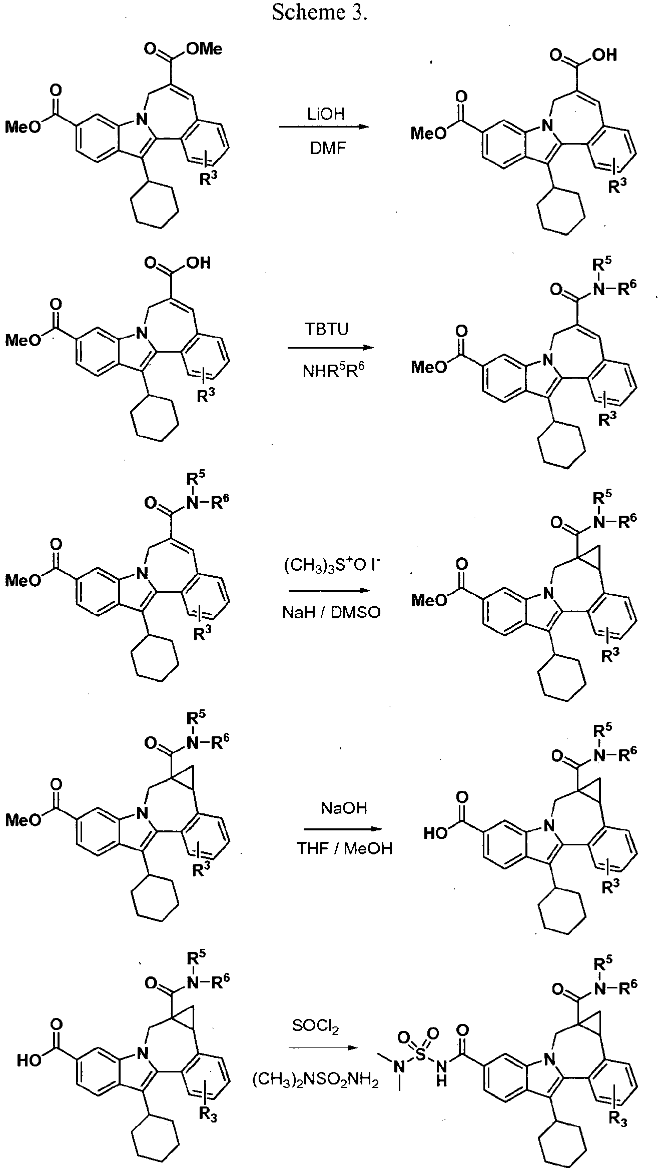

Additional methodology that can be used to make further examples is shown in scheme 4.

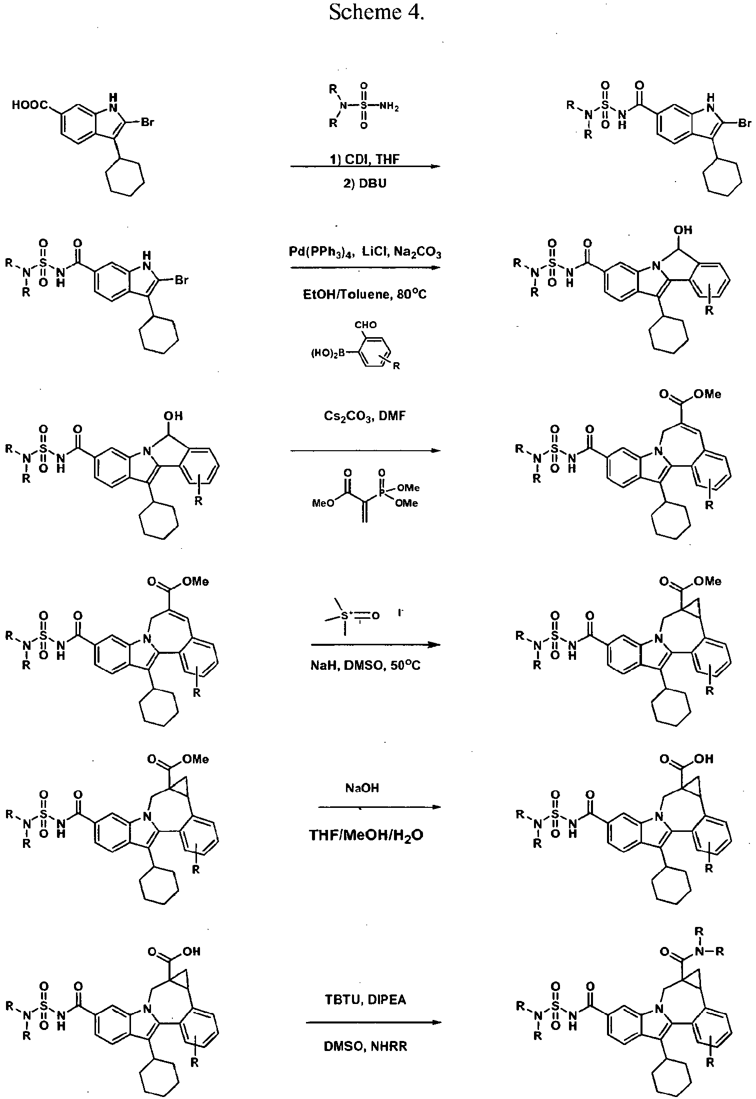

2-Bromo-3-cyclohexyl-1H-indole-6-carboxylic acid, (scheme 1) can be condensed with a variety of sulfonyl ureas, using for example, 1,1'-carbonyldiimidazole in combination with 1,8-diazabicyclo[5.4.0]undec-7-ene in anhydrous THF. The resultant acyl sulfamides can be subjected to known coupling reactions, for example with a diversity of 2-formyl boronic acids or esters using Suzuki coupling conditions, to provide cyclic hemiaminal intermediates of the type depicted. These compounds can be subsequently converted to indolobenzazepine derivatives using the sequence of reactions previously described. Related fused cyclopropyl ester derivatives can be generated by methods known in the art, including treatment of the indolobenzazepine esters with trimethyl sulfoxonium iodide under strongly basic conditions in DMSO. The residual aliphatic ester moiety in the resultant fused cyclopropanes can be hydrolyzed and the product acids can be condensed with a diversity of amines, using for example, O-(1H-benzotriazol-1-yl)-N,N, N',N'-tetramethyluronium tetrafluoroborate and diisopropyl ethyl amine in DMSO, to give examples of cyclopropyl carboxamides.

An intermediate useful for the synthesis of some further compounds involves the preparation of (+/-) 8-cyclohexyl-1,1a,2,12b-tetrahydro-11-methoxy-1a-(methoxycarbonyl)-cycloprop[d]indolo[2,1-a][2]benzazepine-5-carboxylic acid, tert-butyl ester, as shown in Scheme 5.

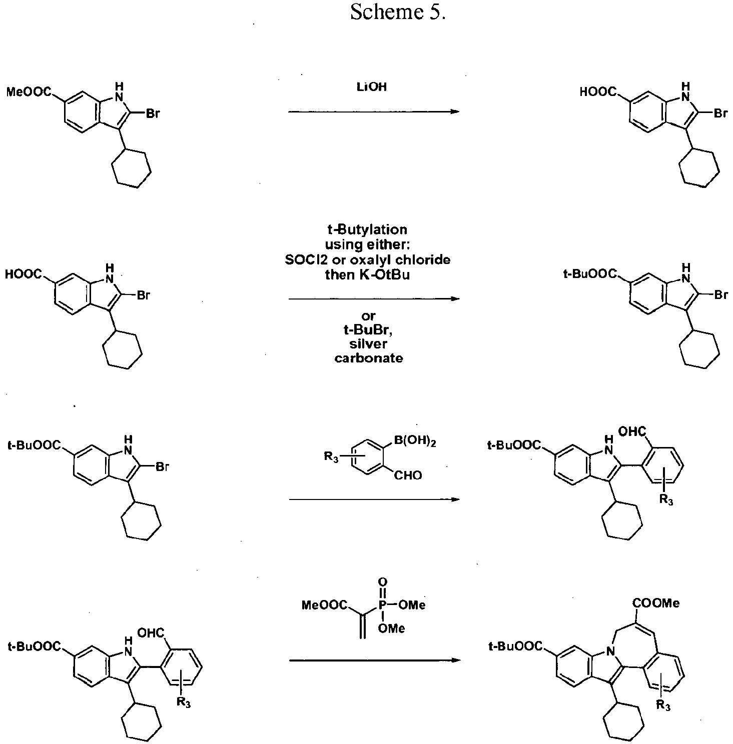

This methodology involves base catalyzed hydrolysis of the indole methyl ester shown, followed by its reaction with either thionyl chloride and potassium tertiary butoxide, or by alkylation with silver carbonate and tertiary butyl bromide. The resultant compound can be transformed using chemistry analogous to that outlined previously to provide the mixed ester indolobenzazepines shown in scheme 5.

The resultant (+/-) 8-cyclohexyl-1,1a,2,12b-tetrahydro-11-methoxy-1a-(methoxycarbonyl)-cycloprop[d]indolo[2,1-a][2]benzazepine-5-carboxylic acid, tert-butyl ester can be useful in an alternative procedure that can be employed for the preparation of acylsulfamide and acylsulfonamide compounds as shown in scheme 6.

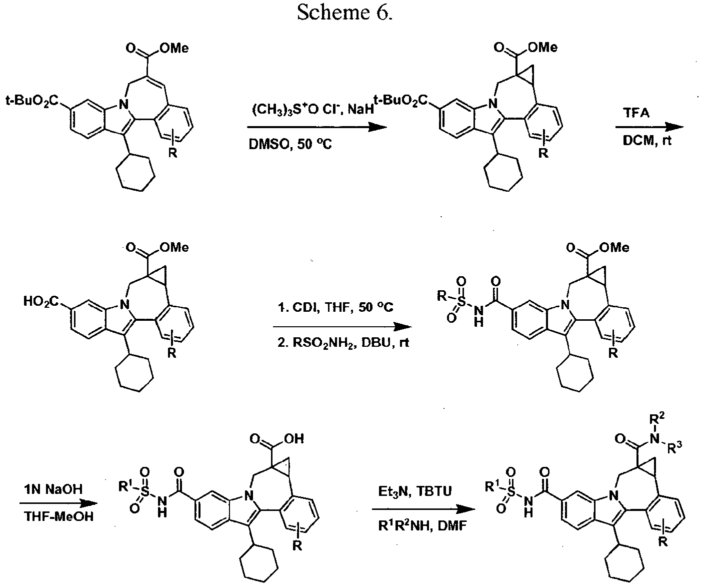

Cyclopropanation of an intermediate t-butyl ester indolobenzazepine and subsequent cleavage of the t-butyl ester group can generate the related indole acid which can be coupled to a diversity of sulfonamides and sulfonylureas. Subsequent hydrolysis of the residual ester moiety affords the related bridged acids, which can be coupled with a diversity of amines, using for example, O-(1H-benzotriazol-1-yl)-N,N, N',N'-tetramethyluronium tetrafluoroborate and diisopropyl ethyl amine in DMSO, to provide further carboxamides examples.

Some of the compounds discussed exist as mixtures of stereoisomers. The invention encompasses all stereoisomers of the compounds. Methods of isolating and separating stereoisomeric mixtures are well known in the art. One method is shown below and involves the syntheses of diastereomeric amides as shown in Scheme 7. Diastereomeric esters can also be prepared.

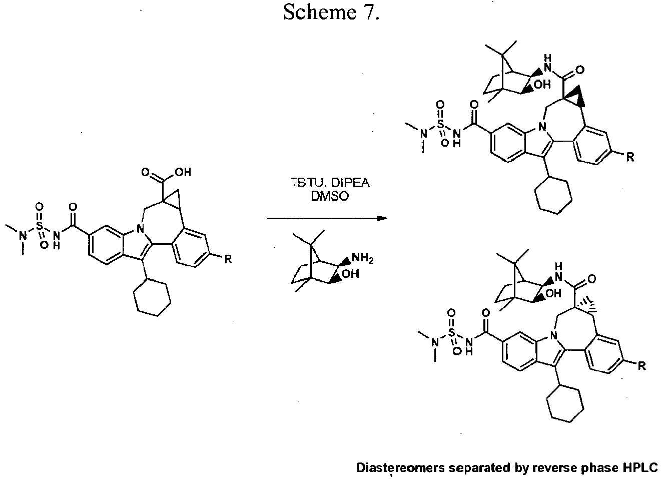

Some diastereomeric amides can be separated using reverse phase HPLC to provide optically active carboxamides. Subsequently, these compounds can be hydrolyzed and the resultant optically active acids can be coupled to a diversity of amines, using for example, O-(1H-benzotriazol-1-yl)-N,N, N',N'-tetramethyluronium tetrafluoroborate and diisopropyl ethyl amine in DMSO, to provide further examples of optically active examples, as shown in scheme 8.

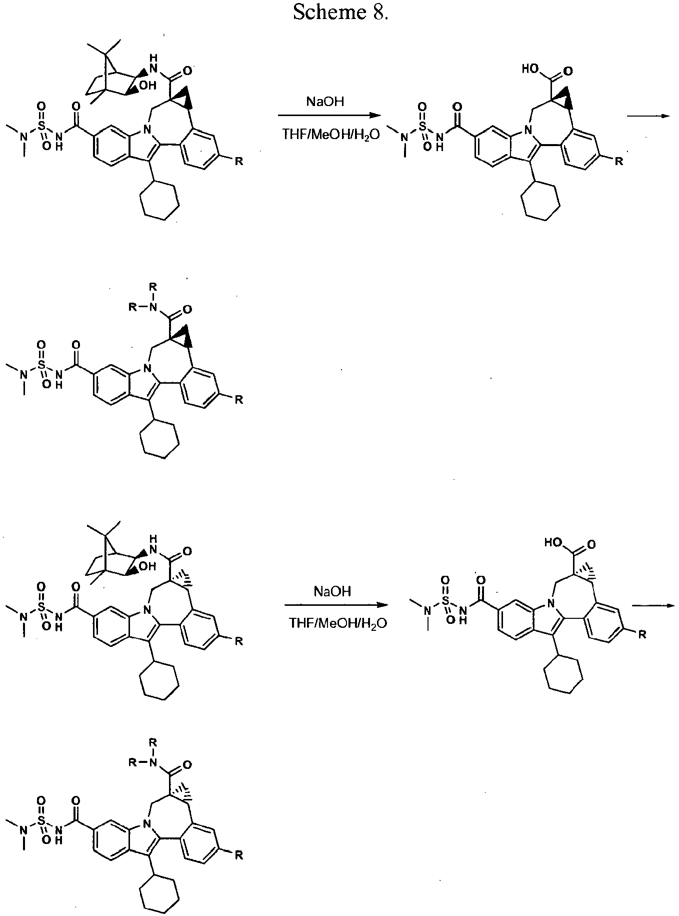

Other standard acid amine coupling methods can also be used to give optically active carboxamides.

Variation in the functionality of the aryl moiety of the fused benzazepine heterocycle compounds can be achieved as shown in scheme 1, for example by using a variety of boronic acids as coupling partners with indole bromide intermediates. Alternatively, a suitably protected reactive functionality in the aryl moiety of these intermediates can be deprotected, and can then be subsequently derivatized using methods known in the art, some examples of which are depicted in scheme 9.

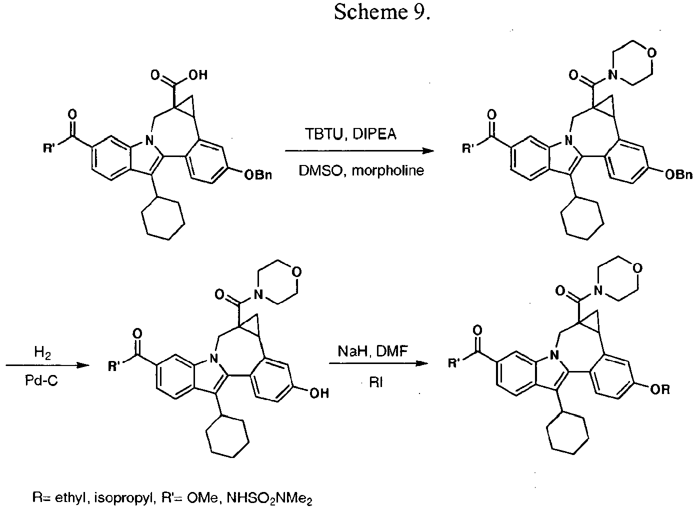

In an additional variation, the intermediate phenols depicted in the above scheme can be converted to triflate derivatives that can be used to prepare further aryl functionalized examples using a diversity of coupling reactions, some of which are outlined in scheme 10.

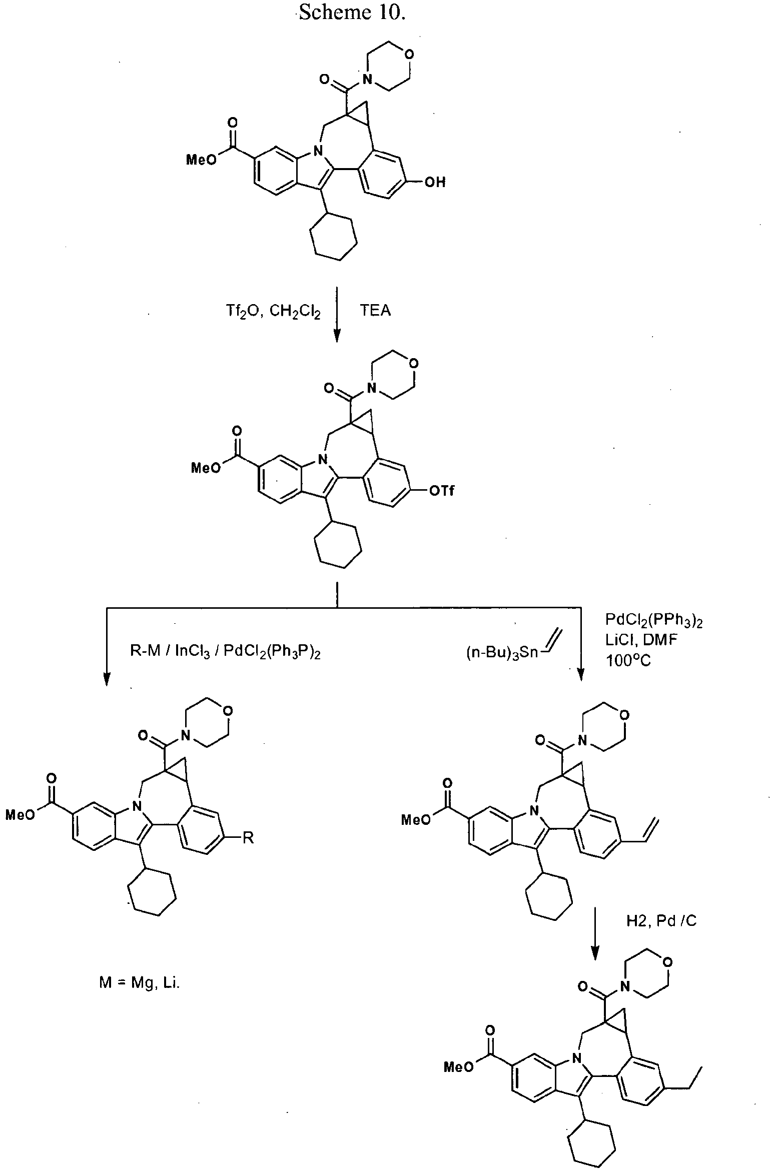

In the case of the examples shown in Scheme 10, the product esters can be hydrolyzed and subsequently coupled with a diversity of sulfonyl ureas to furnish further acyl sulfamide examples, as described previously.

### Biological Methods

Formula I compounds demonstrated activity against HCV NS5B as determined in the following HCV RdRp assays.

*HCV NS5B RdRp cloning, expression, and purification.* The cDNA encoding the NS5B protein of HCV, genotype 1b, was cloned into the pET21a expression vector. The protein was expressed with an 18 amino acid C-terminal truncation to enhance the solubility. The E. coli competent cell line BL21(DE3) was used for expression of the protein. Cultures were grown at 37 °C for ∼ 4 hours until the cultures reached an optical density of 2.0 at 600 nm. The cultures were cooled to 20 °C and induced with 1 mM IPTG. Fresh ampicillin was added to a final concentration of 50 µg/ml and the cells were grown overnight at 20 °C.

Cell pellets (3L) were lysed for purification to yield 15-24 mgs of purified NS5B. The lysis buffer consisted of 20 mM Tris-HCl, pH 7.4, 500 mM NaCl, 0.5% triton X-100, 1 mM DTT, 1mM EDTA, 20% glycerol, 0.5 mg/ml lysozyme, 10 mM MgCl2, 15 ug/ml deoxyribonuclease I, and Complete TM protease inhibitor tablets (Roche). After addition of the lysis buffer, frozen cell pellets were resuspended using a tissue homogenizer. To reduce the viscosity of the sample, aliquots of the lysate were sonicated on ice using a microtip attached to a Branson sonicator. The sonicated lysate was centrifuged at 100,000 x g for 1hr at 4 °C and filtered through a 0.2 µm filter unit (Corning).

The protein was purified using three sequential chromatography steps: Heparin sepharose CL-6B, polyU sepharose 4B, and Hitrap SP sepharose (Pharmacia). The chromatography buffers were identical to the lysis buffer but contained no lysozyme, deoxyribonuclease I, MgCl2 or protease inhibitor and the NaCl concentration of the buffer was adjusted according to the requirements for charging the protein onto the column. Each column was eluted with a NaCl gradient which varied in length from 5-50 column volumes depending on the column type. After the final chromatography step, the resulting purity of the enzyme is >90% based on SDS-PAGE analysis. The enzyme was aliquoted and stored at -80 °C.

*Standard HCV NS5B RdRp enzyme assay.* HCV RdRp genotype 1b assays were run in a final volume of 60 µl in 96 well plates (Costar 3912). The assay buffer is composed of 20 mM Hepes, pH 7.5, 2.5 mM KCl, 2.5 mM MgCl2, 1 mM DTT, 1.6 U RNAse inhibitor (Promega N2515), 0.1 mg/ml BSA (Promega R3961), and 2 % glycerol. All compounds were serially diluted (3-fold) in DMSO and diluted further in water such that the final concentration of DMSO in the assay was 2%. HCV RdRp genotype 1b enzyme was used at a final concentration of 28 nM. A polyA template was used at 6 nM, and a biotinylated oligo-dT12 primer was used at 180 nM final concentration. Template was obtained commercially (Amersham 27-4110). Biotinylated primer was prepared by Sigma Genosys. 3H-UTP was used at 0.6 µCi (0.29 µM total UTP). Reactions were initiated by the addition of enzyme, incubated at 30 °C for 60 min, and stopped by adding 25 µl of 50 mM EDTA containing SPA beads (4 µg/µl, Amersham RPNQ 0007). Plates were read on a Packard Top Count NXT after >1hr incubation at room temperature.

*Modified HCV NS5B RdRp enzyme assay.* A modified enzyme assay was performed essentially as described for the standard enzyme assay except for the following: The biotinylated oligo dT12 primer was precaptured on streptavidin-coated SPA beads by mixing primer and beads in assay buffer and incubating at room temperature for one hour. Unbound primer was removed after centrifugation. The primer-bound beads were resuspended in 20 mM Hepes buffer, pH 7.5 and used in the assay at final concentrations of 20 nM primer and 0.67 µg/µl beads. Order of addition in the assay: enzyme (14 nM) was added to diluted compound followed by the addition of a mixture of template (0.2 nM) , 3H-UTP (0.6 µCi, 0.29 µM), and primer-bound beads, to initiate the reaction; concentrations given are final. Reactions were allowed to proceed for 4 hours at 30° C.

IC₅₀ values for compounds were determined using seven different [I]. IC₅₀ values were calculated from the inhibition using the formula y = A+((B-A)/(1+((C/x)^D))).

*FRET Assay Preparation.* To perform the HCV FRET screening assay, 96-well cell culture plates were used. The FRET peptide (Anaspec, Inc.) (Taliani et al., Anal. Biochem. 1996, 240, 60-67) contains a fluorescence donor, EDANS, near one end of the peptide and an acceptor, DABCYL, near the other end. The fluorescence of the peptide is quenched by intermolecular resonance energy transfer (RET) between the donor and the acceptor, but as the NS3 protease cleaves the peptide the products are released from RET quenching and the fluorescence of the donor becomes apparent. The assay reagent was made as follows: 5X cell Luciferase cell culture lysis reagent from Promega (#E153A) diluted to 1X with dH₂O, NaCl added to 150 mM final, the FRET peptide diluted to 20 uM final from a 2 mM stock.

To prepare plates, HCV replicon cells, with or without a Renilla luciferase reporter gene, were trypsinized and placed into each well of a 96-well plate with titrated test compounds added in columns 3 through 12; columns 1 and 2 contained a control compound (HCV protease inhibitor), and the bottom row contained cells without compound. The plates were then placed in a CO₂ incubator at 37 °C.

*Assays.* Subsequent to addition of the test compounds described above (FRET Assay Preparation), at various times the plate was removed and Alamar blue solution (Trek Diagnostics, #00-100) was added per well as a measure of cellular toxicity. After reading in a Cytoflour 4000 instrument (PE Biosystems), plates were rinsed with PBS and then used for FRET assay by the addition of 30 ul of the FRET peptide assay reagent described above (FRET Assay Preparation) per well. The plate was then placed into the Cytoflour 4000 instrument which had been set to 340 excite/490 emission, automatic mode for 20 cycles and the plate read in a kinetic mode. Typically, the signal to noise using an endpoint analysis after the reads was at least three-fold. Alternatively, after Alamar blue reading, plates were rinsed with PBS, 50 ul of DMEM (high glucose) without phenol red was added and plates were then used for luciferase assay using the Promega Dual-Glo Luciferase Assay System.

Compound analysis was determined by quantification of the relative HCV replicon inhibition and the relative cytotoxicity values. To calculate cytoxicity values, the average Alamar Blue fluorescence signals from the control wells were set as 100% non-toxic. The individual signals in each of the compound test wells were then divided by the average control signal and multiplied by 100% to determine percent cytotoxicity. To calculate the HCV replicon inhibition values, an average background value was obtained from the two wells containing the highest amount of HCV protease inhibitor at the end of the assay period. These numbers were similar to those obtained from naïve Huh-7 cells.

The background numbers were then subtracted from the average signal obtained from the control wells and this number was used as 100% activity. The individual signals in each of the compound test wells were then divided by the averaged control values after background subtraction and multiplied by 100% to determine percent activity. EC₅₀ values for a protease inhibitor titration were calculated as the concentration which caused a 50% reduction in FRET or luciferase activity. The two numbers generated for the compound plate, percent cytoxicity and percent activity were used to determine compounds of interest for further analysis.

Representative data for compounds of the invention are reported in Table 1.

**Table 1.**

| Structure | IC₅₀ | EC₅₀ |
|---|---|---|
| 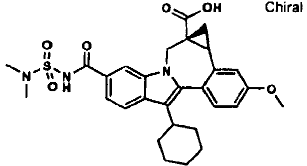 | B | D |
| 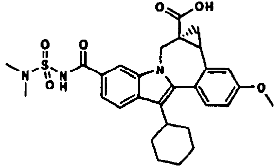 | B | D |
| 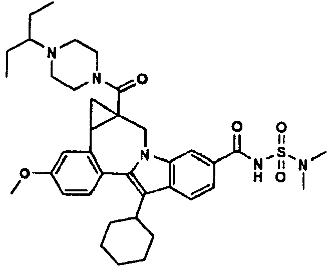 | B | E |
| 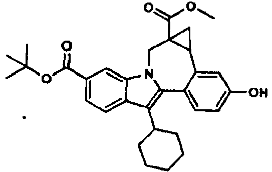 | D | A |
| 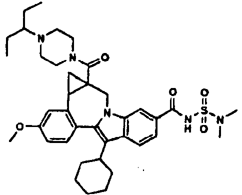 | B | B |
| 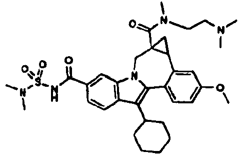 | B | B |
| 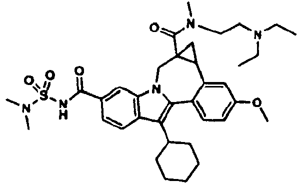 | B | B |
| 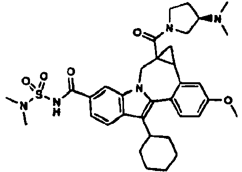 | B | B |
| 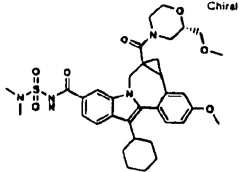 | B | B |
| 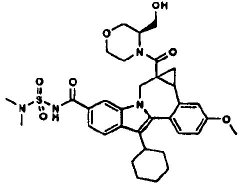 | B | B |
| 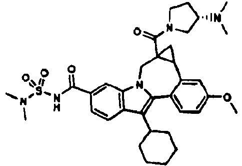 | B | B |
| 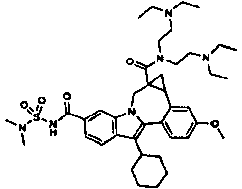 | B | B |
| 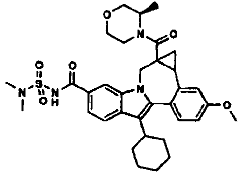 | B | B |
| 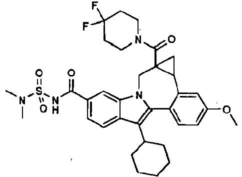 | B | B |
| 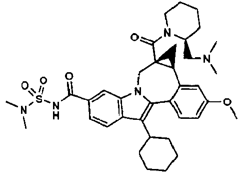 | B | B |
| 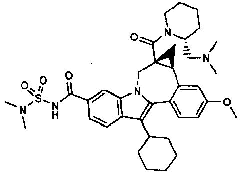 | B | B |
| 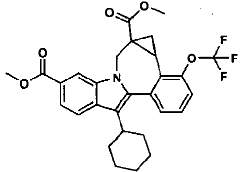 | C | F |
| 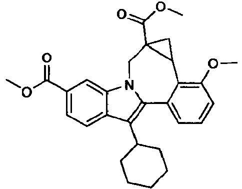 | | |
| 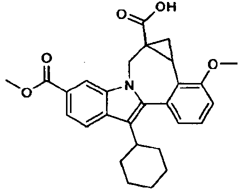 | | |
| 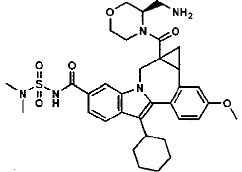 | B | B |
| 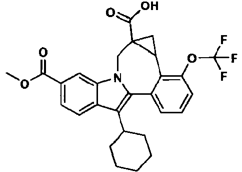 | D | |
| 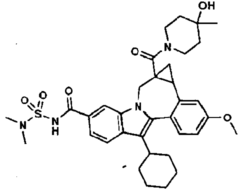 | B | B |
| 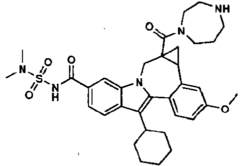 | B | B |
| 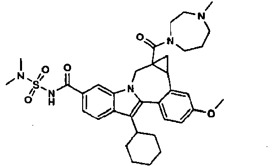 | B | B |
| 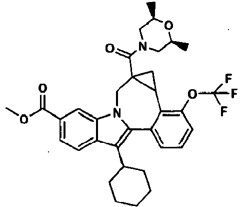 | D | |
| 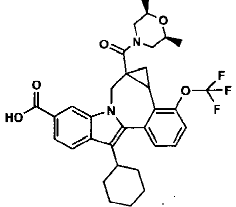 | B | B |
| 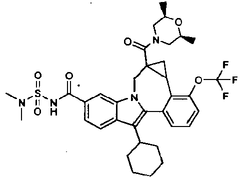 | B | B |
| 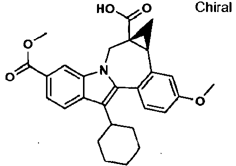 | | |
| 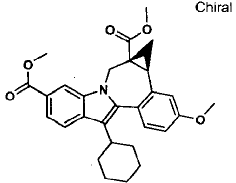 | | |
| 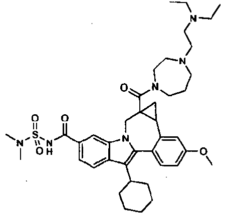 | B | B |
| 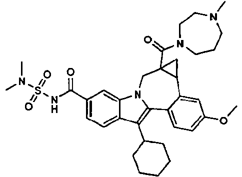 | B | B |
| 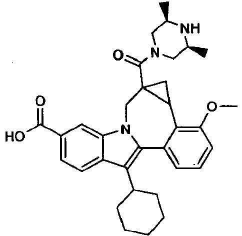 | | |
| 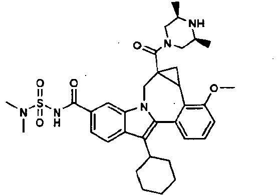 | B | B |
| 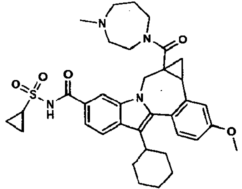 | B | B |
| 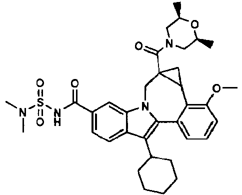 | B | B |
| 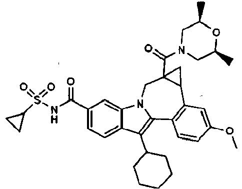 | B | B |
| 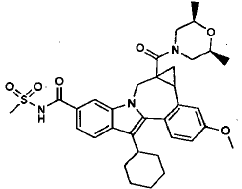 | B | B |
| 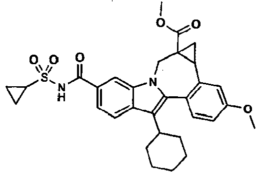 | | |
| 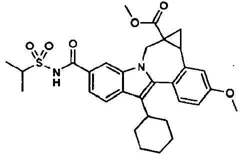 | B | |
| 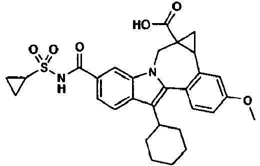 | B | |
| 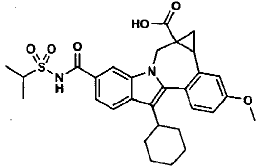 | B | |
| 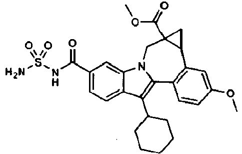 | B | |
| 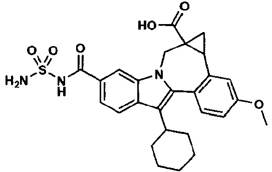 | B | |
| 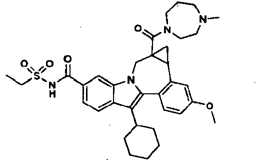 | | |
| 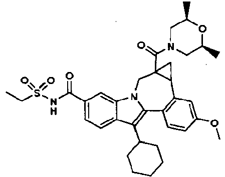 | | |
| 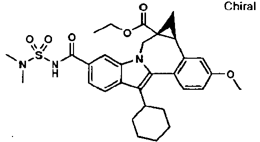 | | |
| 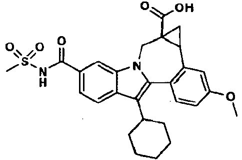 | | |
| 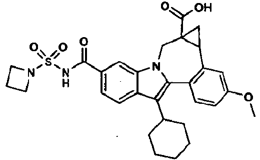 | | |
| 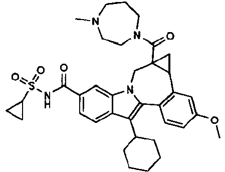 | | |
| 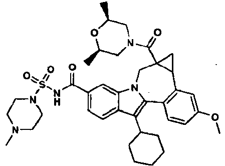 | | |
| 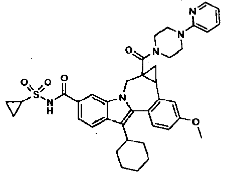 | B | B |
| 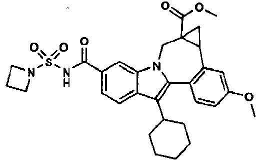 | | |
| 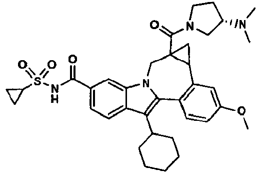 | B | B |
| 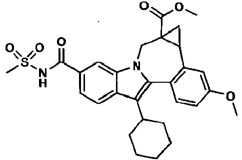 | | |
| 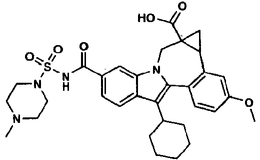 | | |
| 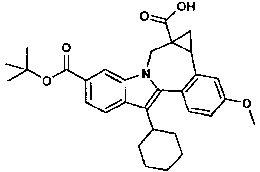 | D | |
| 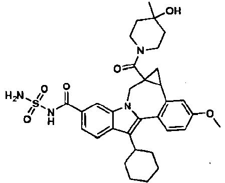 | B | B |
| 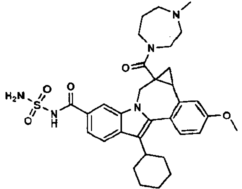 | B | B |
| 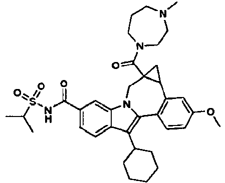 | B | F |
| 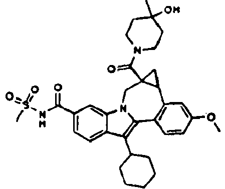 | B | B |
| 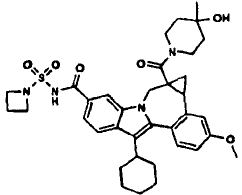 | B | B |
| 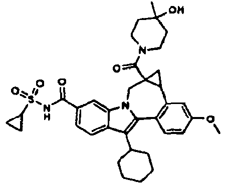 | B | |
| 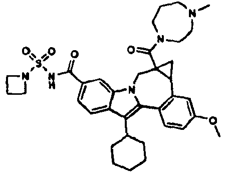 | B | |

| | | |
|---|---|---|
| IC₅₀ A>1 µM; B 0.097 µM - 1 µM; EC₅₀: C > 10µM; D 1 µM - 10 µM; E 1.0 µM - 0.02 µM; F >0.12 µM. IC₅₀ values were determined using the preincubation protocol. EC₅₀ values were determined using the FRET assay. | | |

### Pharmaceutical Compositions and Methods of Treatment

Formula I compounds demonstrate activity against HCV NS5B and can be useful in treating HCV and HCV infection. Therefore, another aspect of the invention is a composition comprising a compound of formula I, or a pharmaceutically acceptable salt thereof, and a pharmaceutically acceptable carrier.

Another aspect of the invention is a composition further comprising a compound having anti-HCV activity.

Another aspect of the invention is a composition where the compound having anti-HCV activity is an interferon. Another aspect of the invention is where the interferon is selected from interferon alpha 28, pegylated interferon alpha, consensus interferon, interferon alpha 2A, and lymphoblastoid interferon tau.

Another aspect of the invention is a composition where the compound having anti-HCV activity is a cyclosporin. Another aspect of the invention is where the cyclosporin is cyclosporin A.

Another aspect of the invention is a composition where the compound having anti-HCV activity is selected from the group consisting of interleukin 2, interleukin 6, interleukin 12, a compound that enhances the development of a type I helper T cell response, interfering RNA, anti-sense RNA, Imiqimod, ribavirin, an inosine 5'-monophospate dehydrogenase inhibitor, amantadine, and rimantadine.

Another aspect of the invention is a composition where the compound having anti-HCV activity is effective to inhibit the function of a target selected from HCV metalloprotease, HCV serine protease, HCV polymerase, HCV helicase, HCV NS4B protein, HCV entry, HCV assembly, HCV egress, HCV NS5A protein, IMPDH, and a nucleoside analog for the treatment of an HCV infection.

Another aspect of the invention is a composition comprising a compound of formula I, or a pharmaceutically acceptable salt thereof, a pharmaceutically acceptable carrier, an interferon and ribavirin.

Another aspect of the invention is a compound of the invention for use in a method of inhibiting the function of the HCV replicon comprising contacting the HCV replicon with a compound of formula I or a pharmaceutically acceptable salt thereof.

Another aspect of the invention is a compound of the invention for use in a method of inhibiting the function of the HCV NS5B protein comprising contacting the HCV NS5B protein with a compound of formula I or a pharmaceutically acceptable salt thereof.

Another aspect of the invention is a compound of the invention for use in a method of treating an HCV infection in a patient comprising administering to the patient a therapeutically effective amount of a compound of formula I or a pharmaceutically acceptable salt thereof. In another embodiment the compound is effective to inhibit the function of the HCV replicon.

In another embodiment the compound is effective to inhibit the function of the HCV NS5B protein.

Another aspect of the invention is a compound of the invention for use in a method of treating an HCV infection in a patient comprising administering to the patient a therapeutically effective amount of a compound of formula I, or a pharmaceutically acceptable salt thereof, in conjunction with (prior to, after, or concurrently) another compound having anti-HCV activity.

Another aspect of the invention is the method where the other compound having anti-HCV activity is an interferon.

Another aspect of the invention is the method where the interferon is selected from interferon alpha 2B, pegylated interferon alpha, consensus interferon, interferon alpha 2A, and lymphoblastoid interferon tau.

Another aspect of the invention is the method where the other compound having anti-HCV activity is a cyclosporin.

Another aspect of the invention is the method where the cyclosporin is cyclosporin A.

Another aspect of the invention is the method where the other compound having anti-HCV activity is selected from interleukin 2, interleukin 6, interleukin 12, a compound that enhances the development of a type 1 helper T cell response, interfering RNA, anti-sense RNA, Imiqimod, ribavirin, an inosine 5'-monophospate dehydrogenase inhibitor, amantadine, and rimantadine.

Another aspect of the invention is the method where the other compound having anti-HCV activity is effective to inhibit the function of a target selected from the group consisting of HCV metalloprotease, HCV serine protease, HCV polymerase, HCV helicase, HCV NS4B protein, HCV entry, HCV assembly, HCV egress, HCV NS5A protein, IMPDH, and a nucleoside analog for the treatment of an HCV infection.

Another aspect of the invention is the method where the other compound having anti-HCV activity is effective to inhibit the function of target in the HCV life cycle other than the HCV NS5B protein.

"Therapeutically effective" means the amount of agent required to provide a meaningful patient benefit as understood by practitioners in the field of hepatitis and HCV infection.

"Patient" means a person infected with the HCV virus and suitable for therapy as understood by practitioners in the field of hepatitis and HCV infection.

"Treatment," "therapy," "regimen," "HCV infection," and related terms are used as understood by practitioners in the field of hepatitis and HCV infection.

The compounds of this invention are generally given as pharmaceutical compositions comprised of a therapeutically effective amount of a compound of Formula I or its pharmaceutically acceptable salt and a pharmaceutically acceptable carrier and may contain conventional excipients. A therapeutically effective amount is that which is needed to provide a meaningful patient benefit. Pharmaceutically acceptable carriers are those conventionally known carriers having acceptable safety profiles. Compositions encompass all common solid and liquid forms including capsules, tablets, losenges, and powders as well as liquid suspensions, syrups, elixers, and solutions. Compositions are made using common formulation techniques, and conventional excipients (such as binding and wetting agents) and vehicles (such as water and alcohols) are generally used for compositions.

Solid compositions are normally formulated in dosage units and compositions providing from about 1 to 1000 mg of the active ingredient per dose are preferred. Some examples of dosages are 1 mg, 10 mg, 100 mg, 250 mg, 500 mg, and 1000 mg. Generally, other agents will be present in a unit range similar to agents of that class used clinically. Typically, this is 0.25-1000 mg/unit.

Liquid compositions are usually in dosage unit ranges. Generally, the liquid composition will be in a unit dosage range of 1-100 mg/mL. Some examples of dosages are 1 mg/mL, 10 mg/mL, 25 mg/mL, 50 mg/mL, and 100 mg/mL. Generally, other agents will be present in a unit range similar to agents of that class used clinically. Typically, this is 1-100 mg/mL.

The invention encompasses all conventional modes of administration; oral and parenteral methods are preferred. Generally, the dosing regimen will be similar to other agents used clinically. Typically, the daily dose will be 1-100 mg/kg body weight daily. Generally, more compound is required orally and less parenterally. The specific dosing regime, however, will be determined by a physician using sound medical judgement.

The invention also encompasses methods where the compound is given in combination therapy. That is, the compound can be used in conjunction with, but separately from, other agents useful in treating hepatitis and HCV infection. In these combination methods, the compound of Formula I will generally be given in a daily dose of 1-100 mg/kg body weight daily in conjunction with other agents. The other agents generally will be given in the amounts used therapeutically. The specific dosing regime, however, will be determined by a physician using sound medical judgement.

Some examples of compounds suitable for compositions and methods are listed in Table 3.

**Table 3.**

| Brand Name | Type of Inhibitor or Target | Source Company |
|---|---|---|
| Omega IFN | IFN-ω | BioMedicines Inc., Emeryville, CA |
| BILN-2061 | serine protease inhibitor | Boehringer Ingelheim Pharma KG, Ingelheim, Germany |
| Summetrel | antiviral | Endo Pharmaceuticals Holdings Inc., Chadds Ford, PA |
| Roferon A | IFN-α2a | F. Hoffmann-La Roche LTD, Basel, Switzerland |
| Pegasys | PEGylated IFN-α2a | F. Hoffmann-La Roche LTD, Basel, Switzerland |
| Pegasys and Ribavirin | PEGylated IFN-α2a/ribavirin | F. Hoffmann-La Roche LTD, Basel, Switzerland |
| CellCept | HCV IgG immunosuppressant | F. Hoffmann-La Roche LTD, Basel, Switzerland |
| Wellferon | lymphoblastoid IFN-αn1 | GlaxoSmithKline plc, Uxbridge, UK |
| Albuferon - α | albumin IFN-α2b | Human Genome Sciences Inc., Rockville, MD |
| Levovirin | ribavirin | ICN Pharmaceuticals, Costa Mesa, CA |
| IDN-6556 | caspase inhibitor | Idun Pharmaceuticals Inc., San Diego, CA |
| IP-501 | antifibrotic | Indevus Pharmaceuticals Inc., Lexington, MA |
| Actimmune | INF-γ | InterMune Inc., Brisbane, CA |
| Infergen A | IFN alfacon-1 | InterMune Pharmaceuticals Inc., Brisbane, CA |
| ISIS 14803 | antisense | ISIS Pharmaceuticals Inc, Carlsbad, CA/Elan Pharmaceuticals Inc., New York, NY |
| JTK-003 | RdRp inhibitor | Japan Tobacco Inc., Tokyo, Japan |
| Pegasys and Ceplene | PEGylated IFN-α2a/ immune modulator | Maxim Pharmaceuticals Inc., San Diego, CA |
| Ceplene | immune modulator | Maxim Pharmaceuticals Inc., San Diego, CA |
| Civacir | HCV IgG immunosuppressant | Nabi Biopharmaceuticals Inc., Boca Raton, FL |
| Intron A and Zadaxin | IFN-α2b/α1-thymosin | RegeneRx Biopharmiceuticals Inc., Bethesda, MD/ SciClone Pharmaceuticals Inc, San Mateo, CA |
| Levovirin | IMPDH inhibitor | Ribapharm Inc., Costa Mesa, CA |
| Viramidine | IMPDH inhibitor | Ribapharm Inc., Costa Mesa, CA |
| Heptazyme | ribozyme | Ribozyme Pharmaceuticals Inc., Boulder, CO |
| Intron A | IFN-α2b | Schering-Plough Corporation, Kenilworth, NJ |
| PEG-Intron | PEGylated IFN-α2b | Schering-Plough Corporation, Kenilworth, NJ |
| Rebetron | IFN-α2b/ribavirin | Schering-Plough Corporation, Kenilworth, NJ |
| Ribavirin | ribavirin | Schering-Plough Corporation, Kenilworth, NJ |
| PEG-Intron / Ribavirin | PEGylated IFN-α2b/ribavirin | Schering-Plough Corporation, Kenilworth, NJ |
| Zadazim | immune modulator | SciClone Pharmaceuticals Inc., San Mateo, CA |
| Rebif | IFN-β1a | Serono, Geneva, Switzerland |
| IFN-β and EMZ701 | IFN-β and EMZ701 | Transition Therapeutics Inc., Ontario, Canada |
| T67 | β-tubulin inhibitor | Tularik Inc., South San Francisco, CA |
| VX-497 | IMPDH inhibitor | Vertex Pharmaceuticals Inc., Cambridge, MA |
| VX-950/LY-570310 | serine protease inhibitor | Vertex Pharmaceuticals Inc., Cambridge, MA/ Eli Lilly and Co. Inc., Indianapolis, IN |
| Omniferon | natural IFN-α | Viragen Inc., Plantation, FL |
| XTL-002 | monoclonal antibody | XTL Biopharmaceuticals Ltd., Rehovot, Isreal |

### Description of Specific Embodiments

Formula I compounds illustrated in the preceding schemes can generally be purified by reverse phase chromatography using a preparative C-18 column employing gradients of methanol - water containing 0.1% of trifluoroacetic acid (TFA), and using a Shimadzu High Perfomance Liquid Preparative Chromatographic System employing an XTERRA 30 x 100 mm S5 column at 40 mL/min flow rate with a 12 min gradient. An Emrys Optimizer personal microwave reactor was used for the microwave assisted reactions. Molecular weights and purities were usually determined using a Shimadzu LCMS using a Phenomenex-Luna 3.0 x 50mm S 10 reverse phase column employing a flow rate of 4 mL min using a 0.1%TFA in methanol / H₂O gradient [0-100% in 2 min, with 3 min run time]. NMR spectra were usually obtained on either a Bruker 500 or 300 MHz instrument. The preparative silicic acid plates were 20 x20 cm with a 1000 micron layer of silica gel GF.

### Intermediate 1

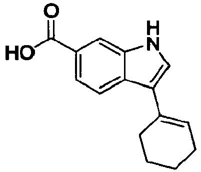

*3-Cyclohexenyl-1H-indole-6-carboxylic acids.* Cyclohexanone (96 mL, 0.926 mol) was added to a stirred solution of methyl indole-6-carboxylic acid (50.0 g, 0.335 mol) in methanol (920 mL) at 22 °C. Methanolic sodium methoxide (416 mL of 25% w/w, 1.82 mol) was added in portions over 10 minutes. The mixture was stirred at reflux for 18 hours, cooled to room temperature, concentrated, diluted with cold water, and acidified with 36% HCl solution. The resulting precipitate was collected by filtration, washed with cold water, and dried over phosphorous pentoxide (0.1 mm) to provide the the title compound as a tan colored solid (80.9 g, 97.5% yield).

### Intermediate 2

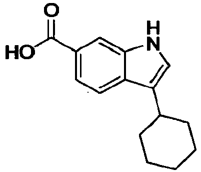

*3-Cyclohexyl-1H-indole-6-carboxylic cacid.* 3-Cyclohexenyl-1H-indole-6-carboxylic acid (38 g) was added to a Parr bottle, followed by methanol (100 mL) and THF (100 mL). The bottle was flushed with argon and 10% palladium on carbon (1.2 g) was added. The flask was then evacuated and subsequently refilled with H₂ to a pressure of 55 psi, and the resultant mixture was shaken for 18 hours at RT. The catalyst was then removed by filtration through celite. Concentration of the filtrate provided the desired product as a pale purple solid (30.6 g, 79%). ESI-MS *m*/*z* 244 (MH⁺).

### Intermediate 3

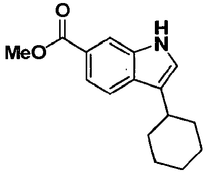

*Methyl 3-cyclohexyl-1H-indole-6-carboxylate.* Thionyl chloride (1 mL) was added to a stirred mixture of 3-cyclohexyl-1H-indole-6-carboxylic acid (30.4 g, 0.125 mol) in methanol (300 mL). The mixture was stirred at reflux for 18 hours, treated with decolorizing carbon, and filtered. The filtrate was concentrated to about 150 mL at which point crystallization occurred. The filtrate was cooled to room temperature and filtered. The solid was washed with cold methanol followed by diethyl ether to provide the desired product as a pale purple solid (22.2 g, 69% yield). ESI-MS *m*/*z* 258 (MH⁺); ¹H NMR (300 MHz, CDCl₃) δ 1.35 (m, 4H), 1.63 (s, 1H), 1.78 (m, 3H), 2.06 (d, *J*=8.05 Hz, 2H, 3.90 (m, 1H), 7.08 (d, *J*=1.83 Hz, 1H), 7.62 (s, 1H), 7.65 (s, 1H),7.74 (d, *J*=1.46 Hz, 1H), 7.77 (d, *J*=1.46 Hz, 1H), 8.08 (s, 1H).

### Intermediate 4

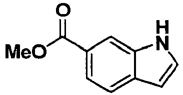

*Methyl 1H-indole-6-carboxylate.* An ethereal solution of diazomethane (620 mL) was added slowly to a cooled, (-15°C) stirred suspension of 6-indole carboxylic acid (45 g, 0.27 mol.) in diethyl ether (250 mL). Upon addition, the reaction mixture was stirred for a further 1h at-15°C, after which the reaction was quenched by the slow addition of acetic acid (50 mL). The resultant mixture was then concentrated under reduced pressure, and the residue purified using flash chromatography on silica (60 - 120), using DCM as eluant.

### Intermediate 5

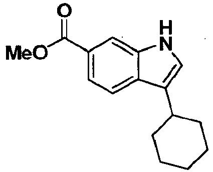

*Methyl 3-cyclohexyl-1H-indole-6-carboxylate.* Cyclohexanone (42.46 mL, 0.40 mol) was added in a single portion to a stirred solution of methyl indole-6-carboxylate (47.8 g, 0.27 m) in dry dichloromethane (500 mL). The reaction mixture was then cooled to 10°C and trifluoroacetic acid (63.13 mL, 0.8 m) was added dropwise followed by triethyl silane (++174.5 mL, 1.09 m). Upon addition, the temperature was allowed to rise to rt, after which it was stirred for a further 12 h. Dichloromethane (200 mL) was then added and the reaction mixture was washed successively with with 10% sodium bicarbonate solution and brine. The organic layer dried over sodium sulfate, filtered and concentrated under vacuum. The resultant residuce was purified by flash chromatography on silica (60 - 120) using hexane - ethyl acetate (9.5:0.5) mixture as eluant. Homogeneous fractions were combined and evaporated to give 60 g of the desired product (85%). Analytical data on this material was consistant with that observed with a sample prepared by the alternative route described above.

### Intermediate 6

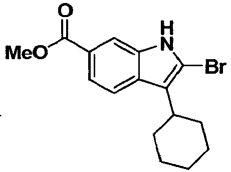

*Methyl 2-bromo-3-cyclohexyl-2-1H-indole-6-carboxylate.* Dry pyridinium tribromide (12.0 g, 38 mmol) was added in one portion to a stirred and cooled (ice/water bath) solution of methyl 3-cyclohexyl-1H-indole-6-carboxylate (7.71 g, 30 mmol) in a mixture of THF (80 mL) and chloroform (80 mL). The flask was removed from the cooling bath and stirring was continued for 2 hours at room temperature. The mixture was sequentially washed with 1M NaHSO₃ (2 x 50 mL) and1N HCl (50 mL). It was then dried over anhydrous sodium sulfate, filtered, and concentrated. The concentrate was treated with hexanes and the resulting precipitate was collected by filtration to provide the desired product as an off-white solid (5.8 g, 58%). ¹H NMR(300 MHz, CDCl₃) δ 1.38 (m, 3H), 1.85 (m, 7H), 2.81 (m, 1H), 7.71 (m, 2H), 8.03 (s, 1H), 8.47 (s, 1H).

The hexane mother liquor was concentrated and the residue was dissolved in hexane/ethyl acetate (5:1). The solution was passed through a pad of silica gel with the same solvents. Concentration of the eluate followed by the addition of hexane (10 mL) resulted in the precipitation of additional product which was collected by filtration to provide 2.8 g (28%) of the desired product.

### Intermediate 7

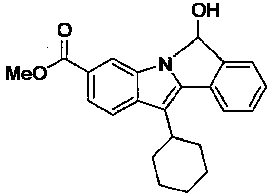

*Methyl 11-cyclohexyl-6-hydroxy-6H-isoindolo[2,1-a]indole-3-carboxylate.* A stirred mixture of methyl 2-bromo-3-cyclohexyl-1H-indole-6-carboxylate (10.1 g, 30 mmol), 2-formylphenylboronic acid (5.4 g, 36 mmol), LiCl (3.8 g (90 mmol) and Pd (PPh₃)₄ (1.6 g, 1.38 mmol) in 1M Na₂CO₃ (40 mL) and 1:1 EtOH-toluene (180 mL) was heated under nitrogen at 85 °C for 3 hours. The reaction mixture was then cooled to RT, and extracted with EtOAc (2X 100 mL). The extracts were washed sequentially with water and brine, then dried (MgSO₄), filtered and conventrated in-vacuo to afforded 13.3 g of crude product. This material was triturated with DCM and hexanes to provide pure desired product (7.52 g, 70%). LC-MS: m/e 360 (M-H); 344 (M-17)⁺. ¹H NMR (400 MHz, CHLOROFORM-D) δ ppm 1.33 - 1.60 (m, 4 H) 1.77 - 2.01 (m, 6 H) 2.80 (d, *J*=11.83 Hz, 1 H) 3.02 - 3.18 (m, 1 H) 3.89 (s, 3 H) 6.49 (d, *J*=11.33 Hz, 1 H) 7.34 (t, *J*=7.55 Hz, 1 H) 7.46 (t, *J*=7.55 Hz, 1 H) 7.62 (d, *J*=7.30 Hz, 1 H) 7.66 - 7.74 (m, 2 H) 7.77 (d, *J*=7.81 Hz, 1 H) 8.21 (s, 1 H).

### Intermediate 8

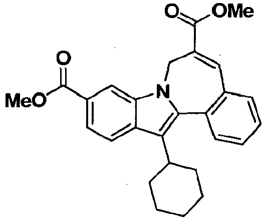

*Methyl 13-cyclohexyl-6-(methoxycarbonyl)-7H-indolo[2,1-a][2]benzazepine-10-carboxylate.* A stirred suspension of methyl 11-cyclohexyl-6-hydroxy-6H-isoindolo[2,1-a]indole-3-carboxylate (3.61 10mmol), Cs₂CO₃ (3.91 g, 12 mmol) and trimethyl 2-phosphonoacetate (2.86g, 14 mmol) in an. DMF (40 mL) was heated at 60 °C under nitrogen for 3 h. The resultant yellow suspension was cooled to rt and water was added with vigorous stirring. A yellow precipitate formed which was collected by filtration. The filtrand was washed with water, and then air dried overnight to afford the title compound as a yellow powder (4.124g, 96%). LC/MS: m/e 430 (MH⁺); ¹H NMR (400 MHz, CHLOROFORM-D) δ ppm 1.30 - 1.46 (m, *J*=14.86 Hz, 2 H) 1.55 (s, 2 H) 1.77 (s, 2 H) 1.85 - 2.18 (m, 4 H) 2.76 - 2.89 (m, 1 H) 3.84 (s, 3 H) 3.95 (s, 3 H) 4.19 (s, 1 H) 5.68 (s, 1 H) 7.38 - 7.63 (m, 4 H) 7.74 (dd, *J*=8.44, 1.39 Hz, 1 H) 7.81 - 7.98 (m, 2 H) 8.29 (d, *J=*1.01 Hz, 1 H).

### Intermediate 9

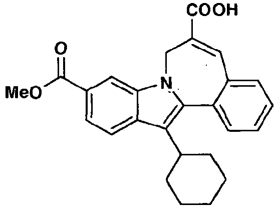

*Methyl 13-cyclohexyl-6-(carboxy)-5H-indolo[2,1-a][2]benzazepine-10-carboxylate.* Methyl 13-cyclohexyl-6-(methoxycarbonyl)-7H-indolo[2,1-a][2]benzazepine-10-carboxylate (308mg, 0.72mmol) was dissolved in N,N-dimethylformamide (5 mL) and treated with LiOH (173mg, 7.2mmol). The mixture was heated at 50 °C for 4hr, afterwhich the solvent was removed in vacuo. The residue was dissolved in H₂O (5mL) and the resultant mixture was acidified by the addition of a 10% aqueous HCL solution. A precipitate formed which was collected by filtration and air dried to afford the title compound as a bright yellow solid (290mg, 97%). ESI-MS m/z [M+1]=415.

### Intermediate 10

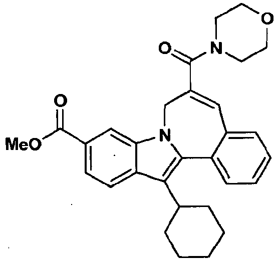

*Methyl 13-cyclohexyl-6-(morpholinylcarbonyl)-5H-inclolo[2,1-a][2]benzazepine-10-carboxylate.* TBTU (145mg, 0.45 mmol) was added to a stirred solution of Methyl 13-cyclohexyl-6-(carboxy)-5H-indolo[2,1-a][2]benzazepine-10-carboxylate (125mg, 0.30 mmol), morpholine (26 µL, 0.30 mmol), and N,N-diisopropylethylamine 200 µL, 1.15 mmol) in DMF (2mL). The mixture was stirred at 22°C for 20 min. The resulting solution was then injected onto a Shimadzu reverse phase preparative HPLC. The product containing fraction was concentrated on a Speed Vac^{®}) to leave methyl 13-cyclohexyl-6-(morpholinylcarbonyl)-5*H*-indolo[2,1-a][2]benzazepine-10-carboxylic acid as a yellow solid (64mg, 44%). ESI-MS *m*/*z* 487 (MH⁺);¹H NMR (500 MHz, CDCl₃) δ 1.21 (m, 1 H), 1.34-1.55 (m, 3 H), 1.77 (m, 2 H), 1.91 (m, 1 H), 2.06 (m, 3 H), 2.83 (m, 1 H), 2.97-3.85 (m, 8 H), 3.97 (s, 3 H), 4.45 (m, 1 H), 5.07 (m, 1 H), 6.89 (s, 1 H), 7.41 (d, 1 H), 7.49 (m, 2 H), 7.57 (m, 1 H), 7.75 (m, 1 H), 7.89 (d, *J*=8.55 Hz, 1 H), 8.15 (s, 1 H).

### Intermediate 11

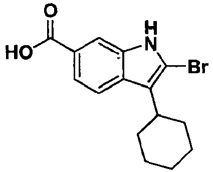

*2-bromo-3-cyclohexyl-2-1H-indole-6-carboxylic acids.* To a solution of methyl 2-bromo-3-cyclohexyl-2-1H-indole-6-carboxylate (8.0g, 23.79 mmol) in THF/MeOH (30 mL/30 mL), 10 N solution of NaOH (23.8 mL, 238 mmoL) was added. The reaction mixture was stirred at 40°C for 6 hrs, then at rt. for overnight. It was then concentrated and acidified with concentrated HCl solution to pH ∼ 4. A brownish solid was collected as crude product. (7.6 g, 99% yield). MS m/ 322(MH⁺), Retention time: 3.696 min.

### Intermediate 12

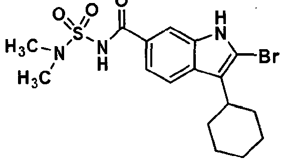

*2-Bromo-3-cyclohexyl-N-[(dimethylamino)sulfonyl]-1H-indole-6-carboxamide.* 1,1'-Carbonyldiimidazole (1.17 g, 7.2 mmol) was added to a stirred solution of 2-bromo-3-cyclohexyl-1H-indole-6-carboxylic acid (2.03 g, 6.3 mmol) in THF (6 mL) at 22°C. The evolution of CO₂ was instantaneous and when it slowed the solution was heated at 50°C for 1 hr and then cooled to 22°C. N,N-Dimethylsulfamide (0.94 g, 7.56 mmol) was added followed by the dropwise addition of a solution of DBU (1.34 g ,8.8 mmol) in THF (4 mL). Stirring was continued for 24 hr. The mixture was partitioned between ethyl acetate and dilute HCl. The ethyl acetate layer was washed with water followed by brine and dried over Na₂SO₄. The extract was concentrated to dryness to leave the title product as a pale yellow friable foam, (2.0 g, 74 %, >90 % purity , estimated from NMR ¹H NMR (300 MHz, DMSO-D6) δ ppm 1.28 - 1.49 (m, 3 H) 1.59 - 2.04 (m, 7 H) 2.74 - 2.82 (m, 1 H) 2.88 (s, 6 H) 7.57 (dd, *J*=8.42, 1.46 Hz, 1 H) 7.74 (d, *J*=8.78 Hz, 1 H) 7.91 (s, 1 H) 11.71 (s, 1 H) 12.08 (s, 1 H).

### Intermediate 13

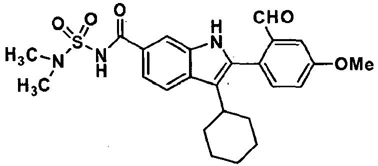

*3-cyclohexyl-N-(N,N-dimethylsulfamoyl)-*2-(2*-formyl-4-methoxyphenyl)*-1*H-indole-6-carboxamide.* A mixture of the *2-Bromo-3-cyclohexyl-N-[(dimethylamino)sulfonyl]-1H-indole-6-carboxamide* (4.28g, 0.01 mol), boronic acid (2.7g, 0.015 mol), 2-dicyclohexylphosphino-2',6'-dimethoxy-biphenyl (41 mg, 0.0001 mol), palladium acetate (11.2 mg), and finely ground potassium carbonate (4.24g, 0.02 mol) in toluene (30 mL) was stirred under reflux and under nitrogen for 30 min, at which time LC/MS analysis showed the reaction to be complete. The reaction mixture was then diluted with ethyl acetate and water, and then acidified with an excess of dilute HCl. The ethyl acetate layer was then collected and washed with dilute HCl, water and brine. The organic solution was then dried (magnesium sulfate), filtered and concentrated to give a gum. The gum was diluted with hexanes (250 ml) and ethyl acetate (25 mL), and the mixture was stirred for 20 hr at 22° C during which time the product was transformed into a bright yellow granular solid (4.8 g) which was used directly without further purification.

### Intermediate 14

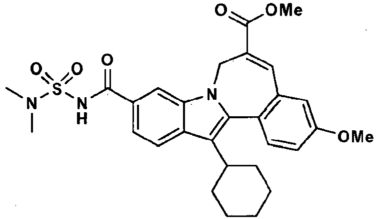

*6-Carbomethoxy-13-cyclohexyl-N-[(dimethylamino)sulfonyl]-5H-indolo[2,1-a] [2] benzazepine-10-carboxamide.* A mixture of the 3-cyclohexyl-N-(N,N-dimethylsulfamoyl)-2-(2-formyl-4-methoxyphenyl)-1H-indole-6-carboxamide (4.8g, 0.01 mol), and cesium carbonate (7.1g, 0.02 mol) and the trimethyl 2-phosphonoacetate (2.86 g, 0.014 mol) in DMF (28mL) was stirred for 20 hr at an oil bath temperature of 55 ° C. The mixture was poured into ice-water and acidified with dilute HCl to precipitate the crude product. The solid was collected, dried and flash chromatographed on SiO₂ (110g) using an ethyl acetate and methylene chloride (1:10) solution containing 2% acetic acid. Homogeneous fractions were combined and evaporated to afford the title compound as a pale yellow solid (3.9g, 71 % yield). MS: 552 (M=H+).

### Intermediate 15

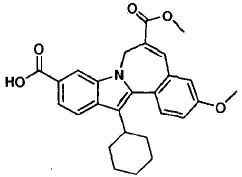

*13-Cyclohexyl-3-methoxy-6-(methoxycarbonyl)-7H-indolo[2,1-a][2]benzazepine-10-carboxylic acid.* Trifluoroacetic acid (30 mL) was added dropwise to a stirring slurry of 10-tert-butyl 6-methyl 13-cyclohexyl-3-methoxy-7*H-*indolo[2,1-*a*][2]benzazepine-6,10-dicarboxylate (10 g, 20 mmol) in dichloroethane (30 mL) under N₂. The clear dark green solution was stirred at rt for 2.5h, concentrated to dryness and stirred with EtOAc (100 mL) overnight. The solids were collected by filtration, washed with EtOAc and Et₂O to yield 13-cyclohexyl-3-methoxy-6-(methoxycarbonyl)-7*H*-indolo[2,1-*a*][2]benzazepine-10-carboxylic acid (8.35 g, 18.8 mmol, 94%) was as a yellow solid which was used without further purification. ¹HNMR (300 MHz, CDCl₃) δ 1.13 - 2.16 (m, 10H), 2.74 - 2.88 (m, 1H), 3.84 (s, 3H), 3.89 (s, 3H), 4.06 - 4.29 (m, 1H), 5.54 - 5.76 (m, 1H), 6.98 (d, *J*= 2.6 Hz, 1H), 7.08 (dd, *J*= 8.4, 2.6 Hz, 1H), 7.52 (d, *J* = 8.4 Hz, 1H), 7.78 (dd, *J*= 8.8, 1.1 Hz, 1H), 7.80 (s, 1H), 7.86 (d, *J*= 8.8 Hz, 1H), 8.34 (d, *J*= 1.1 Hz, 1H). LCMS: m/e 446 (M+H)⁺, ret time 3.21 min, column B, 4 minute gradient.

### Intermediate 16

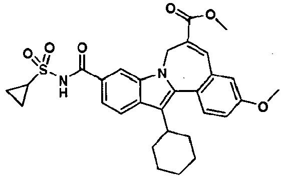

*Methyl 13-cyclohexyl-10-((cyclopropylsulfonyl)carbamoyl)-3-methoxo-7H-indolo[2,1-a][2]benzazepine-6-carboxylate.* 1,1'-Carbonyldiimidazole (1.82 g, 11.2 mmol) was added to a slurry of 13-cyclohexyl-3-methoxy-6-(methoxycarbonyl)-7*H-*indolo[2,1-*a*][2]benzazepine-10-carboxylic acid (3.85 g, 8.65 mmol) in THF (15 mL). The reaction mixture was heated at 60 °C for 1.5h, cooled to rt, treated with cyclopropanesulfonamide (1.36 g, 11.2 mmol), stirred 10 min and then treated with the dropwise addition of a solution of DBU (2.0 mL, 13 mmol) in THF (3 mL). The reaction mixture was stirred at rt overnight, diluted with EtOAc (100 mL) and washed with H₂O (~30 mL), 1N HCl (aq.) (2 x 50 mL) and brine (~30 mL). The combined aqueous layers were extracted with EtOAc (100 mL) and the organic layer was washed with 1N HCl (aq.) (~50 mL). The combined organic layers were washed with brine (~30 mL), dried (MgSO₄), filtered and concentrated. The residue was stirred with Et₂O (~100 mL) for 2h and the solids were collected by filtration, rinsed with Et₂O and dried to yield methyl 13-cyclohexyl-10-((cyclopropylsulfonyl)carbamoyl)-3-methoxy-7*H*-indolo[2,1-a][2]benzazepine-6-carboxylate (4.24 g, 7.73 mmol, 89%) as a pale yellow solid which was used without further purification. ¹HNMR (300 MHz, CDCl₃) δ 1.08 - 2.13 (m, 14H), 2.73 - 2.87 (m, 1H), 3.13 - 3.24 (m, 1H), 3.82 (s, 3H), 3.89 (s, 3H), 4.04 - 4.27 (m, 1H), 5.50 - 5.71 (m, 1H), 6.98 (d, J = 2.6 Hz, 1H), 7.08 (dd, *J* = 8.8, 2.6 Hz, 1H), 7.44 (dd, *J* = 8.4, 1.1 Hz, 1H), 7.50 (d, *J* = 8.8 Hz, 1H), 7.80 (s, 1H), 7.86 (d, *J* = 8.4 Hz, 1H), 8.11 (br s, 1H), 8.78 (br s, 1H). LCMS: m/e 549 (M+H)⁺, ret time 3.79 min, column B, 4 minute gradient.

### Intermediate 17

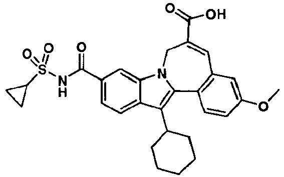

*13-Cyclohexyl-10-((cyclopropylsulfonyl)carbamoyl)-3-methoxy-7H-indolo[2,1-a][2]benzazepine-6-carboxylic acid.* Methyl 13-cyclohexyl-10-((cyclopropylsulfonyl)carbamoyl)-3-methoxy-7*H*-indolo[2,1-*a*][2]benzazepine-6-carboxylate (1.0 g, 1.8 mmol) was dissolved into MeOH//THF (1:1, 24 mL) and treated with 1M aqueous NaOH (5 mL). The reaction mixture was stirred and heated at 60 °C for 1.5h and cooled to rt. The clear solution was neutralized with 1M aqueous HCl (5 mL) and concentrated to remove organic solvents. The resultant solids were collected by filtration, washed with H₂O and dried under vacuum to yield 13-cyclohexyl-10-((cyclopropylsulfonyl)carbamoyl)-3-methoxy-7*H*-indolo[2,1-*a*][2]benzazepine-6-carboxylic acid (1.0 g, 1.7 mmol, 94%) as a bright yellow solid (with 0.75 equiv. of THF) which was used without further purification. ¹HNMR (300 MHz, CD₃OD) δ 1.11 - 2.24 (m, 17H, 3H from THF), 2.81 - 2.96 (m, 1H), 3.17 - 3.28 (m, 1H), 3.69 - 3.79 (m, 3H, from THF), 3.94 (s, 3H), 4.07 - 4.33 (m, 1H), 5.55 - 5.81 (m, 1H), 7.14 - 7.24 (m, 2H), 7.55 - 7.64 (m, 2H), 7.88 - 7.94 (m, 2H), 8.20 (br s, 1H). LCMS: m/e 535 (M+H)⁺, ret time 3.73 min, column B, 4 minute gradient.

### Intermediate 18

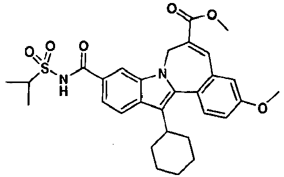

*Methyl 13-cyclohexyl-10-((isopropylsulfonyl)carbamoyl)-3-methoxy-7H-indolo[2,1-a][2]benzazepine-6-carboxylate.* 1,1'-Carbonyldiimidazole (262 mg, 1.62 mmol) was added to a slurry of 13-cyclohexyl-3-methoxy-6-(methoxycarbonyl)-7*H-*indolo[2,1-*a*][2]benzazepine-10-carboxylic acid (603 mg, 1.36 mmol) in THF (3 mL). The reaction mixture was heated at 60 °C for 1.5h, cooled to rt, treated with propane-2-sulfonamide (200 mg, 1.62 mmol), stirred 10 min and then treated with the dropwise addition of a solution of DBU (0.27 mL, 1.8 mmol) in THF (0.75 mL). The reaction mixture was stirred at rt overnight, diluted with EtOAc (15 mL) and washed with H₂O (~5 mL), 1N HCl (aq.) (2 x 10 mL) and brine (~5 mL). The combined aqueous layers were extracted with EtOAc (15 mL) and the organic layer was washed with 1N HCl (aq.) (~10 mL). The combined organic layers were washed with brine (~5 mL), dried (MgSO₄), filtered and concentrated. The residue was stirred with Et₂O (~15 mL) for 2h and the solids were collected by filtration, rinsed with Et₂O and dried to yield methyl 13-cyclohexyl-10-((isopropylsulfonyl)carbamoyl)-3-methoxy-7*H*-indolo[2,1-a][2]benzazepine-6-carboxylate (640 mg, 1.2 mmol, 85%) as a bright yellow solid which was used without further purification. ¹HNMR (300 MHz, CDCl₃) δ 1.12 - 2.13 (m, 10H), 1.47 (d, *J* = 7.0 Hz, 6H), 2.73 - 2.86 (m, 1H), 3.82 (s, 3H), 3.89 (s, 3H), 4.06 - 4.26 (m, 1H), 4.09 (septet, *J* = 7.0 Hz, 1H), 5.51 - 5.71 (m, 1H), 6.98 (d, *J* = 2.6 Hz, 1H), 7.08 (dd, *J* = 8.4, 2.6 Hz, 1H), 7.44 (dd, *J* = 8.4, 1.5 Hz, 1H), 7.50 (d, *J* = 8.4 Hz, 1H), 7.80 (s, 1H), 7.87 (d, *J* = 8.4 Hz, 1H), 8.10 (d, *J* = 1.5 Hz, 1H), 8.57 (s, 1H). LCMS: m/e 551 (M+H)⁺, ret time 3.87 min, column B, 4 minute gradient.

### Intermediate 19

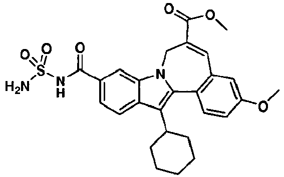

*Methyl 10-((aminosulfonyl)carbamoyl)-13-cyclohexyl-3-methoxy-7H-indolo[2,1-a][2]benzazepine-6-carboxylate.* 1,1'-Carbonyldiimidazole (1.23 g, 7.60 mmol) was added to a slurry of 13-cyclohexyl-3-methoxy-6-(methoxycarbonyl)-7*H-*indolo[2,1-*a*][2]benzazepine-10-carboxylic acid (2.6 g, 5.8 mmol) in THF (11 mL). The reaction mixture was heated at 60 °C for 1.5h, cooled to rt, treated with sulfamide (1.12 g, 11.1.7 mmol), stirred 10 min and then treated with the dropwise addition of a solution of DBU (1.8 mL, 11.7 mmol) in THF (3 mL). The reaction mixture was stirred at rt for 3h, diluted with EtOAc (80 mL) and CH₂Cl₂ (100 mL) and concentrated to dryness. The residue was diluted with CH₂Cl₂ (100 mL) and washed with 1N HCl (aq.) (2 x 100 mL). The combined aqueous layers were extracted with CH₂Cl₂ (100 mL) and the combined organic layers were washed with ½ saturated brine (~50 mL), dried (MgSO₄), filtered and concentrated. The residue was stirred with Et₂O (~75 mL) for 1h and the solids were collected by filtration, rinsed with Et₂O and dried to yield methyl 10-((aminosulfonyl)carbamoyl)-13-cyclohexyl-3-methoxy-7*H*-indolo[2,1-*a*][2]benzazepine-6-carboxylate (2.8 g, 5.3 mmol, 91%) as a bright yellow solid which was used without further purification. ¹HNMR (300 MHz, CDCl₃) δ 1.08 - 2.10 (m, 10H), 2.71 - 2.84 (m, 1H), 3.79 (s, 3H), 3.89 (s, 3H), 4.00 - 4.18 (m, 1H), 5.50 - 5.64 (m, 1H), 5.68 (s, 2H), 6.97 (d, *J* = 2.6 Hz, 1H), 7.07 (dd, *J* = 8.8, 2.6 Hz, 1H), 7.46 (dd, *J* = 8.4, 1.5 Hz, 1H), 7.49 (d, *J* = 8.8 Hz, 1H), 7.78 (s, 1H), 7.82 (d, *J* = 8.4 Hz, 1H), 8.10 (br s, 1H), 9.49 (s, 1H). LCMS: m/e 524 (M+H)⁺, ret time 3.60 min, column B, 4 minute gradient.

### Intermediate 20

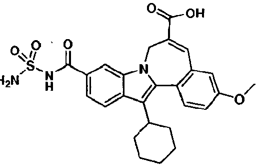

*10-((Aminosulfonyl)carbamoyl)-13-cyclohexyl-3-methoxy-7H-indolo[2,1-a][2]benzazepine-6-carboxylic acid.* Methyl 10-((aminosulfonyl)carbamoyl)-13-cyclohexyl-3-methoxy-7*H*-indolo[2,1-*a*][2]benzazepine-6-carboxylate (725 mg, 1.39 mmol) was dissolved into MeOH//THF (1:1, 16 mL) and treated with 1M aqueous NaOH (3 mL). The reaction mixture was stirred and heated at 60 °C for 0.5h and cooled to rt. The reaction solution was diluted with MeOH/H₂O (2:1, 15 mL), neutralized with 1 M aqueous HCl (3 mL) and concentrated to remove organic solvents. The resultant solids were collected by filtration, washed with H₂O and dried under vacuum to yield 10-((aminosulfonyl)carbamoyl)-13-cyclohexyl-3-methoxy-7*H*-indolo[2,1-*a*][2]benzazepine-6-carboxylic acid (650 g, 1.3 mmol, 92%) as a bright yellow solid which was used without further purification. ¹HNMR (300 MHz, CDCl₃) δ 1.16 - 2.22 (m, 10H), 2.82 - 2.96 (m, 1H), 3.94 (s, 3H), 4.07 - 4.29 (m, 1 H), 5.57 - 5.80 (m, 1H), 7.14 - 7.23 (m, 2H), 7.55 - 7.63 (m, 2H), 7.88 - 7.94 (m 2H), 8.18 (s, 1H). LCMS: m/e 510 (M+H)⁺, ret time 2.85 min, column B, 4 minute gradient.

### Example 1

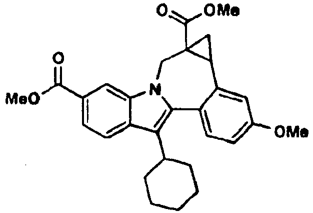

*(+*/*-) 8-Cyclohexyl-1,12b-dihydro-11-methoxy-cycloprop[d]indolo[2,1-a][2]benzazepine-1a,5(1H)-dicarboxylic acid, dimethyl ester.* Trimethylsulfoxonium iodide (1.44 g, 6.528 mmol) was added to a suspension of NaH (162 mg as 60% oil dispersion, 6.746 mmol) in DMSO (30 mL), and the reaction was stirred at rt for 30 min. A solution of 13-cyclohexyl-3-methoxy-7*H*-indolo[2,1-*a*][2]benzazepine-6,10-dicarboxylic acid, dimethyl ester (1.0g, 2.176 mmol) in DMSO (10 mL) was then added, and the resultant mixture was stirred at rt. for 2 hr, and then at 50 °C overnight. The reaction was subsequently quenched by the addition of water, after which an off-white precipitate formed which was collected by filtration. This material was then purified using silica gel flash chromatography using hexanes to 25% ethyl acetate in hexanes as eluent. Homogeneous fractions where combined and evaporated *in vacuo* to give the product as an off-white solid, (815 mg, 79% yield). MS m/z 474(MH⁺), Retention time: 4.161 min.

### Example 2

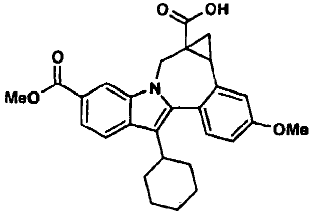

*(+*/*-) 8-cyclohexyl-1,12b-dihydro-11-methoxy-cycloprop[d]indolo[2,1-a][2]benzuzepine-1a,5(2H)-dicarboxylic (acid, 5-methy*/ *ester.* To a solution of (+/-)-8-cyclohexyl-1,12b-dihydro-11-methoxy-cycloprop[*d*]indolo[2,1-*a*][2]benzazepine-1a,5(2*H*)-dicarboxylic acid, dimethyl ester (800, 1.69 mmol) in THP (25 mL), a 1M solution of n-Bu₄NOH (2.5 mL, 2.5 mmol) in methanol was added. Thc reaction mixture was stirred at rt. for three days and then heated at 40 °C for 3 hr. It was concentrated, acidified with 1N HCl solution and extracted with ethyl acetate (2X30 mL). The organic layers were combined, dried (MgSO₄), filtered and concentrated under reduced pressure to give the product as a light-yellow colored solid, (750 mg, 97% yield). MS m/z 460(MH⁺), Retention time: 4.001 min; ¹H NMR (300 MHz, CD₃OD) δ ppm. Compound was observed to exist as inter-converting rotamers, as evidenced from the compound's NMR spectrum.

### Example 3

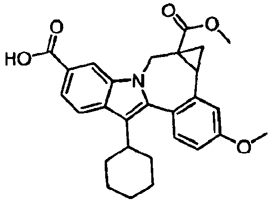

*(+*/*-) 8-cyclohexyl-11-(methyloxy)-1a-((methyloxy)carbonyl)-1,1a,2,12b-retrahydrocyclopropa[d]indolo[2,1-a][2]benzazepine-5-carboxylic acid.* (+/-)-5-(1,1-Dimethylethyl) 1a-methyl 8-cyclohexyl-11-(methyloxy)-1,12b-dihydrocyclopropa[*d*]indolu[2,1-*a*][2]benzazepine-1a,5(2*H*)-dicarboxylate (0.60g, 1.16mMol) was dissolved in 1,2-dichlorocthanc (10mL) and tritluoroacctic acid (10mL) added and the reaction stirred at room temperature under nitrogen and monitored by HPLC until complete. Volatiles were removed *in vacuuo* and benzene added to the residue azetrope removal of trifluoroacetic acid *in vacuuo* to yield product as a yellow foam. ¹H NMR (500 MHz, CHLOROFORM-D) δ ppm 0.34 - 0.48 (m, 0.4 H) 0.91 (s, 0.4 H) 1.17 - 1.33 (m, 2 H) 1.32 - 1.49 (m, 3 H) 1.58 (d, *J*=13.12 Hz, 0.5 H) 1.64 - 1.86 (m, 3.5 H) 1.87 - 2.16 (m, 4.5 H) 2.59 - 2.70 (m, 0.4 H) 2.79 (t, *J*=12.51 Hz, 0.4 H) 2.84 - 2.99 (m, 1.3 H) 3.45 (d, *J*=15.26 Hz, 0.6 H) 3.58 (d, *J*=1.53 Hz, 1.7 H) 3.81 (d, *J*=1.53 Hz, 1.2 H) 3.83 - 3.95 (m, 3 H) 4.09 (d. *J*=15.26 Hz, 0.4 H) 5.21 (d, *J*= 14.95 Hz, 0.4 H) 5.45 (d. *J*=14.95 Hz, 0.6 H) 6.88 - 6.97 (m, 1 H) 7.02 (d, *J*=2.44 Hz, 0.4 H) 7.14 (s, 0.6 H) 7.26 - 7.33 (m, 1 H) 7.74 - 7.92 (m, 2 H) 8.21 (s, 0.4 H) 8.43 (s, 0.6 H); MS m/z 460(MH⁺).

### Example 4

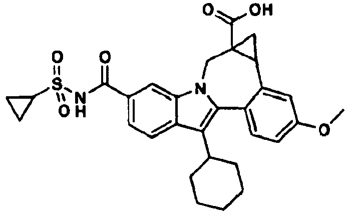

*(+*/*-) 8-cyclohexyl-5-(cyclopropylsulfonylcarbamoyl)-1,1a,2,12b-tetrahydro-11-methoxy-cycloprop[d]indolo[2,1-a][2]benzazepine-1a-carboxylic acid.* A mixture of (+/-) 8-cyclohexyl-[1,1a,2,12b-tetrahydro-11-methoxy-1a-(methoxycarbonyl)-cycloprop[d]indolo[2,1-a][2]benzazcpine-5-carboxylic acid (1 equiv), and carbonyldiimidazole (1.5 equiv) in anhydrous THF was heated at 50 °C for 30 min and allowed to cool to rt. Then 1 equiv of cyclopropanesulfonamide and 1,8-diazabicyclo[5.4.0]undec-7-ene (2 equiv) were added consecutively. The resultant mixture was stirred at rt overnight. After acidic aqueous workup, the isolated crude product was purified by prep. HPLC. The intermediate ester was then hydrolyzed using 1N NaOH in THF-MeOH to afford the title compound. LC/MS: Retention time: 2.030 min; m/c 549 (MH'). ¹H NMR (400 MHz, CDCl₃): The product was observed to exist as inter-convening rotamers, as evidenced from the compound's NMR spectrum.

### Example 5

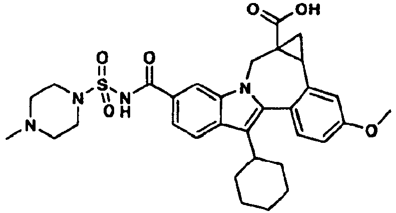

*(+*/*-) 8-cyclohexyl-5-(4-methylpiperazin-1-ylsulfonylcarbamoyl)-1,1a,2,12b-tetrahydro-11-methoxy-cycloprop[d]indolo[2,1-a][2]benzazepine-1a-carboxylic acid.* LC/MS: Retention time: 1.687 min; m/e 607 (MH⁺). ¹H NMR (400 MHz, CDCl₃). Compound was observed to exist as inter-converting rotamers, as evidenced from the compound's NMR spectrum.

### Example 6

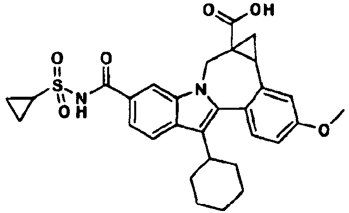

*(+*/*-) 8-cyclohexyl-5-(cyclopronylsulfonylcarbamoyl)-1,1a,2,12b-tetrahydro-11-methoxy-cycloprop[d]indolo[2,1-a][2]benzazepine-1a-carboxylic acid.* LC/MS: Retention time: 2.030 min; m/e 549 (MH⁺). ¹ NMR (400 MHz, CDCl₃). Compound was observed to exist as inter-converting rotamers, as evidenced from the compound's NMR spectrum.

### Example 7

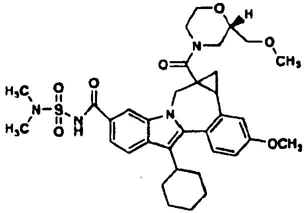

*(+*/*-)-8-cyclohexyl-N-[(dimethylamino)sulfonyl)-1,1a,2,12b-tetrahydro-1a-[[(cis)-(S)-2-methoxymethyl-4-morpho*/*inyl]-carbonyl]-cycloprop[d]indolo[2,1-a][2]benzazepine-5-carboxamide.* TBTU (120 mg, 0.374 mmol) was added to a stirred solution at 22°C uf (+/-)-8-cyclohcxyl-5-[[[(dimethylamino)sulfonyl]amino]carbonyl]-1,12b-dihydro-11-methoxy cycloprop[d]indolo[2,1-a][2]benzazepine-1a(2H)-carboxylic acid, (164.4 mg, 0.298 mmol), (2S)-2-(methoxymethyl)morpholine hydrochloride (63 mg, 0.376 mmol), and TEA (152 mg, 1.50 mmol) in DMSO (1 mL). The mixture was stirred for I hr and was diluted with water. The solution was acidified with dilute HCl to precipitate the product which was collected, washed with cold water, and dried in vacuo over phosphorous pentoxide to afford a mixture of diastereoisomers (162 mg, 82 % yield). A portion of this material was purified on a Shimadzu Preparative liquid chromatograph using an XTetra® 30x100 mm reverse phase column and a gradient of methanol-water containing 1 % TFA. The methanol was removed from the product containing fractions. The precipitated product was extracted into ethyl acetate. The organic layer was washed (water, brine), dried (sodium sulfate), and concentrated to a pale yellow solid. LC/MS: *m*/*z* 665 (MH⁺), rt 2.382 min. Phenomenex-Luna 4.6 x 50mm S10 column.Gradient conditions: 10% MeOH - 90% HOH-0.1% TFA to 90% MeOH - 10% HOH-0.1% TFA in 2 min. ¹H NMR (300 MHz, DMSO-D6) δ ppm 0.72 - 2.07 (m, 13 H) 2.18 - 2.34 (m, 1 H) 2.90 (d, *J*=3.66 Hz, 6 H) 3.12 - 3.67 (m, 7 H) 3.84 (s, 3 H) 3.93 - 4.46 (m, 6 H) 6.93 - 7.36 (m, 3 H) 7.54 - 7.71 (m, *J*=8.42 Hz, 1H) 7.76 - 7.97 (m, 1H) 8.27 (d, *J*=47.94 Hz, 1 H) 11.51 - 11.78 (m, 1 H).

### Example 8

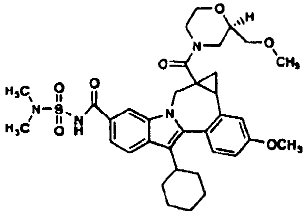

*8-cyclohexyl-N-[(dimethylamino)sulfony*/*]-1,1a,2,12b-tetrahydro-1a-[[(cis)-(R)-2-methoxymethyl-4-morpholinyl] carbonyl]-cycloprop[d]indolo[2,1-a][2]benzazepine-5-carboxamide.* TBTU (110 mg, 0.342 mmol) was added to a stirred solution at 22°C of (+/-)-8-cyclohexyl-5-[[[(dimethylamino)sulfonyl]amino]carbonyl]-1,12b-dihydro-11-methoxy cycloprop[d]indolo[2,1-a][2]benzazepine-1a(2H)-carboxylic acid (157.4 mg, 0.285 mmol), (2R)-2-(methoxymethyl)morpholine hydrochloride (67 mg, 0.399 mmol), and TEA (115mg, 1.14 mmol) in DMSO (1.5 mL). The mixture was stirred for 2 hr and was diluted with water. The solution was acidified with dilute HCl to precipitate the product which was extracted into chloroform. The chloroform solution was washed (water (2x), brine), dried (magnesium sulfate), and concentrated to leave the product as a gum (298 mg). The product was purified on a silicic acid preparative plate. The plate was eluted with methylene chloride - 2 % acetic acid. The product containing bands were comined and extracted with methylene chloride - 10 % MeOH. Removal of the solvents left the titled compound as a mixture of diastereoisomers. LC/MS: *m*/*z* 665 (MH⁺), rt 2.382 min. Phenomenex-Luna 4.6 x 50mm S10 column.Gradient conditions: 10% MeOH - 90% HOH - 0.1% TFA to 90% MeOH - 10% HOH - 0.1% TFA in 2 min. ¹H NMR (500 MHz, DMSO-D6) 8 ppm 0.75 - 2.15 (m, 13 H) 2.88 (s, 6 H) 3.12 - 3.29 (m, 5 H) 3.31 (s, 3 H) 3.83 - 3.93 (m, 5 H) 4.55 - 5.34 (m, *J*=90.95 Hz, 1 H) 6.96 - 7.09 (m, I H) 7.11 - 7.22 (m, I H) 7.24 - 7.31 (m, 1 H) 7.57 - 7.71 (m, 1 H) 7.77 - 7.91 (m, 1 H) 8.10 - 8.38 (m, I H) 11.21 - 12.22 (1 H).

### Example 9

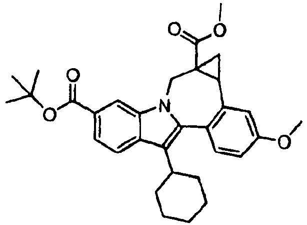

*(+*/*-)-5-(1,1-dimethylethyl) 1a-methyl 8-cyclohexyl-11-(methyloxy)-1,12b-dihydrocyclopropa[d]indolo[2,1-a][2]benzazepine-1a,5(1H)-dicarboxylate.* Trimethylsulfoxonium Iodide (7.20g, 32.7mMol) was suspended in 90mL of anhydrous DMSO, blanketed in nitrogen and 95% sodium hydride (789mg, 31.2mMol) added. The reaction was stirred under nitrogen for 25 minutes until the solution was clear. 10-(1,1-dimethylethyl) 6-methyl 13-cyclohexyl-3-(methyloxy)-7*H*-indolo[2,1-a][2]benzazepine-6,10-dicarboxylate (9.00g, 17.9mMol) was added to the reaction and the reaction heated to 65 C for 4.5 hours. The reaction was partitioned between dichloromethane and 0.1N hydrochloric acid. The aqueous phase was extracted with dichloromethane. The dichloromethane fractions combined and washed 3x with 1.0N hydrochloric acid, dried over sodium sulfate and volatiles removed in vacuuo to yield 10.9g of a brown foam. The reaction was combined with a previous experiment and the product chromatographed on silica gel eluting with dichloromethane to yield 8.3g of product as a light colored amorphous solid. ¹H NMR (500 MHz, CHLOROFORM-D) δ ppm 0.40 (t, *J*=6.10 Hz, 0.5H) 1.17 - 1.30 (m, 2 H) 1.30 - 1.46 (m, 3 H) 1.49 - 1.61 (m, 3 H) 1.63 (d, *J*=4.27 Hz, 9 H) 1.65 - 1.71 (m, 1 H) 1.71 - 1.83 (m, 3 H) 1.91 (d, *J*=9.16 Hz, 1H) 1.94-2.15 (m, 3 H) 2.64 (dd, *J*=9.77, 7.02 Hz, 0.5 H) 2.77 (t, *J*=12.05 Hz, 0.5 H) 2.85 - 2.97 (m, 1 H) 3.42 (d, *J*=14.95 Hz, 0.6 H) 3.57 (d, *J*=1.53 Hz, 1.5 H) 3.79 (d, *J*=1.53 Hz, 1.5 H) 3.88 (s, 3 H) 4.02 - 4.09 (m, 0.6 H) 5.19 (d, *J*=14.95 Hz, 0.5 H) 5.42 (d, *J*=14.65 Hz, 0.5 H) 6.86 - 6.95 (m, I H) 7.01 (d, *J*=2.44 Hz, 0.5 H) 7.12 (d, *J*=1.53 Hz, 0.5 H) 7.26 - 7.30 (m, 0.8 H) 7.63 - 7.75 (m, 1 H) 7.80 (t, *J*=8.39 Hz, 1 H) 8.08 (s, 0.5 H) 8.27 (s, 0.5 H); MS m/z 516(MH⁺).

### Example 10

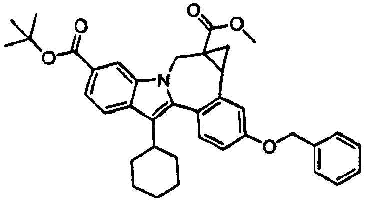

*(+*/*-)-5-(1,1-dimethylethyl) 1a-methyl 8-cyclohexyl-11-((phenylmethyl)oxy)-1,12b-dihydrocyclopropa[d]indolo[2,1-a][2]benzazepine-1a,5(2H)-dicarboxylate.* Trimethylsulfoxonium iodide (8.51g, 38.7mMol) was suspended in 100ml of anhydrous DMSO and sodium hydride (95%,937mg, 37.1mMol) was added and stirred under nitrogen for 30 minutes until the reaction appeared clear and homogenous. 10-(1,1-dimethylethyl) 6-methyl 13-cyclohexyl-3-((phenylmethyl)oxy)-7H-indolo[2,1-a][2]benzazcpine-6,10-dicarboxylate (10.6g, 18.4mMol) was added and the reaction heated under a nitrogen atmosphere to 65 °C for 18hrs. The reaction was partitioned between dichloromethane and 1N hydrochloric acid. The aqueous phase was extracted with dichloromethane and the organic phases combined, washed 3x with 1N hydrochloric acid and dried over sodium sulfate. Solvent was removed *in vacuuo* to yield 11.2g of crude product which was then combined with product from a previous experiment. The pure product was isolated by silica gel chromatography eluting with dichloromethane to yield 11.9g. ¹H NMR (500 MHz, CHLOROFORM-D) δ ppm 0.40 (t, *J*=6.10 Hz, 0.5 H) 1.09 - 1.31 (m, 2.1 H) 1.32 - 1.48 (m, 2.8 H) 1.48 - 1.61 (m, 2.1 H) 1.61 - 1.66 (m, 9 H) 1.66 - 1.85 (m, 3.5 H) 1.86 - 2.18 (m, 4.3 H) 2.63 (t, *J*=8.39 Hz, 0.5 H) 2.78 (m, 0.5 H) 2.84 - 2.97 (m, 1.1 H) 3.42 (d, *J*=14.95 Hz, 0.5 H) 3.49 - 3.63 (m, 1.5 H) 3.73 - 3.86 (m, 1.4 H) 4.06 (d, *J*=15.26 Hz, 0.5 H) 5.06 - 5.24 (m, 2.6 H) 5.42 (d, *J*=14.95 Hz, 0.5 H) 6.93 - 7.04 (m, 1 H) 7.10 (s, 0.5 H) 7.22 (s, 0.6 H) 7.26 - 7.31 (m, 0.9 H) 7.31 - 7.39 (m, 1.1 H) 7.39 - 7.53 (m, 4.2 H) 7.68 (dd, *J*=20.29, 8.39 Hz, 1.1 H) 7.80 (t, *J*=8.39 Hz, 1 H) 8.02 - 8.13 (m, 0.5 H) 8.18 - 8.32 (m, 0.5 H); MS m/z 592(MH⁺).

### Example 11

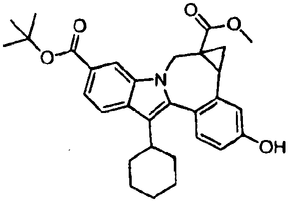

*(+1-)-5-(1,1-dimethylethyl) 1a-methyl 8-cyclohexyl-11-hydroxy-1,2b-dihydrocyclopropa[d]indolo[2,1-a]benzazepine-1a,5(2H)-dicarboxylate.* 5-(1,1-dimethylethyl) (+/-)-1a-methyl 8-cyclohexyl-11-((phenylmethyl)oxy)-1,12b-dihydrocyclopropa[*d*]indolo[2,1-*a*][2]benzazepine-1a,5(2*H*)-dicarboxylate (3.00g, 5.1mMol) was dissolved in 75ml of inhibitor free THF and 70mL of methanol added. The contents were placed under nitrogen and 304mg of 10% palladium on carbon added to the reaction. The reaction was placed under hydrogen (1atm, balloon) and stirred at room temperature for 18hrs. An additional 200mg of 10% palladium on carbon was added to the reaction and the reaction stirred an additional 21 hrs under hydrogen (1atm, balloon). The reaction was filtered through ceilite and volatiles removed in vacuuo to yield 2.7g of product. ¹H NMR (500 MHz, CHLOROFORM-D) δ ppm 0.32-0.44 (m, 0.5H) 1.11-1.29 (m, 2.2 H) 1.29 - 1.46 (m, 3.5H) 1.47-1.61 (m, 2.5 H) 1.63 (d, *J*=4.88 Hz, 9 H) 1.65 - 1.83 (m, 3.6 H) 1.85 - 2.13 (m, 4.1 H) 2.59 (dd, *J*=10.07, 7.02 Hz, 0.5 H) 2.76 (t, *J*=12.21 Hz, 0.5 H) 2.81 - 2.95 (m, 1 H) 3.36 - 3.45 (m, 0.6 H) 3.49 (s, 2.4 H) 3.56 (s, 1.4 H) 3.79 (s, 1.2 H) 4.05 (d, *J*=15.26 Hz, 0.5 H) 5.18 (d, *J*=15.26 Hz, 0.6 H) 5.25 (s, 0.7 H) 5.41 (d, *J*=15.26 Hz, 0.5 H) 6.77 - 6.88 (m, 1 H) 6.95 (d, *J*=2.44 Hz, 0.5 H) 7.08 (d, *J*=2.14 Hz, 0.5 H) 7.21 (t, *J*=8.39 Hz, 1.1 H) 7.63 - 7.74 (m, 1 H) 7.80 (t, *J*=8.24 Hz, 1 H) 8.04 - 8.10 (m, 0.5 H) 8.19 - 8.29 (m, 0.5 H); MS m/z 502(MH⁺); MS m/z 500(M-H)⁻.

### Example 12

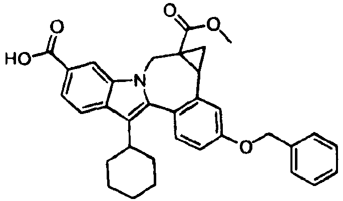

*(+*/*-)-8-cyclohexyl-1a-((methyloxy)carbony*/*)-11-((phenylmethyl)oxy)-1,1a,2,12b-tetrahydrocyclopropa[d]indolo[2,1-a][2]benzazepine-5-carboxylic acid.* 5-(1,1-dimethylethyl) (+/-)-1a-methyl 8-cyclohexyl-11-((phenylmethyl)oxy)-1,12b-dihydrocyclopropa[*d*]indolo[2,1-*a*][2]benzazepine-1a,5(2*H*)-dicarboxylate (3.00g, 5.07mMol) was dissolved in 50mL of 1,2-dichloroethane and 50mL of trifluoroacetic acid was added to the reaction via addition funnel over 5 minutes. The reaction was stirred at room temperature for 1hr and the volatiles were removed *in vacuuo.* Residual trifluoroacetic acid was removed by azetrope with benzene and the off white solid was dried *in vacuuo* to yield 2.8g of product. ¹H NMR (500 MHz, CHLOROFORM-D) δ ppm 0.43 (t, *J*=6.26 Hz, 0.4 H) 1.06 - 1.16 (m,0.3 H) 1.18 - 1.33 (m, 2.4 H) 1.33 - 1.50 (m, 2.7 H) 1.58 (d, *J*=13.12 Hz, 0.5 H) 1.65 - 1.72 (m, 0.7 H) 1.71 - 1.85 (m, 2.7 H) 1.93 (d, *J*=7.63 Hz, 1.3 H) 1.96 - 2.18 (m, 2.9 H) 2.61 - 2.70 (m, 0.4 H) 2.80 (t, *J*=12.21 Hz, 0.4 H) 2.86 - 2.99 (m, 1.2 H) 3.45 (d, *J*=15.26 Hz, 0.6 H) 3.53 - 3.64 (m, 1.8 H) 3.76 - 3.86 (m, 1.1 H) 4.10 (d, *J*=15.26 Hz, 0.4 H) 5.09 - 5.19 (m, 2 H) 5.22 (d, *J*=15.26 Hz, 0.4 H) 5.38 - 5.51 (m, 0.6 H) 6.95 - 7.05 (m, 1 H) 7.1 (d, *J*=2.14 Hz, 0.4 H) 7.26 - 7.31 (m, 0.7 H) 7.3 3 - 7.40 (m, I H) 7.43 (q, *J*=7.43 Hz, 2 H) 7.44 - 7.50 (m, 2 H) 7.72 - 7.93 (m, 2 H) 8.21 (s, 0.4 H) 8.37 - 8.50 (m, 0.6 H); MS m/z 536(MH⁺); MS m/z 534(M-H)-.

### Example 13

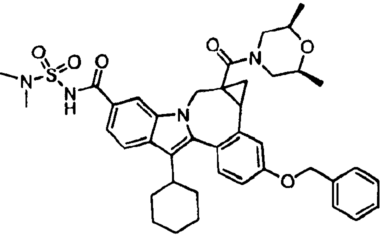

*(+*/*-)-8-cyclohexyl-N-((dimethylamino)sulfonyl)-1a-(((cis)-2,6-dimethyl-4-morpholinyl)carbonyl-11-((phenylmethyl)oxy)-1,1a,2,12b-tetrahydrocyclopropa[d]indolo[2,1-a][2]benzazepine-5-carboxamide.* To a stirred *solution of (+*/*-) Cycloprop[d]indolo[2,1-a][2]benzazepine-1a(2H)-carboxylic acid, 8-cyclohexy*/*-5-[[[(dimethylamino)sulfonyl]amino]carbonyl]-1,12b-dihydro-11-(phenylmethoxy)*-. (1.01g, 1.61mMol) in 16mL) of DMF was added TBTU (647mg, 2.01 mmol) The reaction was stirred at room temperature under a nitrogen atmosphere for 20 minutes then DMAP (600mg, 4.91mMol) was added followed by cis-2,6-dimethylmorpholine (0.30mL, 2.45mMol). The reaction was stirred for 38hrs at room temperature under nitrogen then poured into 50mL of water. Hydrochloric acid (20ml of 0.1N) was added to the aqueous suspension and a pale yellow solid collected by filtration and rinsed with 0.1N hydrochloric acid. The precipitate was dissolved in dichloromethane and washed 2x with 0.1N hydrochloric acid, dried over sodium sulfate and solvent removed in vacuuo to yield 1.02g of crude product. The aqueous phase was back extracted with dichloromethane and dried over sodium sulfate to yield 0.25g of an oil which partially crystallized on standing. The organic isolates were combined and chromatographed on silica gel eluting with 5% ethyl acetate, 1% acetic acid in dichloromethane to yield 0.79g (68%) of a very pale yellow solid. ¹H NMR (500 MHz, CHLOROFORM-D) δ ppm 0.37 (t, *J=5.34* Hz, 0.5 H) 0.89 (s, 1 H) 0.96 - 1.11 (m, 2.4 H) 1.12 - 1.32 (m, 5.2 H) 1.32 - 1.50 (m, 2.8 H) 1.50 - 1.70 (m, 2.2 H) 1.69 - 1.87 (m, 3.7 H) 1.87 - 2.08 (m, 4.6 H) 2.09 (s, 1.4 H) 2.34 - 2.46 (m, 0.6 H) 2.54 - 2.64 (m, 0.8 H) 2.73 - 2.85 (m, 0.8 H) 2.85 - 2.96 (m, 1.3 H) 2.99 (d, *J*=20.75 Hz, 0.7 H) 3.05 (s, 5.1 H) 3.44 - 3.77 (m, 1.5 H) 4.04-4.24 (m,1H) 4.68 (d, *J*=14.95 Hz, 0.5 H) 5.04 (d, *J*=16.48 Hz, 0.4 H) 5.09 - 5.19 (m, 1.8 H) 6.92 - 7.06 (m, 1 H) 7.09 - 7.23 (m, 1.2 H) 7.26 - 7.33 (m, 1 H) 7.33 - 7.52 (m, 5.5 H) 7.80 - 7.91 (m, 1 H) 7.92 - 8.05(m, 1 H) 8.81 (s, 0.5 H) 9.05 (s, 0.3 H); MS m/z 725(MH⁺); MS m/z 723(M-H)⁻.

Experimentals for Examples 14-26 use the general methods described below until further noted. LCMS data: Stop time: Gradient time + 1 minute; Starting conc: 0% B unless otherwise noted; Ending conc: 100% B unless otherwise noted; Eluent A: 5% CH₃CN / 95% H₂O with 10mM NH₄OAc (for columns A, D and E); 10 % MeOH / 90 % H₂O with 0.1% TFA (for culumns B and C); Eluent B: 95% CH₃CN / 5% H₂O with 10mM N₄OAc (for columns A, D and E): 90 % MeOH / 10 % H₂O with 0.1% TFA (for columns B and C): Column A: Phenomenex 10µ 4.6 x 50 mm C18- Column B: Phenomenex C18 10µ 3.0 x 50 mm; Column C: Phenomenex 4.6 x 50 mm C18 10µ. Column D: Phenomenex Lina C18 5µ 3.0 x 50 mm; Column E: Phenomenex 5µ 4.6 x 50 mm C 18. Preparative HPLC data: Conditions for H₂O/CH₃CN with 10mM NH₄OAc buffer; Gradient: Linear over 20 min. unless otherwise noted; Starting conc: 15% B unless otherwise noted; Ending conc: 100% B; Eluent A: 5% CH₃CN / 95% H₂O with 10mM NH₄OAc; Eluent B: 95% CH₃CN / 5% H₂O with 10mM NH₄OAc; Column: Sunfire Prep C₁₈ OBD 5µ 30 x 100 mm; Conditions for H₂O/MeOH with 0.1% TFA buffer; Gradient: Linear over 20 min. unless otherwise noted; Starting conc: 30% B unless otherwise noted; Ending conc: 100% B; Eluent A: 10% MeOH / 90 % H₂O with 0.1% TFA; Eluent B: 90 % MeOH / 10 % H₂O with 0.1% TFA; Column: phenomenex 21 x 100 mmC 18 H₂O.

### Example 14

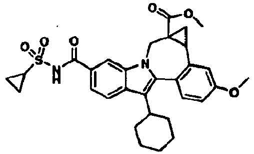

*Methyl 8-cyclohexyl-5-((cyclopropylsulfonyl)carbamoyl)-11-methoxy-1,12b-dihydrocyclopropa[d]indolo[2,1-a][2]benzazepine-1a(2H)-carboxylate.* To slurry of sodium hydride (60% dispersion in mineral oil, 370 mg, 9.2 mmol) in DMSO (8 mL) stirring under N₂ was added trimethylsulfoxonium iodide (2.03 g, 9.2 mmol). The reaction mixture was stirred for 45 min and then methyl 13-cyclohexyl-10-((cyclopropylsulfonyl)carbamoyl)-3-methoxy-7*H*-indolo[2,1-*a*][2]benzazepine-6-carboxylate (2.2 g, 4.0 mmol) in DMSO (5 mL) was added (flask rinsed with DMSO (2 x 3 mL)). The reaction mixture was stirred 1h, poured into 0.25N HCl (100 mL), and extracted with EtOAc (150 mL). The organic layer was washed with brine (20 mL) and the combined aqueous layers were extracted with EtOAc (100 mL). The combine organic layers were washed with brine (∼20 mL), dried (MgSO₄), filtered and concentrated to dryness. The residue was stirred with EtOAC/Et₂O (1:3, 50 mL) and the solids were removed by filtration. The motherliquor was concentrated and dried under high vacuum to yield methyl 8-cyclohexyl-5-((cyclopropylsulfonyl)carbamoyl)-11-methoxy-1,12b-dihydrocyclopropa[*d*]indolo[2,1-*a*][2]benzazepine-1a(2*H*)-carboxylate (1.92 g, 3.4 mmol, 85%) as a yellow solid which was used without further purification. Presents as a ∼2:1 mixture of rotamers or atrope isomers. ¹HNMR (300 MHz, CD₃OD) δ 0.19 - 0.26 (m, 0.4H), 0.78 - 2.19 (m, 15.6H), 2.64 - 3.02 (m, 2H), 3.16 - 3.28 (m, 1H), 3.41 (d, *J* = 15.0 Hz, 0.6H), 3.51 (s, 1.8H), 3.80 (s, 1.2H), 3.88 (s, 3H), 4.00 (d, *J* = 15.0 Hz, 0.4H), 5.22 (d, *J* = 15.0 Hz, 0.4H), 5.42 (d, *J* = 15.0 Hz, 0.6H), 6.93 - 7.01 (m, 1H), 7.12 (d, *J* = 2.6 Hz, 0.4H), 7.19 (d, *J* = 2.6 Hz, 0.6H), 7.25 (d, *J* = 8.8 Hz, 0.6H), 7.29 (d, *J* = 8.8 Hz, 0.4H), 7.55 (dd, *J =* 8.8, 1.5 Hz, 0.6H), 7.63 (dd, *J* = 8.8, 1.5 Hz, 0.4H), 7.85 (d, *J* = 8.8 Hz, 0.6H), 7.88 (d, *J* = 8.8 Hz, 0.4H), 8.08 (d, *J* = 1.5 Hz, 0.4H), 8.31 (d, *J* = 1.5 Hz, 0.6H). LCMS: m/e 563 (M+H)⁺, ret time 3.75 min, column B, 4 minute gradient.

### Example 15

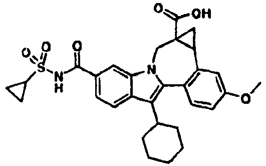

*8-Cyc*/*ohexyl-5-((cyclopropylsu*/*fonyl)carbamoyl)-11-methoxy-1,12b-dihydrocyclopropa[d]indolo[2,1-a][2]benzazepine-1a(2H)-carboxy*/*ic acid.* Methyl 8-cyclohexyl-5-((cyclopropylsulfonyl)carbamoyl)-11-methoxy-1,12b-dihydrocyclopropa[*d*]indolo[2,1-*a*][2]benzazepine-1a(2*H*)-carboxylate (1.92 g, 3.41 mmol) was dissolved into MeOH//THF (1:1, 40 mL) and treated with 1M aqueous NaOH (8 mL). The reaction mixture was stirred and heated at 60 °C for 2h and cooled to rt. The clear solution was neutralized with 1M aqueous HCl (8 mL) and concentrated to remove organic solvents. The resultant solids were collected by filtration, washed with H₂O and dried under vacuum to yield 8-cyclohexyl-5-((cyclopropylsulfonyl)carbamoyl)-11-methoxy-1,12b-dihydrocyclopropa[*d*]indolo[2,1-a][2]benzazepine-1a(2*H*)-carboxylic acid (1.66 g, 3.03 mmol, 89%) as a yellow powder which was used without further purification. Presents as a 1:1 mixture of rotamers or atrope isomers. ¹NMR (300 MHz, CDCl₃) δ 0.32 (t, *J* = 6.2 Hz, 0.5H), 0.71 - 2.12 (m, 15.5H), 2.61 - 2.94 (m, 2H), 3.16 - 3.27 (m, 1H), 3.41 (d,*J* = 15.0 Hz, 0.5H), 3.82 (s, 1.5H), 3.86 (s, 1.5H), 3.99 (d, *J* = 15.0 Hz, 0.5H), 5.28 (d, *J* = 15.0 Hz, 0.5H), 5.49 (d, *J* = 15.0 Hz, 0.5H), 6.85 (dd, *J* = 8.4, 2.6 Hz, 0.5H), 6.91 (dd, *J* = 8.4, 2.6 Hz, 0.5H), 6.96 (d, *J* = 2.6 Hz, 0.5H), 7.08 (d, *J* = 2.6 Hz, 0.5H), 7.19 (d, *J* = 8.4 Hz, 0.5H), 7.24 (d, *J* = 8.4 Hz, 0.5H), 7.61 (d, *J* = 8.4 Hz, 0.5H), 7.67 (d, *J* = 8.4 Hz, 0.5H), 7.83 (d. *J* = 8.4 Hz, 0.5H), 7.85 (d, *J* = 8.4 Hz, 0.5H), 8.06 (s, 0.5H), 8.35 (s, 0.5H), 9.31 - 10.35 (m, 1H). LCMS: m/e 547 (M-H)⁻, ret time 2.06 min, column A, 4 minute gradient.

### Example 16

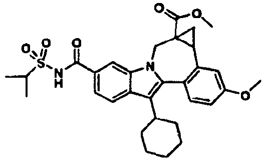

*Methyl 8-cyclohexyl-5-((isopropylsulfonyl)carbamoyl)-11-methoxy-1,12b-dihydrocyclopropa[d]indolo[2,1-a][2]benzazepine-1a(2H)-carboxylate.* To slurry of sodium hydride (60% dispersion in mineral oil, 97 mg, 2.4 mmol) in DMSO (2 mL) stirring under N₂ was added trimethylsulfoxonium iodide (530 g, 2.4 mmol). The reaction mixture was stirred for 45 min and then methyl 13-cyclohcxyl-10-((isopropylsulfonyl)carbamoyl)-3-methoxy-7*H*-indolo[2,1-a][2]benzazepine-6-carboxylate (578 g, 1.05 mmol) in DMSO (1.5 mL) was added (flask rinsed with DMSO (2 x 0.75 mL)). The reaction mixture was stirred 1h, poured into 0.25N HCl (25 mL), and extracted with EtOAc (40 mL). The organic layer was washed with brine (10 ml) and the combined aqueous layers were extracted with EtOAc (25 mL). The combine organic layers were washed with brine (∼10 mL), dried (MgSO₄). filtered and concentrated to dryness. The residue was stirred with EtOAc/Et₂O (1:4, 10 mL) and the solids were removed by filtration. The motherliquor was concentrated and dried under high vacuum to yield methyl 8-cyclohexyl-5-((isopropylsulfonyl)carbamoyl)-11-methoxy-1,12b-dihydrocyclopropa[d]indolu[2,1-a][2]benzazepine-1a(2*H*)-carboxylate (620 mg, 1.0 mmol, quant.) as a yellow solid which was used without further purification. Presents as a ∼2:1 mixture of rotamers or atrope isomers. ¹HNMR (300 MHz, CDCl₃) δ 0.32 - 0.39 (m, 0.4H), 0.77 - 2.09 (m, 17.6H), 2.60 - 2.96 (m, 2H), 3.41 (d, *J* = 15.0 Hz, 0.6H), 3.53 (s, 1.8H), 3.79 (s, 1.2H), 3.87 (s, 3H), 4.02 - 4.14 (m, 1.4H), 5.14 (d, *J* = 15.0 Hz, 0.4H), 5.39 (d, *J* = 15.0 Hz, 0.6H), 6.89 (dd, *J* = 8.4, 2.6 Hz, 0.4H), 6.91 (dd, *J* = 8.4, 2.6 Hz, 0.6H), 7.00 (d, *J* = 2.6 Hz, 0.4H), 7.11 (d, *J* = 2.6 Hz, 0.6H), 7.23 (d, *J* = 8.4 Hz, 0.6H), 7.25 (d, *J* = 8.4 Hz, 0.4H), 7.38 (dd, *J* = 8.4, 1.5 Hz, 0.6H), 7.43 (dd, *J* = 8.4, 1.5 Hz, 0.4H), 7.83 (d, *J* = 8.4 Hz, 0.6H), 7.86 (d, *J* = 8.4 Hz, 0.4H), 7.96 (d, *J* = 1.5 Hz, 0.4H), 8.20 (d, *J* = 1.5 Hz, 0.6H), 8.39 (s, 0.4H), 8.43 (s, 0.6H). LCMS: m/c 563 (M-H)⁻, ret time 3.00 min, column A, 4 minute gradient.

### Example 17

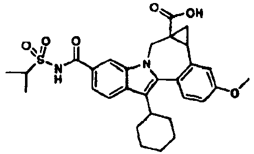

*8-Cyclohexyl-5-((isopropylsulfonyl)carbamoyl)-11-methoxy-1,12b-dihydrocyc*/*opropa[d]indolo[2,1-a][2]benzazepine-1a(2H)-carboxylic acid.* Methyl 8-cyclohexyl-5-((isupropylsulfonyl)carbamoyl)-11-methoxy-1,12b-dihydrocyclopropa[d]indolo[2,1-*a*][2]benzazepine-1a(2H)-carboxylate (606 mg, 1.07 mmol) was dissolved into MeOH//THF(1:1, 14 mL) and treated with 1M aqueous NaOH (2.5 mL). The reaction mixture was stirred and heated at 60 °C for 2h and cooled to rt. The clear solution was neutralized with 1M aqueous HCl (2.5 mL) and concentrated to remove organic solvents. The residue was stirred with H₂O (10 mL) overnight and the resultant solids were collected by filtration, washed with H₂O and dried under vacuum to yield 8-cyclohexyl-5-((isopropylsulfonyl)carbamoyl)-11-methoxy-1,12b-dihydrocyclopropa[*d*]indolo[2,1-*a*][2]benzazepine-1a(2*H*)-carboxylic acid (530 mg, 0.96 mmol, 90%) as a bright yellow solid which was used without further purification. Presents as a ∼2:1 mixture of rotamers or atrope isomers. ¹HNMR (300 MHz, CD₃OD) δ 0.23 - 0.30 (m, 0.4H), 0.80 - 2.24 (m, 17.6H), 2.70 - 3.11 (m, 2H), 3.46 (d, *J* = 15.0 Hz, 0.6H), 3.95 (s, 3H), 3.93 - 4.10 (m, 1.4H), 5.29 (d, *J* = 15.0 Hz, 0.4H), 5.48 (d, *J* = 15.0 Hz, 0.6H), 6.98 - 7.05 (m, 1H), 7.16 (d, *J* = 2.6 Hz, 0.4H), 7.23 (d, *J* = 2.6 Hz, 0.6H), 7.29 (d, *J* = 8.8 Hz, 0.6H), 7.33 (d, *J* = 8.8 Hz, 0.4H). 7.56 (dd, *J* = 8.8, 1.5 Hz, 0.6H), 7.64 (dd, *J* = 8.4, 1.5 Hz, 0.4H), 7.87 (d, *J* = 8.8 Hz, 0.6H), 7.92 (d, *J* = 8.4 Hz, 0.4H), 8.13 (d, *J* = 1.5 Hz, 0.4H), 8.31 (d, *J* = 1.5 Hz, 0.6H). LCMS: m/e 551 (M+H)⁻, ret time 3.74 min, column B, 4 minute gradient.

### Example 18

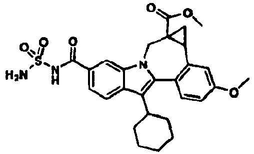

*Methyl 5-((aminosulfonyl)carbamoyl)-8-cyclohexyl-11-methoxy-1,12b-dihydrocyclopropa[d]indolo[2,1-a][2]benzazepine-1a(2H)-carboxylate.* To slurry of sodium hydride (60% dispersion in mineral oil, 350 mg, 8.8 mmol) in DMSO (8 mL) stirring under N₂ was added trimethylsulfoxonium iodide (1.93 g, 8.8 mmol) in three portions. The reaction mixture was stirred for 0.5h and then methyl 10-((aminosulfonyl)carbamoyl)-13-cyclohexyl-3-methoxy-7*H*-indolo[2,1-*a*][2]benzazepine-6-carboxylate (2.0 g, 3.8 mmol) in DMSO (8 mL) was added (flask rinsed with DMSO (2 x 2 mL)). The reaction mixture was stirred I h, poured into 0.25N HCl (100 mL), and diluted with CH₂Cl₂ (100 mL). The solution was filtered to collect solids, and the organic layer of the motherliquor was separated and concentrated to dryness. The residue was dissolved into EtOAc (∼150 mL) was washed with H₂O (∼50 mL) and brine (∼50 mL) dried (MgSO₄), filtered and concentrated to dryness. The residue was stirred with EtOAc/Et₂O (4:1, 50 mL) and the solids were collected by filtration and washed with EtOAc. These solids were combined with the initially collected solids to yield methyl 5-((aminusulfonyl)carbamoyl)-8-cyclohexyl-11-methoxy-1,12b-dihydrocyclopropa[*d*]indolo[2,1-*a*][2]benzazepine-1a(2*H*)-carboxylate (1.39 g, 2.6 mmol, 68%) as a tan solid which was used without further purification. Presents as a 1:1 mixture of rotamers or atrope isomers. ¹HNMR (300 MHz, DMSO-d₆) δ 0.13 - 0.21 (m.0.5H), 1.06 - 2.12 (m, 11.5H), 2.64 - 2.94 (m, 2H), 3.46 (s, 1.5H), 3.49 (d, *J* = 15.0 Hz, 0.5H), 3.75 (s, 1.5H), 3.85 (s, 3H), 4.02 (d, *J* = 15.0 Hz, 0.5H), 5.21 (d, *J* = 15.0 Hz, 0.5H), 5.42 (d, *J* = 15.0 Hz, 0.5H), 6.99 - 7.09 (m, 1H), 7.17 - 7.31 (m, 1H), 7.41 (s, 0.5H), 7.43 (s, 0.5H), 7.66 - 7.56 (m, 1H), 7.82 (d, *J* = 8.4 Hz, 0.5H), 7.87 (d, *J* = 8.8 Hz, 0.5H), 8.25 (s, 0.5H), 8.47 (s, 0.5H), 11.62 (s, 0.5H), 11.69 (s, 0.5H), LCMS: m/c 538 (M+H)⁻, ret time 3.56 min, column B, 4 minute gradient.

### Example 19

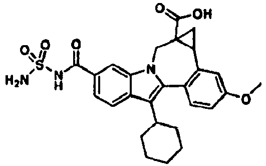

*5-((Aminosu*/*fony*/*)carbamoy*/*)-8-cyclohexyl-11-methoxy-1,12b-dihydrocyclopropa[d]indolo[2,1-a][2]benzazepine-1a(2H)-carboxylic acid.* Methyl 5-((aminosulfonyl)carbamoyl)-8-cyclohexyl-11-methoxy-1, 12b-dihydrocyclopropa[*d*]indolo[2,1-a][2]benzazepine-1a(2H)-carboxylate (1.1 mg, 2.0 mmol) was dissolved into MeOH//THF (1:1,24 mL) and treated with 1M aqueous NaOH (5 mL). The reaction mixture was stirred and heated at 60 °C for 2h and cooled to rt. The clear solution was neutralized with 1M aqueous HCl (5 mL) and concentrated to remove organic solvents. The residue was stirred with H₂O (10 mL) for 1h and the resultant solids were collected by filtration, washed with H₂O and dried under vacuum to yield 5-((aminosulfonyl)carbamoyl)-8-cyclohexyl-11-methoxy-1,12b-dihydrocyclopropa[*d*]indolo[2,1-*a*][2]benzazepine-1a(2H)-carboxylic acid (1.05 mg, 2.0 mmol, 98%) as a light yellow solid which was used without further purification. Presents as a 1:1 mixture of rotamers or atrope isomers. ¹HNMR (300 MHz, DMSO-d₆) δ 0.08 - 0.17 (m, 0.5H), 0.79 - 2.13 (m, 11.5H), 2.65 - 2.94 (m, 2H), 3.44 (d, *J* = 14.6 Hz, 0.5H), 3.85 (s, 3H), 3.96 (d, *J* = 14.6 Hz, 0.5H), 5.20 (d, *J* = 14.6 Hz, 0.5H), 5.40 (d, *J* = 14.6 Hz, 0.5H), 6.98 - 7.08 (m, 1H), 7.17 - 7.46 (m, 4H), 7.58 (d, *J* = 8.1 Hz, 0.5H), 7.62 (d, *J* = 8.1Hz, 0.5H), 7.81 (d, *J* = 8.8 Hz, 0.5H), 7.87 (d, *J* = 8.8 Hz, 0.5H), 8.25 (s, 0.5H), 8.44 (s, 0.5H), 11.48 - 13.19 (m, 2H). LCMS: m/c 524 (M+H)⁻, ret time 3.51 min, column B, 4 minute gradient.

### Example 20

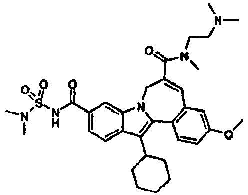

*13-Cyclohexyl-N°-(2-(dimethylamino)ethyl)-N¹⁰-((dimethylamino)sulfonyl)-3-methoxy-N⁶-methyl-7H-indolo[2,1,-a][2]benzazepine-6,10-dicarboxamide.* Methyl 13-cyclohexyl-10-(((dimethylamino)sulfonyl)carbamoyl)-3-methoxy-7*H*-indolo[2,1-*a*][2]benzazepine-6-carboxylate (700 mg, 1.27 mmol) was dissolved into MeOH//THF (1:1, 14 mL) and treated with 1M aqueous NaOH (3 mL). The reaction mixture was stirred and heated at 80 °C with microwave irradiation for 15 min and cooled to rt. The clear solution was neutralized with I M aqueous HCl (3 mL) and concentrated to remove organic solvents. The residue was stirred with H₂O (10 mL) for 1h and the resultant solids were collected by filtration, washed with H₂O and dried under vacuum. To a solution of these solids, *N*¹,*N*¹,*N*²-trimethylethane-1,2-diamine (191 mg, 1.88 mmol) and triethylamine (0.700 mL) in DMF (5 mL) was added HATU (620 mg, 1.63 mmol). The reaction mixture was stirred at rt for 1h, diluted with H₂O (25 mL) and 1M HCl (aq.) (5 mL) and stirred 20 The precipitates were collected by filtration, rinsed with H₂O and dried to yield impure 13-cyclohexyl-*N*⁶-(2-(dimethylamino)ethyl)-*N*¹⁰-((dimethylamino)sulfonyl)-3-methoxy-*N*⁶-methyl-7*H*-indolo[2,1-*a*][2]benzazepine-6,10-dicarboxamide (960 mg) as a yellow power. A crude sample of 13-cyclohexyl-*N*⁶-(2-(dimethylamino)ethyl)-N¹⁰-((dimethylamino)sulfonyl)-3-methoxy-*N*⁶-methyl-7*N*-indolo[2,1-*a*][2]benzazepine-6,10-dicarboxamide (100 mg) was dissolved into MeOH/DMF (3:1, 4 mL), tiltcrcd and purified by preparative HPLC (H₂O/CH₃CN with 10mM NH₄OAc buffer) to yield 13-cyclohexyl-*N*⁶-(2-(dimethylamino)ethyl)-N¹⁰-((dimethylamino)sulfonyl)-3-methoxy-*N*⁶-methyl-7*H*-indolo[2,1-*a*][2]benzazepine-6,10-dicarboxamide (78 mg, 0.13 mmol, 95%) ¹HNMR (300 MHz, CD₃OD) δ 1.11 - 3.07 (m, 24H), 2.99 (s, 6H), 3.50 - 3.76 (m, 2H), 3.93 (s, 3H), 4.27 - 4.44 (m, 1H), 5.05 - 5.25 (m, 1H), 7.05 (s, 1H) 7.0 (d, *J* = 2.6Hz, 1H), 7.16 (dd, *J* = H.13, 2.6Hz, 1H), 7.56 (d, *J* = 8.4Hz, 1H), 7.64 (dd, *J* = 8.8, 1.5 Hz, 1H), 7.88 (d, *J* = 8.4 Hz, 1H), 8.16 (br s, 1H). LCMS: m/e 620 (M-H)⁻, rct time 2.32 min, column A, 4 minute gradient.

### Example 21

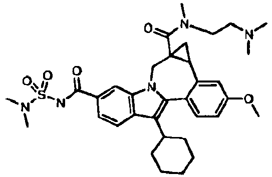

*8-Cyclohexyl-N^{1a}-(2-(dimethylamino)ethyl)-N⁵-((dimethylamino)sulfonyl)-11-methoxy-N^{1a}-methy*/*-1,12^{b}-dihydrocyclopropa[d]indolo[2,1-a][2]benzazepine-1a,5(2H)-dicarboxamide.* To a solution of 8-cyclohcxyl-5-(((dimethylamino)sulfonyl)carbamoyl)-11-methoxy-1,12b-dihydrocyclopropa[d]indolo[2,1-*a*][2]benzazepine-1a(2*H*)-carboxylic acid (120 mg, 0.22 mmol) and *N*¹,*N*¹,*N*²-trimethylethane-1,2-diamine (29 mg, 0.28 mmol) in DMF (2 mL) and TEA (0.1 mL) was added HATU (108 mg, 0.28 mmol). The reaction was stirred at rt for 3h, diluted with MeOH and purified by prep HPLC (H₂O/MeOH with 0.1% TFA buffer) to yield 8-cyclohexyl-*N*^{1a}-(2-(dimethylamino)ethyl)-*N*⁵-((dimethylamino)sulfonyl)-11-methoxy-*N*^{1a}-methyl-1,12b-dihydrocyclopropa[*d*]indolo[2,1-*a*][2]benzazepine-1a,5(2*H*)dicarboxamide (104 mg, 0.16 mmol, 74%) as a yellow solid. Prcscnts as a 1:4 mixture of rotamers or atrope isomers. ¹H NMR (300 MHz, McOD) δ ppm 8.05 - 8.12 (m, 0.2H), 7.86 - 7.98 (m, 1.8H), 7.59 (dd, *J*=8.4, 1.5 Hz, 0.8H), 7.53 - 7.64 (m, 0.2H), 7.34 (d, *J*=8.4 Hz, 1H), 7.15 - 7.22 (m, 1H), 7.04 (dd, *J*=8.4, 2.6 Hz, 1H), 5.11 (d, *J*=15.4 Hz, 0.8H), 4.80 - 4.90 (m, 0.2H), 4.10 - 4.20 (m, 0.2H), 3.92 (s, 0.6H), 3.91 (s, 2.4H), 3.08 - 3.86 (m, 4.8H), 3.04 (s, 4.8H), 3.03 (s, 1.2H), 2.54 - 3.05 (m, 11H), 1.06 - 2.21 (m, 11.8H), 0.13 -0.23 (m, 0.2H). LCMS: m/e 636 (M+H)⁺, Column A, Gradient time: 2 min, ret time 1.20 min. LCMS: m/e 660 (M+H)⁺, Column C, Gradient time: 2 min, ret time 1.91 min.

### Example 22

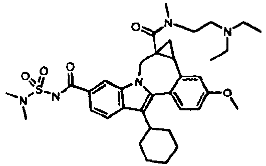

*8-Cyclohexyl-N^{1a}-(2-(diethylamino)ethyl)-N⁵-((dimethylamino)sulfonyl)-11-methoxy-N^{1a}-methyl-1,12b-dihydrocyclopropa[d]indolo[2,1-a][2]benzazepine-1a,5(2H)-dicarboxamide.* To a solution of 8-cyclohexyl-5-(((dimethylamino)sulfonyl)carbamoyl)-11-methoxy-1,12b-dihydrocyclopropa[d]indolo[2,1-*a*][2]benzazepine-la(2*H*)-carboxylic acid (120 mg, 0.22 mmol) and *N*¹,*N*¹-diethyl-*N*²-methylethane-1,2-diamine (31 mg, 0.24 mmol) in DMF (2 mL) and TEA (0.1 mL) was added HATU (90 mg, 0.24 mmol). The reaction was stirred at rt for 16h, diluted with MeOH and purified by prep HPLC (H₂O/MeOH with 0.1% TFA buffer) to yield 8-cyclohexyl-*N*^{1a}-(2-(diethylamino)ethyl)-*N*⁵-((dimethylamino)sulfonyl)-11-methoxy-*N*^{1a}-methyl-1,12b-dihydrocyclopropa[*d*]indolo[2,1-*a*][2]benzazepine-1a,5(2*H*)-dicarboxamide (87 mg, 0.13 mmol, 73%) as a yellow solid. Presents as a 1:7 mixture of rotamers or atrope isomers. ¹H NMR (300 MHz, MeOD) δ ppm 8.08 - 8.12 (m, 0.12H), 7.98 (s, 0.88H), 7.93 (d, *J*=8.4 Hz, 1H), 7.60 (d, *J*=8.4 Hz, 0.88H), 7.58 - 7.64 (m, 0.12H), 7.35 (d, *J*=8.4 Hz, 1H), 7.20 (s, 1H), 7.04 (dd, *J*=8.4, 2.2 Hz, 1H), 5.14 (d, *J*=15.4 Hz, 0.88H), 4.77 - 4.91 (m, 0.121-1), 4.13 - 4.23 (m, 0.12H), 3.92 (s, 0.36H), 3.91 (s. 2.64H), 3.04 (s, 6H), 2.52 - 3.87 (m, 13.88H), 0.99 - 2.20 (m, 17.88H), 0.16 - 0.25 (m, 0.12H). LCMS: m/e 664 (M+H)⁺, Column A, Gradient time: 2 min, ret time 1.28 min.

### Example 23

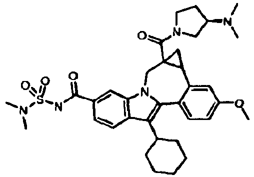

*8-Cyclohexyl-1a-(((3R)-3-(dimethylamino)-1-pyrrolidinyl)carbonyl)-N-((dimethylamino)sulfonyl)-11-methoxy-1,1a,2,12b-tetrahydrocyclopropa[d]indolo[2,1-a][2]benzazepine-5-carboxamide.* To a solution of 8-cyclohexyl-5-(((dimethylamino)sulfonyl)carbamoyl)-11-methoxy-1,12b-dihydrocyclopropa[*d*]indolo[2,1-*a*][2]benzazepine-1a(2*H*)-carboxylic acid (60 mg, 0.11 mmol) and (R)-*N,N*-dimethylpyrrolidin-3-amine (16 mg, 0,14 mmol) in DMF (1 mL) and TEA (0.06 mL) was added HATU (54 mg, 0.14 mmol). The reaction was stirred at rt for 2h, diluted with MeOH and purified by prep HPLC (H₂O/MeOH with 0.1% TFA buffer) to yield 8-cyclohexyl-1a-(((3*R*)-3-(dimethylamino)-1-pyrrolidinyl)carbonyl)-*N*-((dimethylamino)sulfonyl)-11-methoxy-1,1a,2,12b-tetrahydrocyclopropa[*d*]indolo[2,1-*a*][2]benzazepine-5-carboxamide (66 mg, 0.10 mmol, 93%) as a yellow solid. Presents as a 3:1 mixture of diatereomers. ¹H NMR (300 MHz, MeOD) δ ppm 8.12 (br s, 0.25H), 7.86 - 8.01 (m, 1.75H), 7.57 - 7.65 (m, 0.25H), 7.59 (d, *J*=8.4 Hz, 0.75H), 7.34 (d, *J*=8.4 Hz, 0.75H), 7.27 - 7.35 (m, 0.25H), 7.16 - 7.23 (m, 1H), 7.04 (dd, *J*=8.4, 2.6 Hz, 0.75H), 6.96 - 7.04 (m, 0.25H), 5.13 (dd, *J*=15.4, 2.2 Hz, 0.75H), 4.15 - 4.24 (m, 0.25H), 3.92 (s, 0.75H), 3.90 (s, 2.25H), 3.62 (dd, *J*=15.4, 4.4 Hz, 0.75H), 3.40 - 4.11 (m, 3.25H), 3.14 - 3.29 (m, I H), 3.03 (s, 1.5H), 3.04 (s, 4.5H), 2.83 - 2.98 (m, 6H), 2.47 - 3.09 (m, 3H), 1.03 - 2.40 (m, 13.75H), 0.09 - 0.22 (m, 0.25H), LCMS: m/e 648 (M+H)⁺. Column A, Gradient lime: 2 min. rct time 1.14 min.

### Example 24

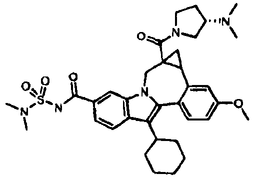

*8-Cyclohexyl-1a-(((3S)-3-(dimethy*/*amino)-1-pyrrolidiny*/*)carbony*/*)-N-((dimethylamino)su*/*fonyl)-11-methoxy-1,1a,2,12b-tetrahydrocyclopropa[d]indolo[2,1-a][2]benzazepine-5-carboxamide.* To a solution of 8-cyclohexyl-5-(((dimethylamino)sulfonyl)carbamoyl)-11-methoxy-1,12b-dihydrocyclopropa[*d*]indolo[2,1-*a*][2]benzazepine-1a(2*H*)-carboxylic acid (60 mg, 0.11 mmol) and (*S*)-*N*,*N*-dimethylpyrrolidin-3-amine (16 mg, 0.14 mmol) in DMF (1 mL) and TEA (0.06 mL) was added HATU (54 mg, 0.14 mmol). The reaction was stirred at rt for 1h, diluted with McOH and purified by prep HPLC (H₂O/MeOH with 0.1% TFA buffer) to yield 8-cyclohexyl-1a-(((3S)-3-(dimethylamino)-1-pyrrolidinyl)carbonyl)-*N*-((dimethylamino)sulfonyl)-11-methoxy-1,1a,2,12b-tetrahydrocyclopropa[*d*]indolo[2,1-*a*][2]benzazepine-5-carboxamide (66 mg, 0.10 mmol, 93%) as a yellow solid. Presents as a 3:1 mixture of diastercomers. ¹H NMR (300 MHz, MeOD) δ ppm 8.13 (br s, 0.25H), 7.86 -8.01 (m, 1.75H), 7.57 - 7.65 (m, 0.25H), 7.59 (d, *J*=8.4 Hz, 0.75H), 7.35 (d, *J*=8.4 Hz, 0.75H); 7.27 - 7.35 (m, 0.25H), 7.16 - 7.23 (m, 1H), 7.04 (dd, *J*=8.4, 2.6 Hz, 0.75H), 6.96 - 7.04 (m, 0.25H), 5.14 (d, *J*=15.4, 0.75H), 4.15 - 4.24 (m, 0.25H), 3.92 (s, 0.75H), 3.91 (s, 2.25H), 3.62 (d, *J*=15.4, 4.4 Hz, 0.75H), 3.40 - 4.11 (m, 3.25H), 3.14 - 3.29 (m, 1H), 3.03 (s, 1.5H), 3.04 (s, 4.5H), 2.83 - 2.98 (m, 6H), 2.47 - 3.09 (m, 3H), 1.03 - 2.40 (m, 13.75H), 0.09 - 0.22 (m, 0.25H). LCMS: m/e 648 (M+H)⁺, Column A, Gradient time: 4 min, ret time 2.13 min.

### Example 25

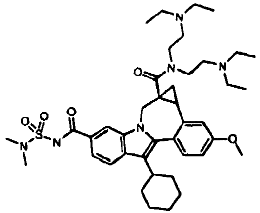

*8-Cyclohexyl-N^{1a},N^{1a}-bis(2-(diethylamino)ethy*/*)-N⁵*-*((dimethylamino)sulfonyl)-11-methoxy-1,12b-dihydrocyclopropa[d]indolo[2,1-a][2]benzazepine-1a,5(2H)-dicarboxamide.* To a solution of 8-cyclohcxyl-5-(((dimethylamino)sulfonyl)carbamoyl)-11-methoxy-1,12b-dihydrocyclopropa[*d*]indolo[2,1-*a*][2]benzazepine-1a(2*H*)-carboxylic acid (60 mg, 0.11 mmol) and *N*¹-(2-(diethylamino)ethyl)-*N*²,*N*²-diethylethane-1,2-diamine (31 mg, 0.14 mmol) in DMF (1 mL) and TEA (0.06 mL) was added HATU (54 mg, 0.14 mmol). The reaction was stirred at rt for 1h, diluted with MeOH and purified by prep HPLC (H₂O/MeOH with 0.1% TFA buffer) to yield 8-cyclohexyl-*N*^{1a},*N*^{1a}-bis(2-(diethylamino)ethyl)-*N*⁵-((dimethylamino)sulfonyl)-11-methoxy-1,12b-dihydrocyclopropa[*d*]indolo[2,1-*a*][2]benzazepine-1a,5(2*H*)-dicarboxamide(78 mg, 0.10 mmol, 93%) as a yellow solid. Presents as a 1:5 mixture of rotamers or atrope isomers. ¹H NMR (300 MHz, MeOD) δ ppm 8.20 (d, *J*=1.5 Hz, 0.16H), 8.00 (d, *J*=1.5 Hz, 0.84H), 7.93 (d, *J*=8.4 Hz, 0.84H), 7.92 - 7.98 (m, 0.16H), 7.63 (dd, *J*=8.4, 1.5 Hz, 0.16H), 7.58 (dd, *J*=8.4, 1.5 Hz, 0.84H), 7.34 - 7.39 (m, 0.16H), 7.35 (d, *J*=8.4 Hz, 0.84H), 7.24 (d, *J*=2.6 Hz, 0.16H), 7.20 (d, *J*=2.6 Hz, 0.84H), 7.05 (dd, *J*=8.4, 2.6 Hz, 1H), 5.13 (d, *J*=15.4 Hz, 0.84H), 4.86 - 5.02 (m, 0.16H), 4.06 - 4.38 (m, 2H), 3.94 (s, 0.48H), 3.91 (s, 2.52H), 3.70 (d, *J*=15.4 Hz, 0.84H), 3.06 - 3.86 (m, 11.16H), 3.03 (s, 6H), 2.48 - 3.01 (m, 4H), 0.85 - 2.19 (m, 24.84H), 0.20 - 0.28 (m, 0.16H). LCMS: m/e 749 (M+H)⁺, Column A, Gradient time: 4 min, ret time 2.77 min.

### Example 26

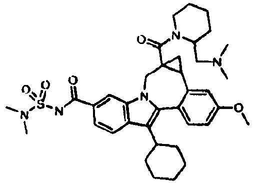

*rac-(1aR,12bS)-8-Cyclohexyl-1a-(((2S)-2-((dimethylamino)methyl)-1-piperidinyl)carbonyl)-N-((dimethylamino)sulfonyl)-11-methoxy-1,1a,2,12b-tetrahydrocyclopropa[d]indolo[2,1-a][2]benzazepine-5-carboxamide and rac-(1aR,12bS)-8-cyclohexyl-1a-(((2R)-1-((dimethylamino)methyl)-1-piperidinyl)carbonyl)-N-((dimethylamino)sulfonyl)-11-methoxy-1,1a,2,12b-tetrahydrocyclopropa[d]indolo[2,1-a][2]benzazepine-5-carboxamide.* To a solution of 8-cyclohexyl-5-(((dimethylamino)sulfonyl)carbamoyl)-11-methoxy-1,12b-dihydrocyclopropa[*d*]indulo[2,1-*a*][2]benzazepine-1a(2*H*)-carboxylic acid (60 mg, 0.11 mmol) *N,N*-dimethyl-1-(piperidin-2-yl)methanamine (20 mg, 0.14 mmol) in DMF (1 mL) and TEA (0.06 mL) was added HATU (54 mg, 0.14 mmol). The reaction was stirred at rt for 1h, diluted with MeOH and purified by prep HPLC (H₂O/MeOH with 0.1% TFA buffer) to yield two sets of diastereomeric products:
Isomer A: first eluting product: (26 mg, 0.039 mmol, 35%) as a yellow solid. Presents as a 1:6 ratio of rotamers or atrope isomers. ¹H NMR (300 MHz, MeOD) δ ppm 8.03 (s, 1H), 7.92 (d, *J*=8.4 Hz, 1H), 7.57 - 7.64 (m, 0.15H), 7.58 (dd, *J*=8.4, 1.5 Hz, 0.85H), 7.33 (d, *J*=8.4 Hz, 0.85H), 7.29 - 7.36 (m, 0.15H), 7.20 (d, *J*=2.6 Hz, 0.85H), 7.18 (d, J=2.6 Hz, 0.15H), 7.04 (dd, *J*=8.4, 2.6 Hz, 0.85H), 6.99 - 7.05 (m, 0.15H), 5.06 (d, *J*=15.4 Hz, 1H), 4.73 - 4.80 (m, 0.15H), 4.47 - 4.58 (m, 0.85H), 3.92 (s, 0.45H), 3.91 (s, 2.55H), 3.70 (d, J=15.4 Hz, 1H), 3.03 (s, 6H), 2.80 - 4.18 (m, 11H), 2.54 - 2.66 (m, 1H), 1.13 - 2.20 (m, 17.85H), 0.17 - 0.26 (m, 0.15H). LCMS: m/e 676 (M+H)⁺, Column C, Gradient time: 2 min, ret time 1.91 min.
Isomer B: second eluting product: (18 mg, 0.027 mmol, 25%) as a yellow solid. Presents as a 3:7 ratio of rotamers or atropc isomers. ¹H NMR (300 MHz, MeOD) δ ppm 8.15 (d, *J*=1.5 Hz, 0.3H), 8.00 (d, *J*=1.5 Hz, 0.7H), 7.92 (d, *J*=8.4 Hz, 1H), 7.63 (dd, *J*=8.4, 1.5 Hz, 0.3H), 7.59 (dd, *J*=8.4, 1.5 Hz, 0.7H), 7.37 (d, *J*=8.4 Hz, 0.3H), 7.33 (d, *J*=8.4 Hz, 0.7H), 7.20 (d, *J*=2.6 Hz, 0.7H), 7.17 (d, *J*=2.6 Hz, 0.3H). 7.04 (dd, *J*=8.4, 2.6 Hz, 1H), 5.11 (d, *J*=15.4 Hz, 1H), 4.66 - 4.95 (m, 1H), 4.11 (d, *J*=15.4 Hz, 0.3H), 3.92 (s, 0.9H), 3.91 (s, 2.1H), 3.67 (d, *J*=15.4 Hz, 0.7H), 3.37 - 4.15 (m, 3H), 3.04 (s, 6H), 2.52 - 3.28 (m, 9H), 1.02 - 2.20 (m, 17.7H), 0.10 - 0.21 (m, 0.3H). LCMS: m/e 676 (M+H)⁺, Column C, Gradient time: 2 min, ret time 1.95 min.

Examples 27-31 were analyzed by the following LC/MS method: Column: PHENOMENNEX-LUNA 3.0 x 50mm S10; Mobile Phase: (A) 10:90 methanol-water; (B) 90:10 methanol-water; Buffer: 0.1% TFA; Gradient Range: 0-100% B; Gradient Time: 2 min; Flow Rate: 4 mL/min; Analysis Time: 3 min; Detection: Detector 1: UV at 220 nm; Detector 2: MS (ESI+).

### Example 27

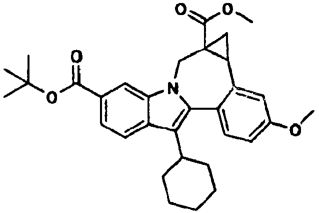

*(+*/*-)-8-Cyclohexyl-1,1a,2,12b-tetrahydro-11-methoxy-1a-(methoxycarbonyl) -cycloprop[d]indolo[2,1-a][2]benzazepine-5-carboxylic acid, tert-butyl ester.* Dry NaH (96 mg, 4 mmol) was added to a stirred suspension of trimethylsulfoxonium chloride (567 mg, 4.4 mmol) in an. DMSO (10 mL) under nitrogen. The resultant mixture was stirred at rt for 30-45 min and then neat olefin (1.0, 2 mmol) was added in small portions. The suspension was diluted with DMSO (5 mL) and heated at 50 "C for 3-4 h. Reaction mixture was allowed to cool to rt and water was added. Precipitated solid was filtered and washed with water and then air dried overnight to afford 1.15 g of crude product which was purified by flash column chromatography (silica gel, 3% MeOH in DCM), to provide pure desired cyclopropyl compound (0.96 g), as a off-white solid: LC/MS: Retention time 3.816 min; m/c 516 (MH⁺). ¹H NMR (400 MHz, CDCl₃): The product was observed to exist as inter-converting rotamers. LC/MS: Retention time 3.816 min; m/e 516 (MH⁺). ¹H NMR (400 MHz, CDCl₃): The product was observed to exist as inter-converting rotamers.

### Example 28

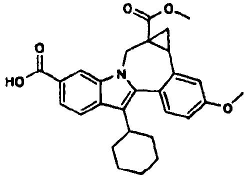

*(+*/*-)-8-Cyclohexyl-1,1a,2,12b-tetrahydro-11-methoxy-1a-(methoxycarbonyl) -cycloprop[d]indolo[2,1-a]-[2]benzazepine-5-carboxylic acid.* The *tert*-butyl ester (515 mg, 1 mmol) and TFA (5 mL) in an. DCM (10 mL) was stirred at rt until hydrolysis is complete (8-12 hr). Excess TFA and DCM were evaporated to dryness to afford desired acid (0.47g, 100%) as a light beige solid. LC/MS: Retention time 2.245 min; m/e 460 (MH⁺),. ¹H NMR (400 MHz, CDCl₃): The product was observed to exist as inter-converting rotamers. Retention time 2.245 min; m/e 460 (MH⁺). ¹H NMR (400 MHz, CDCl₃). The product was observed to exist as inter-converting rotamers.

### Example 29

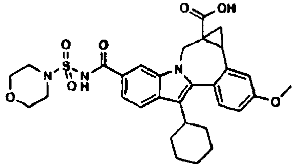

*(+*/*-)-8-cyclohexyl-5-(morpholinosulfonylcarbamoyl)-1,1a,2,12b-tetrahydro-11-methoxy-cycloprop[d]indolo[2,1-a][2]benzazepine-1a-carboxylic acid.* A mixture of acid (1 equiv) and carbonyldiimidazole (1.5 equiv) in an. THF was heated at 50 °C fur 30 min and allowed to cool to rt. Then 1 equiv of either sulfamide or sulfonamide and DBU (2 cquiv) were added consecutively. The resultant mixture was stirred at rt overnight. After acidic aqueous workup, isolated crude product was purified by prep. HPLC to afford the product. The ester moiety of was hydrolyzed using 1N NaOH in THF-MeOH to provide the corresponding acids. The acids were purified by prep HPLC and isolated. LC/MS: Retention time: 1.968 min; m/e 460 (MHz⁺). ¹H NMR (400 MHz, CDCl₃): The product was observed to exist as inter-converting rotamers.

### Example 30

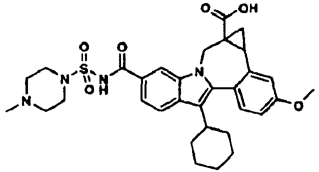

*(+*/*-)-8-cyclohexyl-5-(4-methylpiperazin-1-ylsulfonylcarbamoyl)-1,1a,2,12b-tetrahydro-11-methoxy-cycloprop[d]indolo[2,1-a][2]benzazepine-1a-carboxylic acid.* The product was purified by prep HPLC and isolated in mono TFA salt form as a beige solid. LC/MS: Retention time: 1.687 min; m/e 607 (MH⁺). ¹H NMR (400 MHz, CDCl₃): The product was observed to exist as inter-converting rotamers.

### Example 31

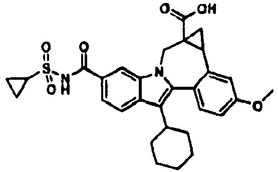

*(+*/*-)-8-cyclohexyl-5-(cyclopropylsulfonylcarbamoyl)-1,1a,2,12b-tetrahydro-11-methoxy-cycloprop[d]indolo[2,1-a][2]benzazepine-1a-carboxylic acid.* LC/MS: Retention time: 2.030 min; m/e 549 (MH⁺). ¹H NMR (400 MHz, CDCl₃): The product was observed to exist as inter-converting rotamers.

Example 32-38 were analyzed by the following LC/MS method: Start % B: 0; Final % B: 100; Gradient time: 3 min; Stop time: 4 min; Flow rate: 4 ml/min; Wavelenth: 220; Solvent A: 10% MeOH / 90% H₂O / 0.1% Trifluoroacetic Acid; Solvent B: 10% H₂O / 90% MeOH / 0.1% Trifluoroacetic Acid; Column: XBridge 4.6 x 50 mm S5.

### Example 32

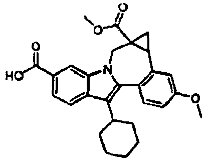

A mixture of acid (1.3 g, 2.83 mmol) and CDI (0.64 g, 3.97 mmol) in THF (20 mL) was heated at 50 °C for 0.5 h, cooled down and added methylsulfonamide (0.4 g, 4.2 mmol) and DBU (0.264 mL, 1.77 mmol). The mixture was stirred for 20 h and diluted with EtOAc, washed with cold 1N HCl (2x), brine, dried (MgSO4), removed the solvent and purified by flash (Biotage 40 M) to afford the compound 1-2 (1.28 g, 85%) as a pale yellow solid. LC-MS retention time: 3.51; MS m/z 537 (M+H). The product was observed to exist as inter-converting rotamers. The major isomer: ¹H NMR (400 MHz, CHLOROFORM-D) δ ppm 1.11 - 2.17 (m, 12 H), 2.84 - 2.98 (m, 2 H), 3.43 (d, *J*=14.86 Hz, 1 H), 3.49 (s, 3 H), 3.55 (s, 3 H), 3.89 (s. 3 H), 5.40 (d, *J*=15.11 Hz, 1 H), 6.91 - 6.96 (m, 1 H), 7.13 (d, *J*=2.52 Hz, 1 H), 7.22 - 7.27 (m, 1 H), 7.39 (dd, *J*=8.31, 1.51 Hz, 1 H), 7.85 (d, *J*=8.81 Hz, I H), 8.23 (d, *J*=1.26 Hz, 1 H), 8.75 (s, 1 H).

### Example 33

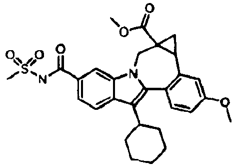

To a solution of the acid (1.28 g, 2.4 mmol) in THF (5 mL) and MeOH (5 mL) was added NaOH (1N, 12 mL, 12 mmol). After being stirred at room temperature for 3 h, the mixture was diluted with EtOAc, washed with cold 1N HCl, brine, dried (MgSO4), and removed the solvent in vacuo to afford the compound 1-3 as a beige solid (1.20 g, 96%). LC-MS retention time: 3.46; MS m/z 523 (M+H). The product was observed to exist as inter-converting rotamers (∼1/1) ¹H NMR (400 MHz, CHLOROFORM-D).

### Example 34

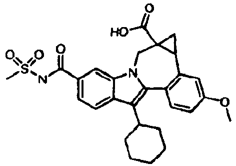

Typical general procedure for amine coupling: To a mixture of the acid (0.060g, 0.11 mmol) and a secondary / tertiary amine containing diamine bishydrochloric acid salt (0.034g, 0.17 mmol) in DMC (1.5 mL) was added Et₃N (0.096 mL, 0.69 mmol) and HBTU (0.065g, 0.17 mmol). The mixture was stirred at room temperature for 0.5 h, diluted with MeOH, removed the solvent. The residue was dissolved in methanol, filtered, and purified by prep-HPLC to afford A TFA salt of an amide (0.0378g, 82%).

### Example 35

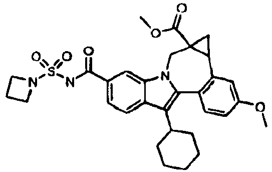

Prepared from the acid (0.96g, 59%). LC-MS retention time: 3.58; MS m/z 578 (M+H). compound was observed to exist as inter-converting rotamers (3/4). The major isomer: ¹H NMR (400 MHz, CHLOROFORM-D) δ ppm 1.16 - 1.59 (m, 4 H), 1.72 (dd, *J*=9.44, 4.15 Hz, 3 H), 1.88 - 2.12 (m, 4 H), 2.24 - 2.36 (m, 2 H), 2.75 - 2.97 (m, 2 H), 3.44 (d, *J*=14.86 Hz, 1 H), 3.56 (s, 3 H), 3.89 (s, 3 H), 4.09 (d, 1 H), 4.24 - 4.37 (m, 4 H), 5.41 (d, *J*=14.86 Hz, 1 H), 6.92 - 6.96 (m, 1 H), 7.13 (d, *J*=2.01 Hz, 1 H), 7.24 - 7.30 (m, 1 H), 7.39 (dd, *J*=8.31, 1.51 Hz, 1 H), 7.84 - 7.88 (m, 1 H), 8.24 (d, *J*=1.51 Hz, H).

### Example 36

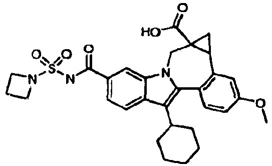

Prepared from the ester (0.93 g, 100%). LC-MS retention time: 3.51; MS m/z 564 (M+H). Compound was observed to exist as inter-converting rotamers (∼3/4). The major isomer: ¹H NMR (400 MHz, ppm 0.34 - 0.42 (m, 1 H), 1.15 - 2.10 (m, 11 H), 2.22 - 2.38 (m, 2 H), 2.65 - 2.78 (m, 1 H), 2.84 - 2.94 (m, *J*=3.02 Hz, 1 H), 3.84 (s, 3 H), 4.03 (d, *J*=15.11 Hz, 1 H), 4.21 - 4.43 (m, 4 H), 5.34 (d, *J*=14.86 Hz, 1 H), 6.87 (dd, *J*=8.56, 2.77 Hz, 1 H), 6.98 (d, *J*=2.52 Hz, 1 H), 7.21 (d, *J*=8.31 Hz, 1 H), 7.69 - 7.75 (m, 1 H), 7.86 - 7.90 (m, 1 H), 8.13 (s, 1 H).

### Example 37

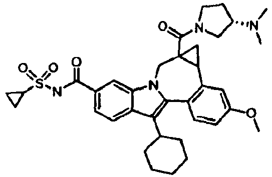

*(+*/*-)-8-cyclohexyl-N-(cyclopropylsulfony*/*)-1,1a,2,12b-tetrahydro-11-methoxy-1a-((S)-1-(3-(dimethylamino)pyrrolidin-1-yl))-8-carbony*/*)-cycloprop[d]indolo[2,1-a][2]benzazepine-5-carboxamide.* A TFA salt was prepared (0.0249 g, 36%). LC-MS retention time: 3.04; MS m/z 645 (M+H).

### Example 38

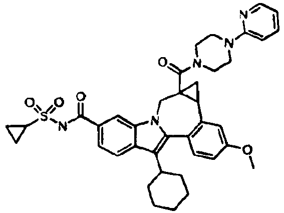

*(+*/*-)-8-cyclohexyl-N-(cyclopropylsulfonyl)-1,1a,2,12b-tetrahydro-11-methoxy-1a-(1-(4-(pyridin-2-yl)piperazin-1-yl)-8-carbonyl)-cycloprop[d]indolo[2,1-a][2]benzazepine-5-carboxamide.* A TFA salt was prepared (0.0447 g, 61%). LC-MS retention time: 3.06; MS m/z 694 (M+H). LC-MS retention time: 2.99; MS m/z 657 (M+H). Compound was observed to exist as inter-converting rotamers (∼2/1). The major isomer: ¹H NMR (400 MHz, ppm 0.92 - 2.09 (m, 16 H), 2.66 (dd, *J*=8.94. 5.92 Hz, 1 H), 2.87 - 2.98 (m, I H), 3.01 - 3.10 (m, 1 H), 3.10 - 4.09 (m, 8 H), 3.60 (d, *J*=15.36 Hz, 1 H), 3.90 (s, 3 H), 5.01 (d, *J*=15.36 Hz, 1 H), 6.86 - 6.98 (m, 2 H), 7.11 (d, *J*=2.27 Hz, 1 H), 7.26 - 7.29 (m, 1 H), 7.48 (d, *J*=8.31 Hz, 1 H), 7.81 - 7.94 (m, 2 H), 7.98 - 8.07 (m, 2 H).

### Example 39

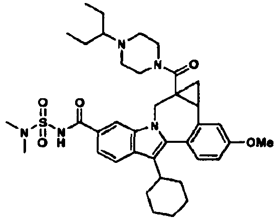

*8-Cyclohexyl-N-((dimethylamino)sulfonyl)-1a-((4-(1-ethylpropyl)-1-piperazinyl)carbonyl)-11-methoxy-1,1a,2,12b-tetrahydrocyclopropa[d]indolo[2,1-a][2]benzazepine-5-carboxamide.* To a mixture of the acid, 8-cyclohexyl-5-(((dimethylamino)sulfonyl)carbamoyl)-11-methoxy-1,12*b-*dihydrocyclopropa[*d*]indolo[2,1-*a*][2]benzazepine-1*a*(2*H*)-carboxylic acid, (50 mg, 90.6 µmol), *N,N,N',N'*-tetramethyluronium tetrafluoroborate (TBTU, 150 mg, 467 µmol) and 1-(3-pentyl)piperazine (24 mg, 153 µmol) in DMF (2 ml) at r.t. under N₂ was added *N,N*-diisopropylethylamine (0.11 ml, 631 µmol). The reaction mixture was stirred at r.t. for 4 hr. 5 min., and then concentrated. The residue was diluted with MeOH (4 ml), and purified by Shimadzu-VP preparative reverse phase HPLC with separation method: Solvent A = 10% MeOH-90% H₂O-0.1% TFA, Solvent B = 90% MeOH-10%H₂O-0.1% TFA, Start %B = 0, Final %B = 100, Gradient time = 6min, Flow Rate = 30mL/min, Column: Xtcrra Prep MS C 18 5u 30x50mm, Fraction Collection: 6.23 - 6.82 min. (UV detection at 220 nm) to give the trifluoroacetic acid salt of the product (48.3 mg) as an off white solid; Analytical HPLC method: Solvent A = 10% MeOH-90% H₂O-0.1% TFA, Solvent B = 90% MeOH-10%H₂O-0.1% TFA, Start %B = 0, Final %B = 100, Gradient time = 2 min, Flow Rate = 5 ml/min, Column: Xterra MS C18 S7 3.0 x 50mm; LC/MS: (ES+) m/z (M+H)⁺ = 690.56, HPLC Rₜ = 1.675 min. Analytical HPLC method: Solvent A = 5% MeCN-95% H₂O-10 mM NH₄OAc, Solvent B = 95% MeCN-5%H₂O-10 mM NH₄OAc, Start %B = 0, Final %B = 100, Gradient time = 2 min, Flow Rate = 5 ml/min, Column: Phenomenex Lina C18 5um 3.0 x 50mm; LC/MS: (ES+) m/z (M+H)⁺ = 690.49, HPLC Rₜ = 1.690 min.

Examples 40-43 were prepared in a similar manner to Example 39.

### Example 40

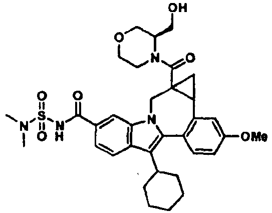

*8-Cyclohexyl-N-((dimethylamino)sulfonyl)-1a-(((3R)-3-(hydroxymethyl)-4-morpholinyl)carbonyl)-11-methoxy-1,1a,2,12b-tetrahydrocyclopropa[d]indolo[2,1-a][2]benzazepine-5-carboxamide.* Analytical H PLC method: Solvent A = 10% MeOH-90% H₂O-0.1% TFA, Solvent B = 90% MeOH-10%H₂O-0.1% TFA, Start %B = 0, Final %B = 100, Gradient time = 2 min, Flow Rate = 5 ml/min, Column: Xterra MS C18 S7 3.0 x 50mm; LC/MS: (ES+) m/z (M+H)⁺ = 651.25, HPLC Rₜ = 1.822 min. Analytical HPLC method: Solvent A = 5% MeCN-95% H₂O-10 mM NH₄OAc, Solvent B = 95% MeCN-5%H₂O-10 mM NH₄OAc, Start %B = 0, Final %B = 100, Gradient time = 2 min, Flow Rate = 5 ml/min, Column: Phenomenex Lina C18 5um 3.0 x 50mm; LC/MS: (ES+) m/z (M+H)⁺ = 651.45, HPLC Rₜ = 1.063 min.

### Example 41

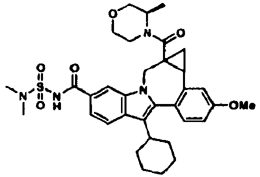

*8-Cyclohexyl-N-((dimethylamino)sulfonyl)-11-methoxy-1a-(((3R)-3-methy*/*-4-morpholinyl)carbonyl)-1,1a,2,12b-tetrahydrocyclopropa[d]indolo[2,1-a][2]benzazepine-5-carboxamide.* Analytical HPLC method: Solvent A = 10% MeOH-90% H₂O-0.1% TFA, Solvent B = 90% MeOH-10%H₂O-0.1% TFA, Start %B = 0, Final %B = 100, Gradient time = 2 min, Flow Rate = 5 ml/min, Column: Xterra MS C18 S7 3.0 x 50mm; LC/MS: (ES+) m/z (M+H)⁺ = 635.26, HPLC Rₜ = 1.867 min. Analytical HPLC method: Solvent A = 5% MeCN-95% H₂O-10 mM NH₄OAc, Solvent B = 95% MeCN-5%H₂O-10 mM NH₄OAc, Start %B = 0, Final %B = 100, Gradient time = 2 min, Flow Rate = 5 ml/min, Column: Phenomenex Lina C18 5um 3.0 x 50mm; LC/MS: (ES+) m/z (M+H)⁺ = 635.63, HPLC Rₜ = 1.113 min.

### Example 42

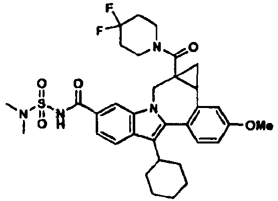

*8-Cyclohexyl-1a-((4,4-difluoro-1-piperidinyl)carbonyl)-N-((dimethylamino)sulfonyl)-11-methoxy-1,1a,2,12b-tetrahydrocyclopropa[d]indolo[2,1-a][2]benzazepine-5-carboxamide.* Analytical HPLC method: Solvent A = 10% MeOH-90% H₂O-0.1% TFA, Solvent B = 90% MeOH-10%H₂O-0.1% TFA, Start %B = 0, Final %B = 100, Gradient time = 2 min, Flow Rate = 5 ml/min, Column: Xterra MS C18 S7 3.0 x 50mm; LC/MS: (ES+) m/z (M+H)⁺ = 655.26, HPLC Rₜ = 1.922 min. Analytical HPLC method: Solvent A = 5% MeCN-95% H₂O-10 mM NH₄OAc, Solvent B = 95% MeCN-5%H₂O-10 mM NH₄OAc, Start %B = 0, Final %B = 100, Gradient time = 2 min, Flow Rate = 5 ml/min, Column: Phenomenex Lina C18 5um 3.0 x 50mm; LC/MS: (ES+) m/z (M+H)⁺ = 655.32, HPLC Rₜ = 1.212 min.

### Example 43

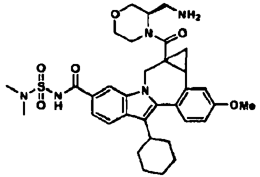

*1a-(((3R)-3-(Aminomethyl)-4-morpholinyl)carbonyl)-8-cyclohexyl-N-((dimethylamino)sulfonyl)-11-methoxy-1,1a,2,12b-tetrahydrocyclopropa[d]indolo[2,1-a][2]benzazepine-5-carboxamide.* The product was prepared as a trifluoroacetic acid salt from (8-cyclohexyl-*N-*((dimethylamino)sulfonyl)-1a-(((3*R*)-3-(hydroxymethyl)-4-morpholinyl)carbonyl)-11-methoxy-1,1a,2,12b-tetrahydrocyclopropa[*d*]indolo[2,1-*a*][2]benzazepine-5-carboxamide) via the reaction of the corresponding chloride with ammonia in MeOH under microwave irradiation at 100°C for 5 min. Analytical HPLC method: Solvent A = 10% MeOH-90% H₂O-0.1% TFA, Solvent B = 90% MeOH-10%H₂O-0.1% TFA, Start %B = 0, Final %B = 100, Gradient time = 2 min, Flow Rate = 5 ml/min, Column: Xterra MS C18 S7 3.0 x 50mm; LC/MS: (ES+) m/z (M+H)⁺ = 650.20, HPLC R₄ = 1.603 min. Analytical HPLC method: Solvent A = 5% MeCN-95% H₂O-10 mM NH₄OAc, Solvent B = 95% MeCN-5%H₂O-10 mM NH₄OAc, Start %B = 0, Final %B = 100, Gradient time = 2 min, Flow Rate = 5 ml/min, Column: Phenomenex Lina C18 5um 3.0 x 50mm; LC/MS: (ES+) m/z (M+H)⁺ = 650.02, HPLC Rₜ = 1.167 min.

The general procedures below pertain to the experimental procedures that follow until noted. The acid (0.055 mmol, 1 eq.) was dissolved in dried DMF and followed by adding HATU (0.083 mmol, 1.5 eq.) and DIPEA(0.083, 1.5 eq.). The solution was stirred for 2 minutes and added into pre-weighted amine (0.083 mmol, 1.5 eq.) at room temperature. The mixture was stirred 14 h and purified by prep-HPLC. HPLC gradient methods: Method A: Column: Agilent SB CN4.6x100mm 3.5 um; mobile phase: water, 10 mM NH₄OH, ACN; Method B: Column: Phenomenex Gemini 4.6x100mm 5 um C18; mobile phase: water, 10 mM NH₄OH, ACN; Method C: Column: Waters x-Bidge C18 150x4.6mm 5 micron; mobile phase: water, 10 mM NH₄OH, ACN; Method D: Column: Waters Xbridge 2.1x50mm 5 um C18; mobile phase: water, 10 mM NH₄OH, ACN.

| Structure | HPLC retention time | HPLC purity | Mass | HPLC Method |
|---|---|---|---|---|
| 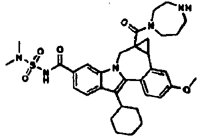 | 4.86 | 88.9 | 634.36 | Method A |
| 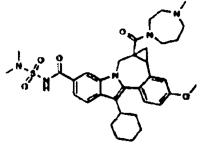 | 4.88 | 100 | 648.37 | Method A |
| 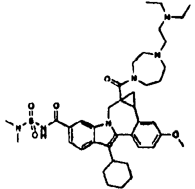 | 5.33 | 98.5 | 733.42 | Method B |
| 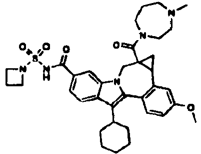 | 7.93 | 93.9 | 659.97 | Method C |
| 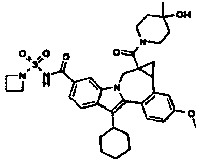 | 8.14 | 99.3 | 661.24 | Method C |
| 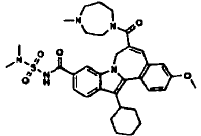 | 2.45 | 96.2 | 634.77 | Method D |
| 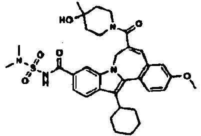 | 2.82 | 97.9 | 634.74 | Method D |
| 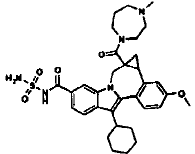 | 2.04 | 96.3 | 619.84 | Method D |
| 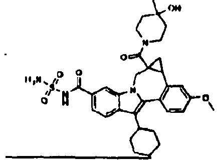 | 2.27 | 100 | 620.83 | Method D |
| 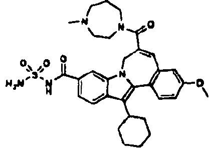 | 7.11 | 100 | 606.14 | Method D |
| 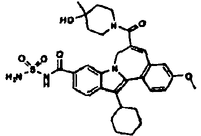 | 7.7 | 100 | 607.11 | Method D |
| 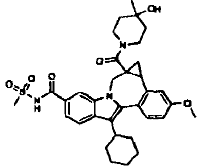 | 2.18 | 93.4 | 620.37 | Method D |
| 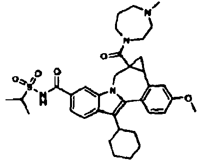 | 7.48 | 100 | 647.21 | Method D |
| 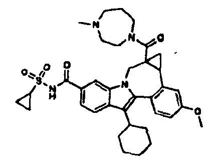 | 2.13 | 100 | 645.37 | Method D |
| 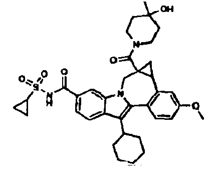 | 2.33 | 95.8 | 646.35 | Method D |
| 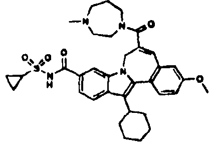 | 2.17 | 100 | 630.79 | Method D |
| 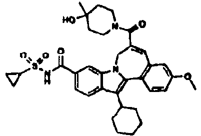 | 2.41 | 100 | 631.8 | Method D |
| 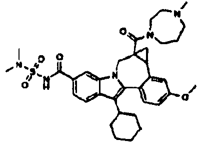 | 2.19 | 100.0 | 648.39 | Method D |
| 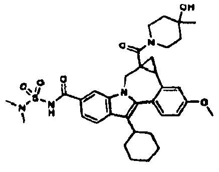 | 2.62 | 98.9 | 649.39 | Method D |

## Claims

1. A compound selected from the group consisting of 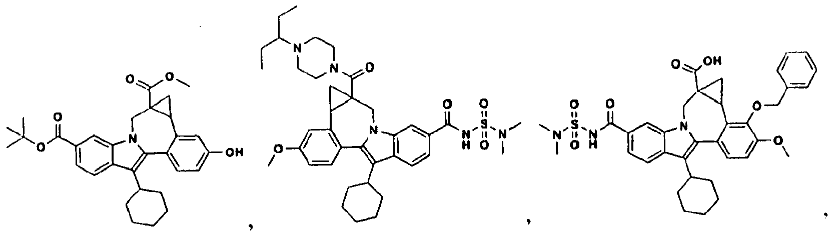 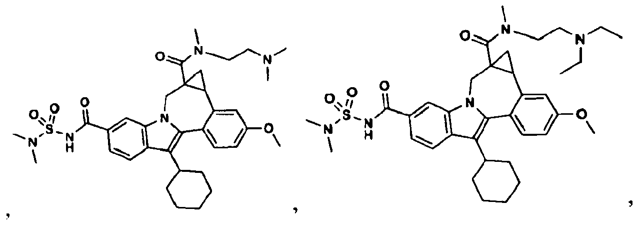 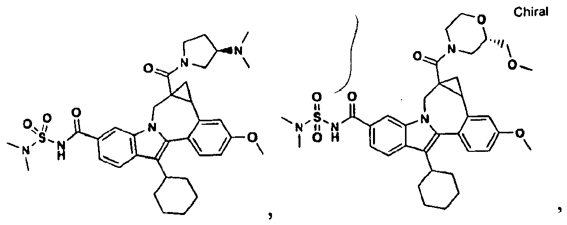 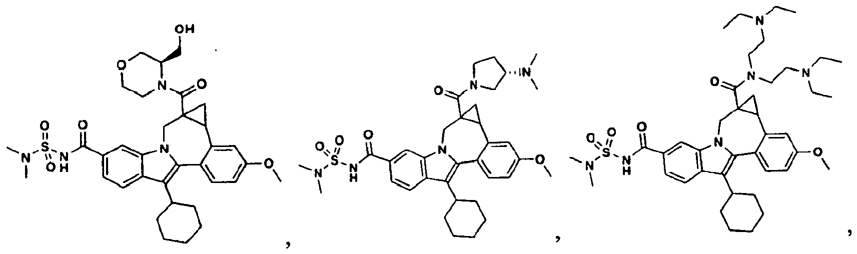 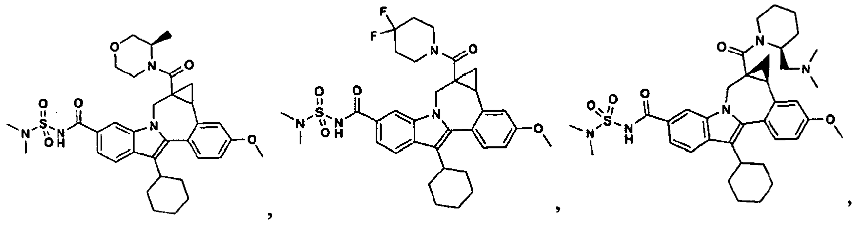 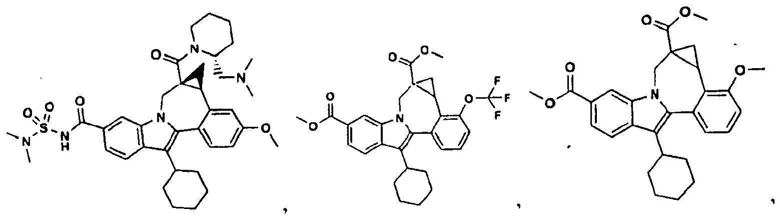 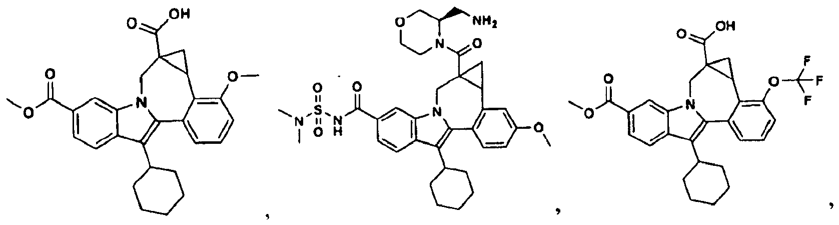 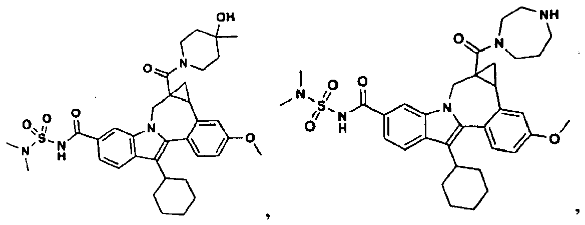 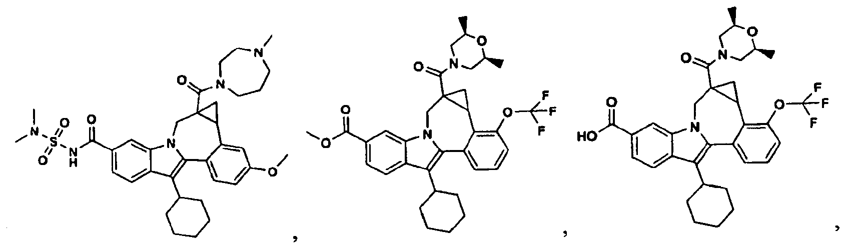 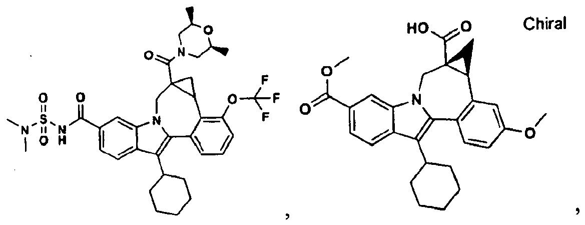 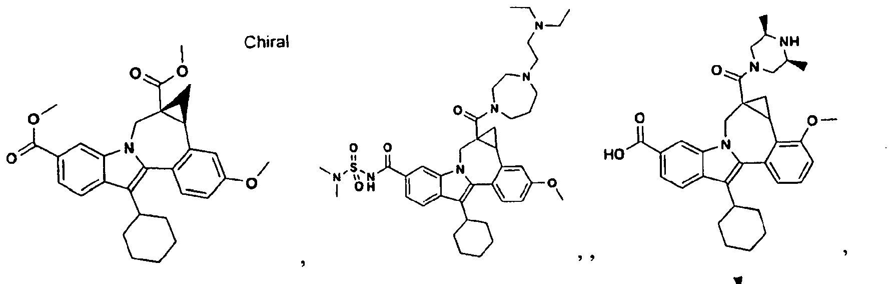 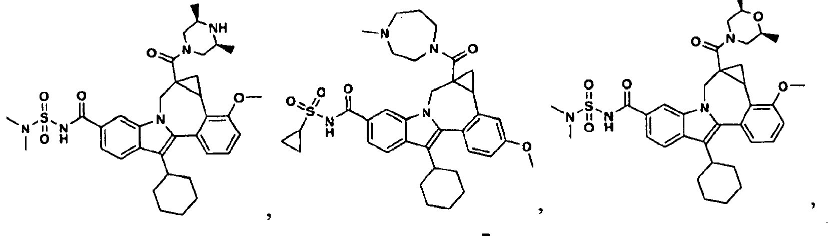 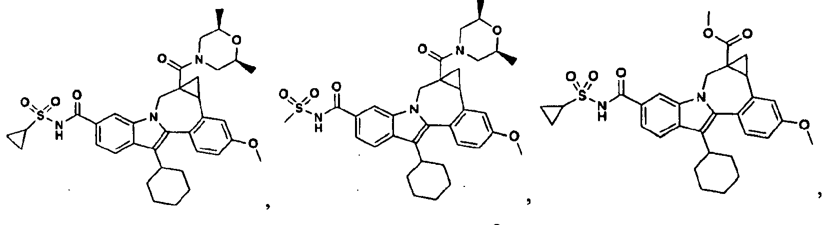 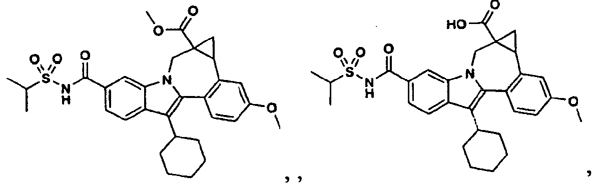 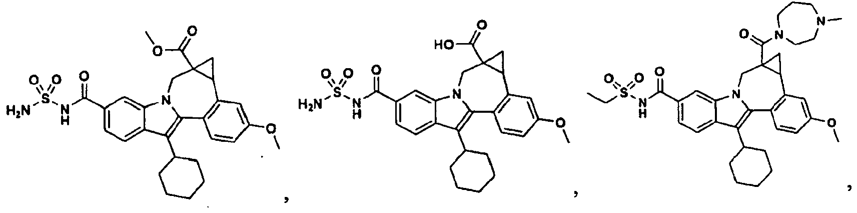 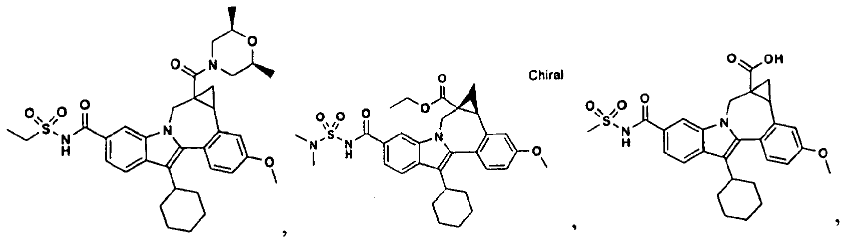 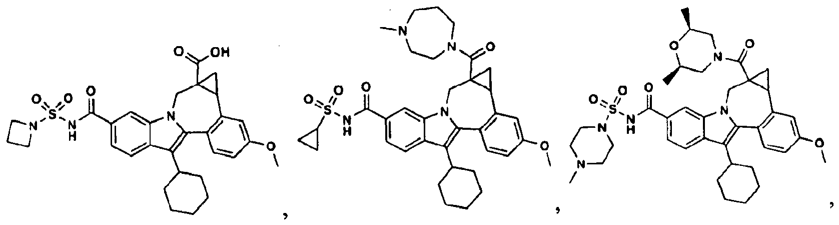 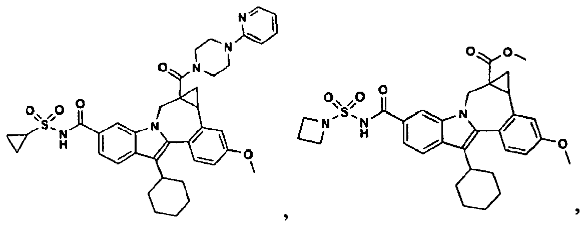 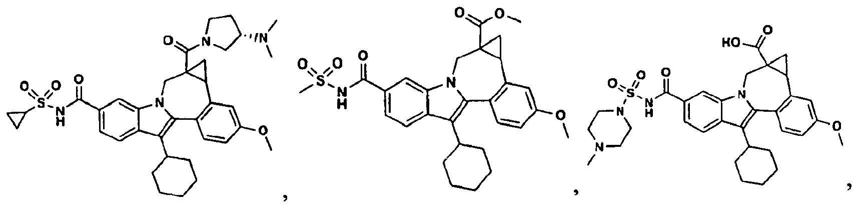 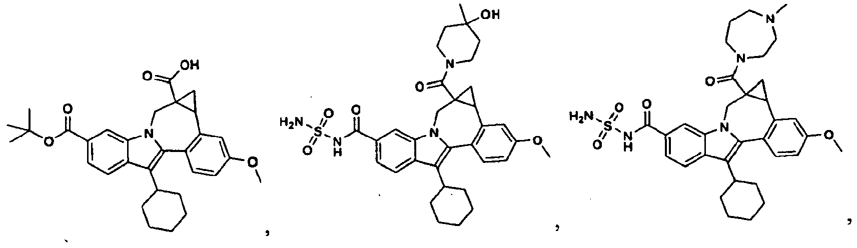 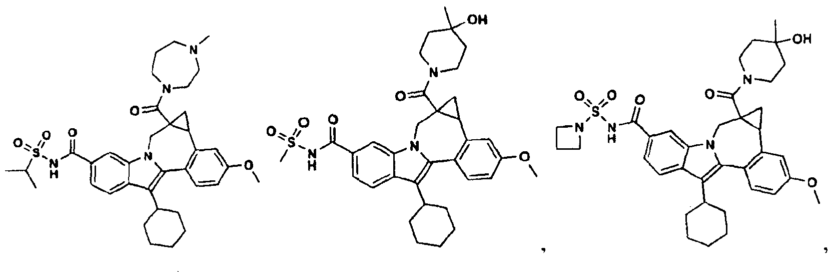 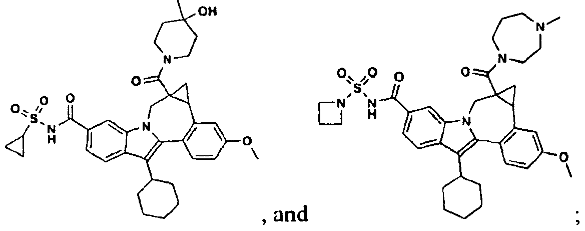 or a pharmaceutically acceptable salt thereof.

2. A composition comprising a compound of claim 1, or a pharmaceutically acceptable salt thereof, and a pharmaceutically acceptable carrier.

3. The composition of claim 2 further comprising at least one additional compound having therapeutic benefits for HCV wherein the compound is selected from the group consisting of interferons, cyclosporins, interleukins, HCV metalloprotease inhibitors, HCV serine protease inhibitors, HCV polymerase inhibitors, HCV helicase inhibitors, HCV NS4B protein inhibitors, HCV entry inhibitors, HCV assembly inhibitors, HCV egress inhibitors, HCV NS5A protein inhibitors, HCV NS5B protein inhibitors, and HCV replicon inhibitors.

4. A compound of claim 1 for use in a method of treating hepatitis C infection.

5. The compound for use according to claim 4 further comprising the use of at least one additional compound having therapeutic benefits for HCV wherein the compound is selected from the group consisting of interferons, cyclosporins, interleukins, HCV metalloprotease inhibitors, HCV serine protease inhibitors, HCV polymerase inhibitors, HCV helicase inhibitors, HCV NS4B protein inhibitors, HCV entry inhibitors, HCV assembly inhibitors, HCV egress inhibitors, HCV NS5A protein inhibitors, HCV NS5B protein inhibitors, and HCV replicon inhibitors.

## Patentansprüche

1. Verbindung, ausgewählt aus den folgenden Verbindungen: 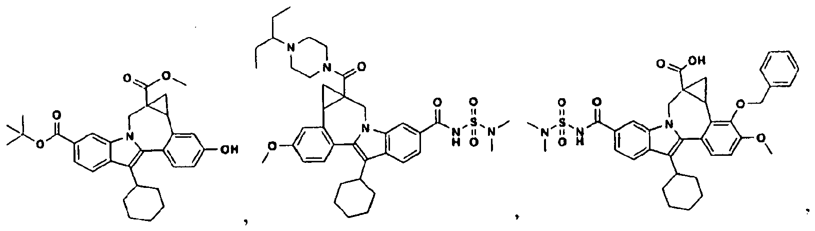 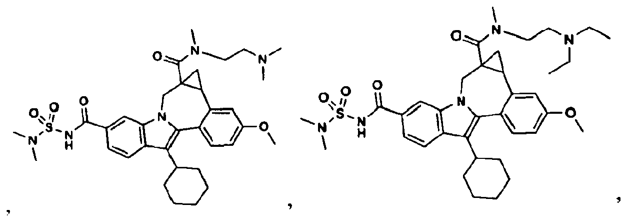 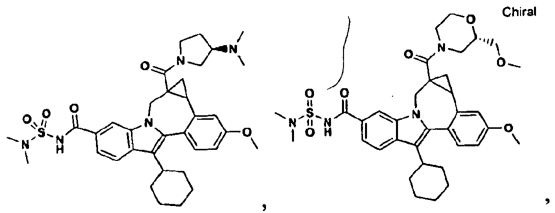 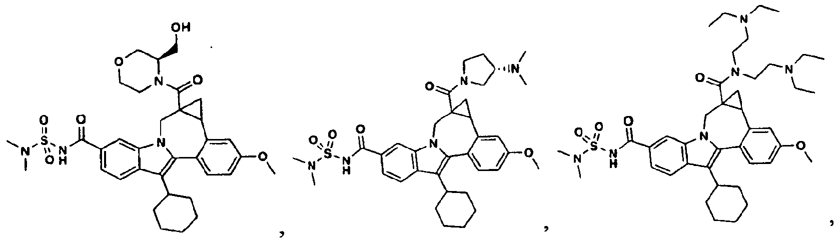 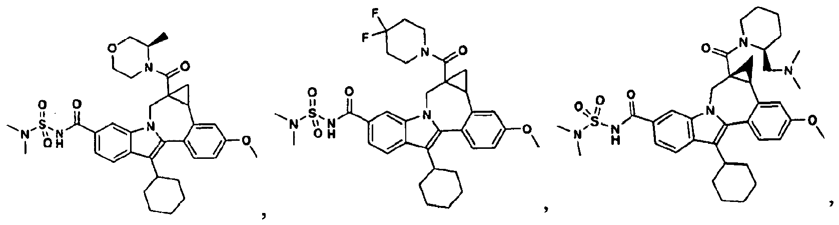 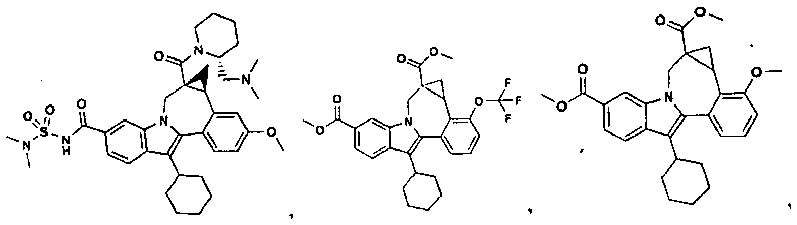 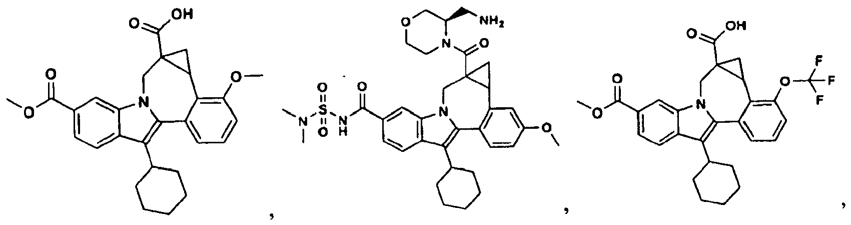 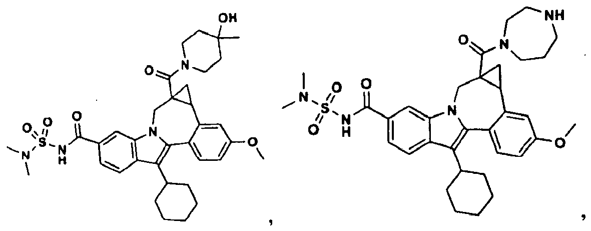 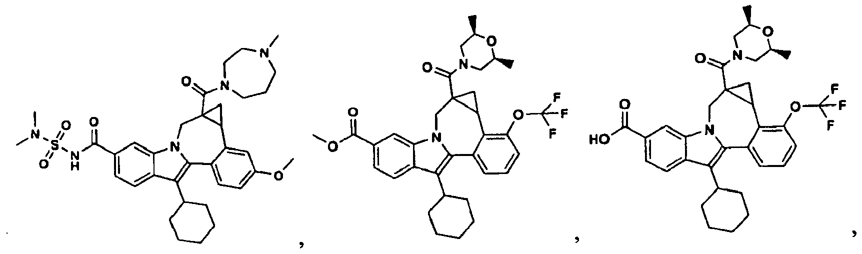 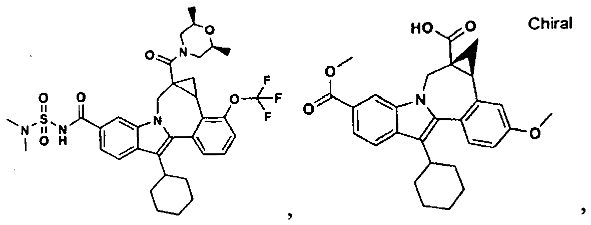 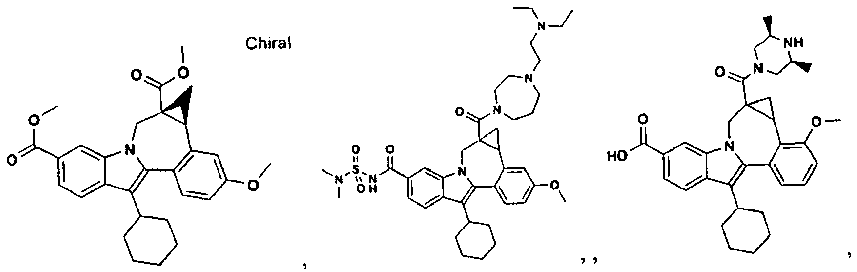 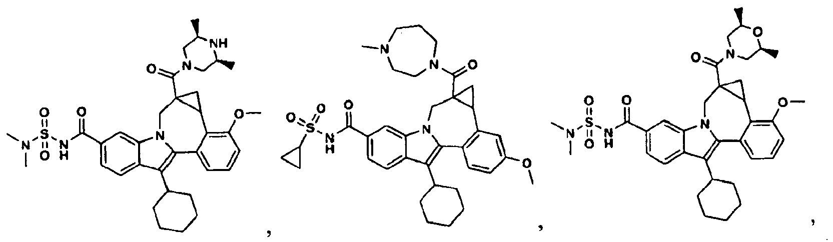 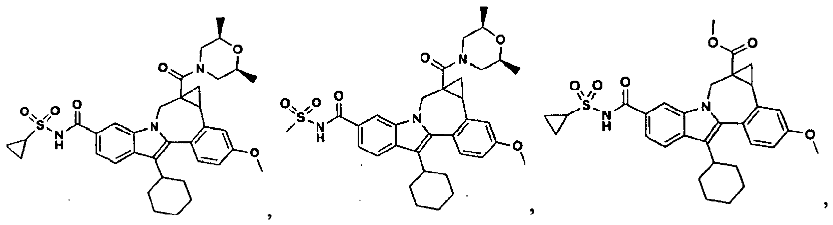 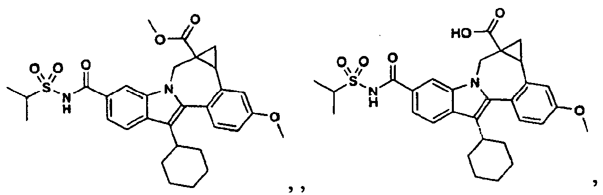 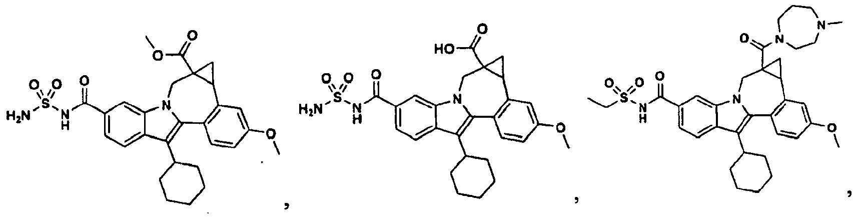 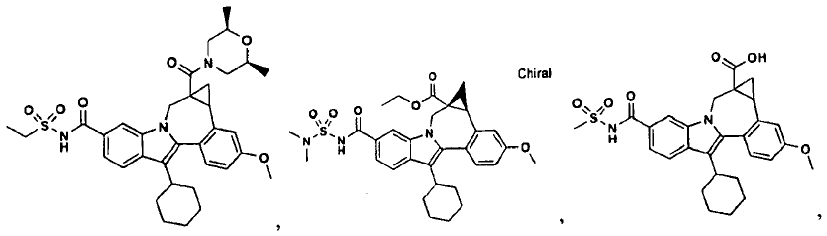 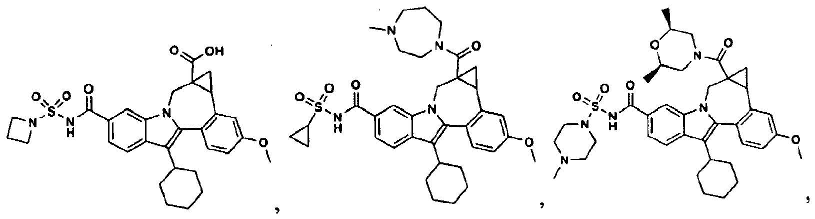 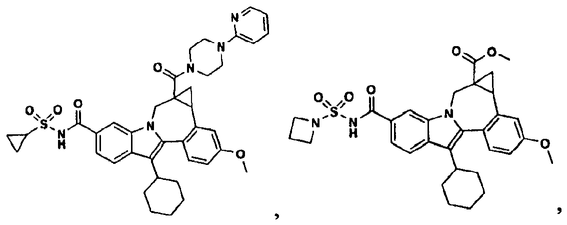 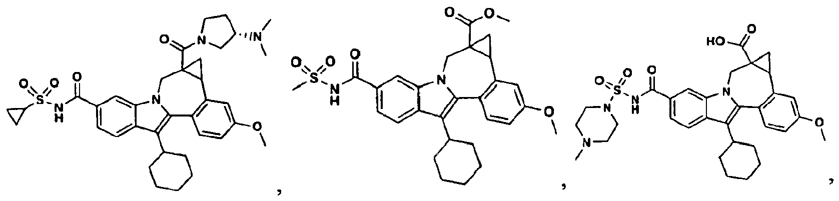 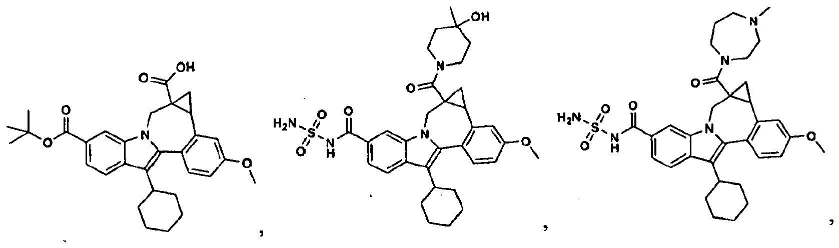 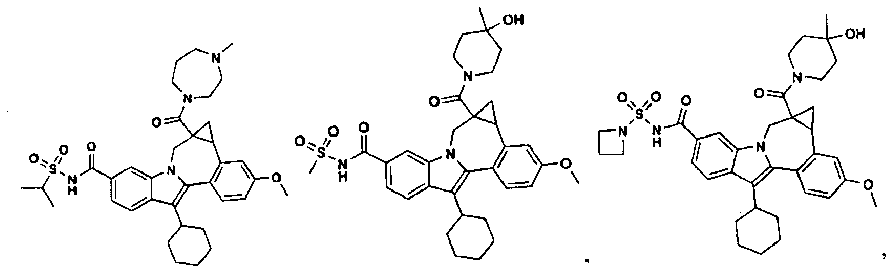 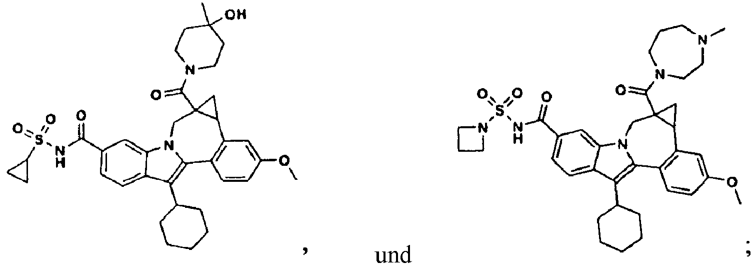 oder ein pharmazeutisch akzeptierbares Salz davon.

2. Zusammensetzung, die eine Verbindung nach Anspruch 1 oder ein pharmazeutisch akzeptierbares Salz davon und einen pharmazeutisch akzeptierbaren Träger aufweist.

3. Zusammensetzung nach Anspruch 2, die ferner mindestens eine zusätzliche Verbindung von therapeutischem Nutzen für HCV aufweist, wobei die Verbindung aus Interferonen, Cyclosporinen, Interleukinen, HCV-Metalloproteasehemmern, HCV-Serinproteasehemmem, HCV-Polymerasehemmern, HCV-Helicasehemmern, HCV-NS4B-Proteinhemmem, HCV-Eintrittshemmern, HCV-Assembly-Hemmern, HCV-Austrittshemmern, HCV-NS5A-Proteinhemmern, HCV-NSSB-Proteinhemmern und HCV-Replicon-Hemmern ausgewählt ist.

4. Verbindung nach Anspruch 1 zur Verwendung bei einer Methode zur Behandlung von Hepatitis C-Infektion.

5. Verbindung zur Verwendung nach Anspruch 4, die ferner die Verwendung mindestens einer zusätzlichen Verbindung von therapeutischem Nutzen für HCV umfasst, wobei die Verbindung aus Interferonen, Cyclosporinen, Interleukinen, HCV-Metalloproteasehemmem, HCV-Serinproteasehemmern, HCV-Polymerasehemmern, HCV-Helicasehemmern, HCV-NS4B-Proteinhemmem, HCV-Eintrittshemmem, HCV-Assembly-Hemmern, HCV-Austrittshemmern, HCV-NS5A-Proteinhemmern, HCV-NS5B-Proteinhemmem und HCV-Replicon-Hemmern ausgewählt ist.

## Revendications

1. Composé sélectionné parmi le groupe consistant en: 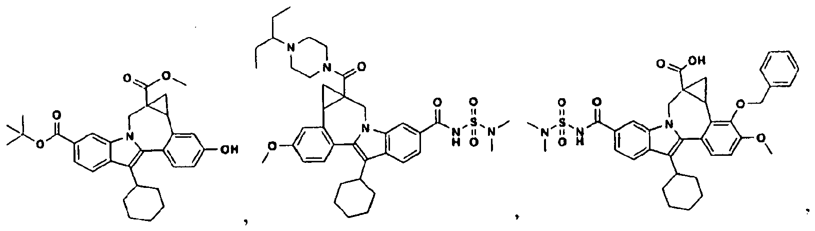 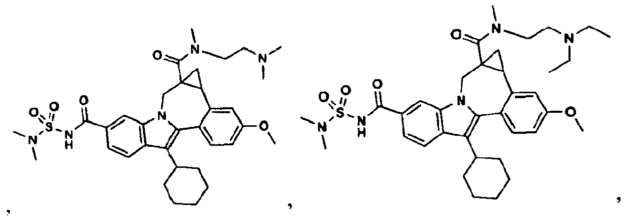 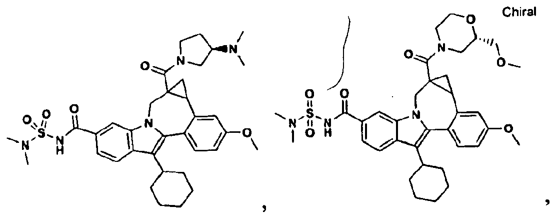 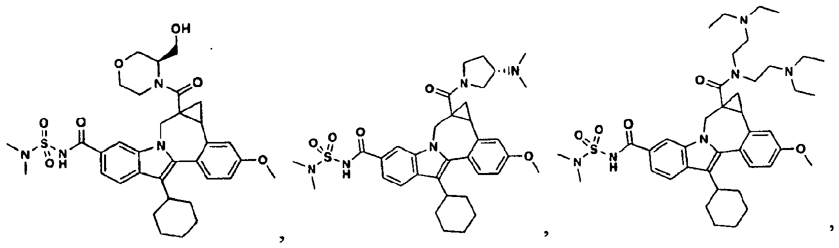 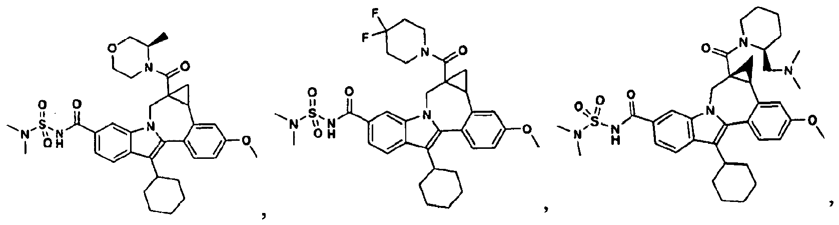 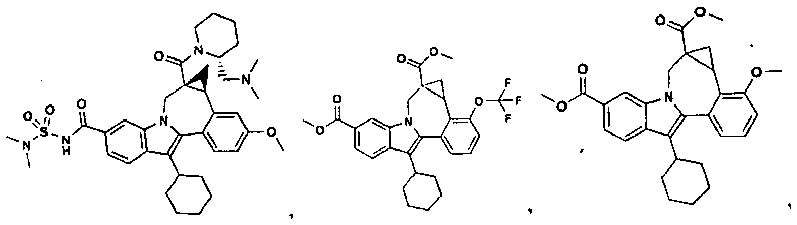 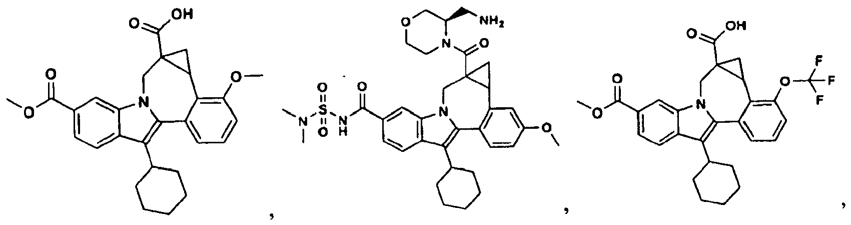 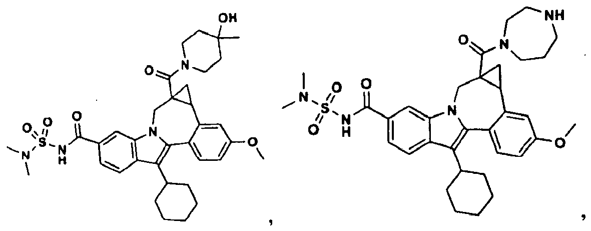 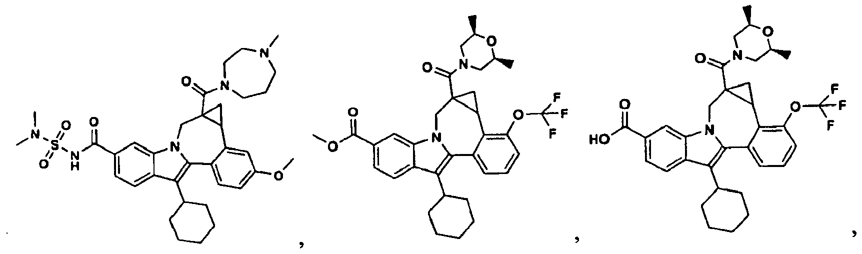 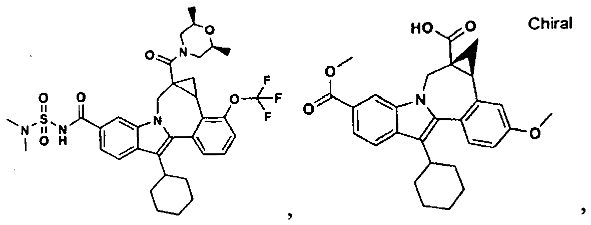 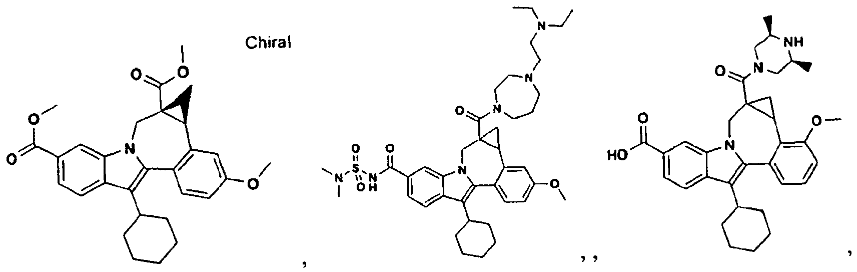 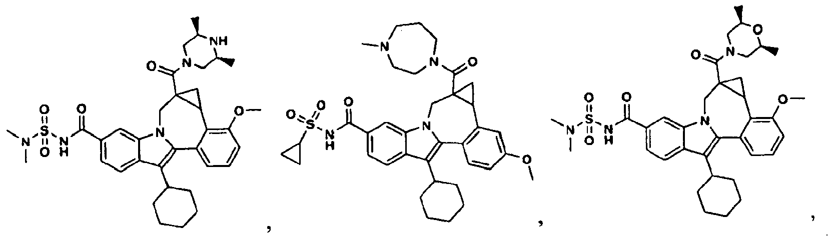 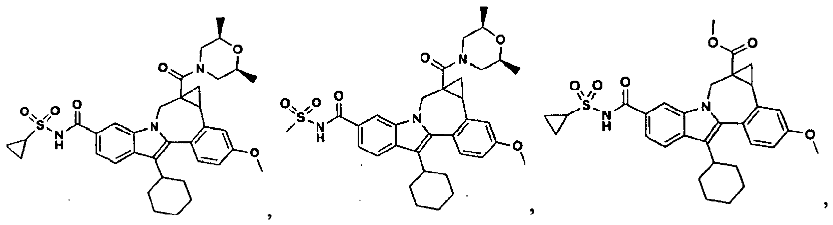 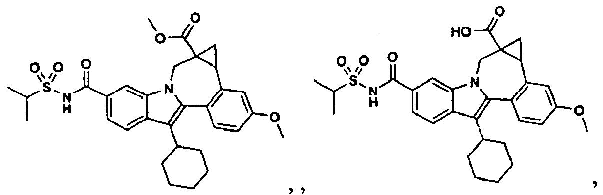 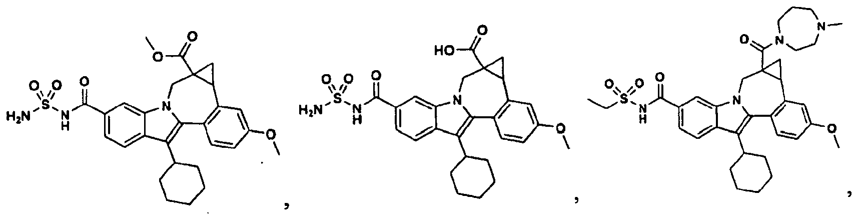 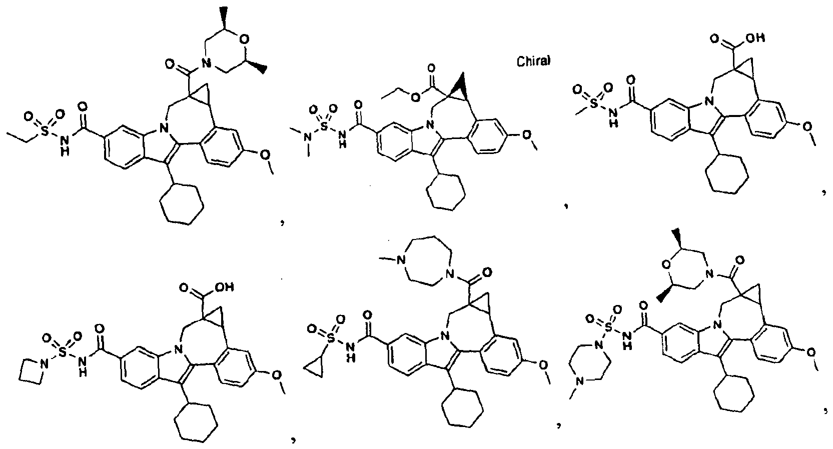 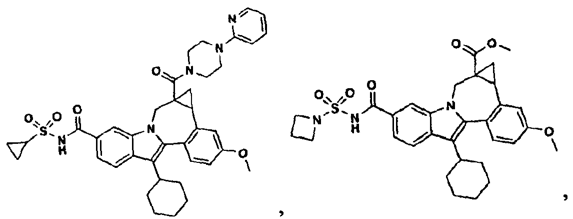 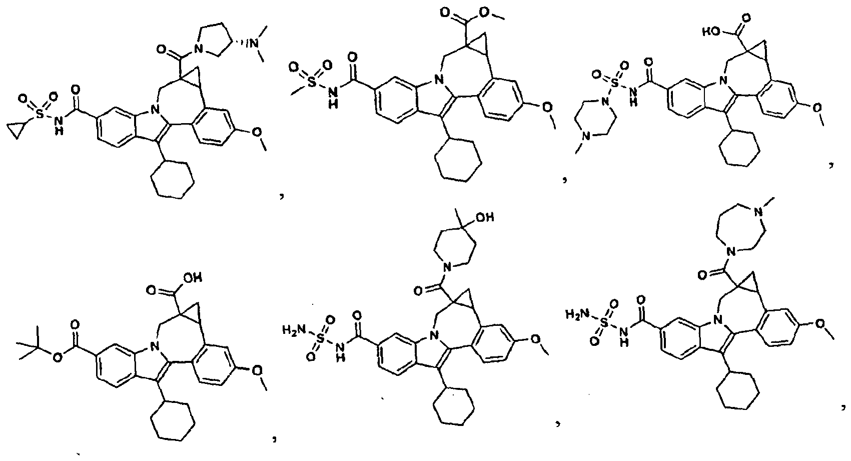 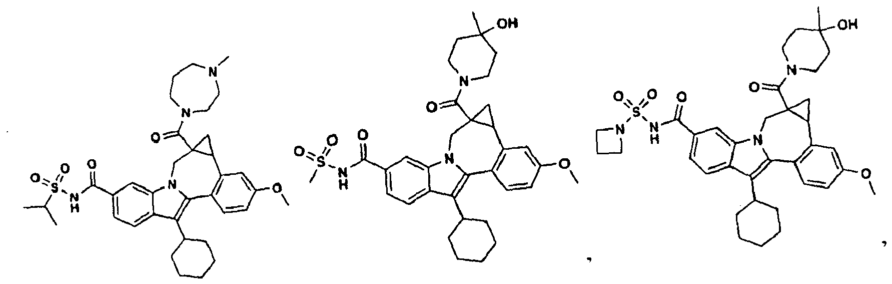 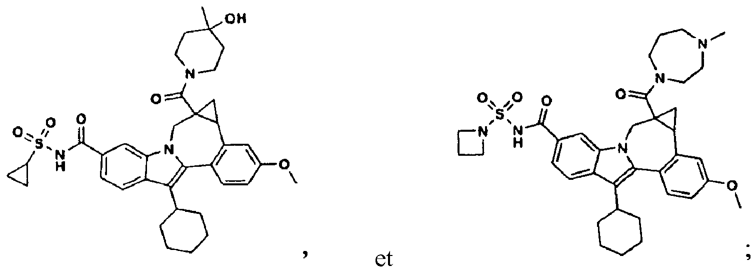 ou un sel pharmaceutiquement acceptable de celui-ci.

2. Composition comprenant un composé selon la revendication 1, ou un sel pharmaceutiquement acceptable de celui-ci, et un véhicule pharmaceutiquement acceptable.

3. Composition selon la revendication 2, comprenant en outre au moins un composé supplémentaire ayant des bénéfices thérapeutiques pour le VHC, dans laquelle le composé est sélectionné parmi le groupe consistant en interférons, cyclosporines, interleukines, inhibiteurs de la métalloprotéase du VHC, inhibiteurs de la sérine protéase du VHC, inhibiteurs de la polymérase du VHC, inhibiteurs de l'hélicase du VHC, inhibiteurs de la protéine NS4B du VHC, inhibiteurs d'entrée du VHC, inhibiteurs d'assemblage du VHC, inhibiteurs de sortie du VHC, inhibiteurs de la protéine NS5A du VHC, inhibiteurs de la protéine NS5B du VHC et inhibiteurs de réplicons du VHC.

4. Composé selon la revendication 1, à utiliser dans une méthode de traitement d'une infection d'hépatite C.

5. Composé à utiliser selon la revendication 4, comprenant en outre l'utilisation d'au moins un composé supplémentaire ayant des bénéfices thérapeutiques pour le VHC, où le composé est sélectionné parmi le groupe consistant en interférons, cyclosporines, interleukines, inhibiteurs de la métalloprotéase du VHC, inhibiteurs de la sérine protéase du VHC, inhibiteurs de la polymérase du VHC, inhibiteurs de l'hélicase du VHC, inhibiteurs de la protéine NS4B du VHC, inhibiteurs d'entrée du VHC, inhibiteurs d'assemblage du VHC, inhibiteurs de sortie du VHC, inhibiteurs de la protéine NS5A du VHC, inhibiteurs de la protéine NS5B du VHC et inhibiteurs de réplicons du VHC.
